(19) Europäisches Patentamt
European Patent Office
Office européen des brevets

(11) EP 4 062 920 A1

(12) EUROPEAN PATENT APPLICATION
published in accordance with Art. 153(4) EPC

(43) Date of publication:
**28.09.2022 Bulletin 2022/39**

(21) Application number: **20880671.1**

(22) Date of filing: **28.10.2020**

(51) International Patent Classification (IPC):
***A61K 35/15** (2015.01)* ***A61K 35/17** (2015.01)*
***A61K 38/16** (2006.01)* ***A61K 38/19** (2006.01)*
***A61K 38/20** (2006.01)* ***A61K 39/395** (2006.01)*
***A61K 45/00** (2006.01)* ***A61P 35/00** (2006.01)*
***C07K 14/47** (2006.01)* ***C07K 14/54** (2006.01)*
***C07K 14/725** (2006.01)* ***C07K 16/28** (2006.01)*
***C07K 19/00** (2006.01)* ***C12N 5/10** (2006.01)*
***C12N 15/12** (2006.01)* ***C12N 15/13** (2006.01)*
***C12N 15/24** (2006.01)* ***C12N 15/62** (2006.01)*
***C12N 15/867** (2006.01)*

(52) Cooperative Patent Classification (CPC):
**A61K 35/15; A61K 35/17; A61K 38/16; A61K 38/19; A61K 38/20; A61K 39/395; A61K 45/00; A61P 35/00; C07K 14/47; C07K 14/54; C07K 16/28; C07K 19/00; C12N 5/10; C12N 15/62; C12N 15/867**

(86) International application number:
**PCT/JP2020/040503**

(87) International publication number:
**WO 2021/085497 (06.05.2021 Gazette 2021/18)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.10.2019 JP 2019195407**

(71) Applicant: **Noile-Immune Biotech, Inc.**
**Tokyo 1050012 (JP)**

(72) Inventors:
- **TAMADA, Koji**
  **Ube-shi, Yamaguchi 755-8505 (JP)**
- **SAKODA, Yukimi**
  **Ube-shi, Yamaguchi 755-8505 (JP)**
- **ADACHI, Keishi**
  **Ube-shi, Yamaguchi 755-8505 (JP)**

(74) Representative: **V.O.**
**P.O. Box 87930**
**2508 DH Den Haag (NL)**

# (54) DRUG FOR TREATING CANCER, COMBINATION DRUG, DRUG COMPOSITION, IMMUNE RESPONSIVE CELL, NUCLEIC ACID DELIVERY VEHICLE, AND PRODUCT

(57) An exemplary combination drug includes (a1) an immunoresponsive cell expressing interleukine-7, CCL19, and a cell surface molecule that specifically recognizes a cancer antigen or (a2) one or more kinds of cells, one or more kinds of nucleic acid delivery vehicles, or a combination thereof, which cooperatively include a nucleic acid encoding interleukine-7 and a nucleic acid encoding CCL19; and (b) an immunosuppression inhibitor, and the drug is for use in treatment of a cancer in a subject. An exemplary immunoresponsive cell expresses interleukin-7, CCL19, an immunosuppression inhibiting polypeptide, and a cell surface molecule that specifically recognizes a cancer antigen.

EP 4 062 920 A1

FIG.4

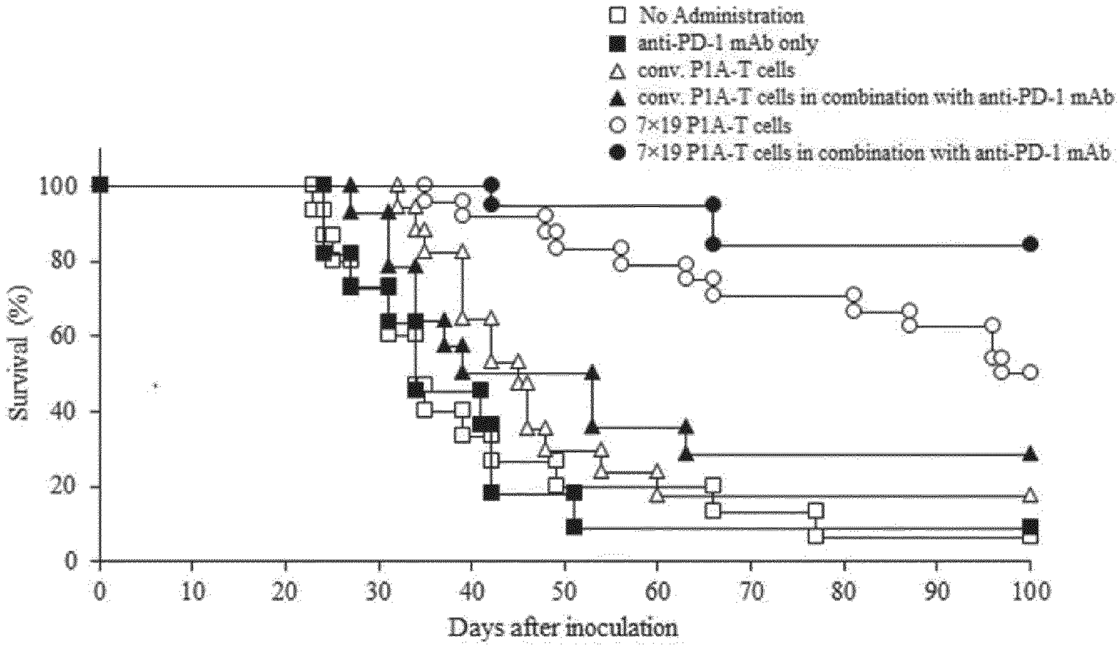
No Administration
anti-PD-1 mAb only
conv. P1A-T cells
conv. P1A-T cells in combination with anti-PD-1 mAb
7×19 P1A-T cells
7×19 P1A-T cells in combination with anti-PD-1 mAb
Survival (%)
100
80
60
40
20
0
0
10
20
30
40
50
60
70
80
90
100
Days after inoculation

SEQUENCE LISTING

<110> Noile-Immune Biotech Inc.

<120> Medicament, combination medicament, medical composition, immunoresponsive cell, nucleic acid delivery vehicle, and product for treating cancer

<130> CO-F05142-00

<150> JP2019-195407
<151> 2019-10-28

<160> 18

<170> PatentIn version 3.5

<210> 1
<211> 22
<212> DNA
<213> Artificial

<220>
<223> 5'-R primer

<400> 1
gtctaccagg cattcgcttc at 22

<210> 2
<211> 24
<212> DNA
<213> Artificial

<220>
<223> 3-TRa-C primer

<400> 2
tcagctggac cacagccgca gcgt 24

<210> 3
<211> 21
<212> DNA
<213> Artificial

<220>
<223> 3-TRb-C1 primer

<400> 3
tcagaaatcc tttctcttga c 21

<210> 4
<211> 24
<212> DNA
<213> Artificial

<220>
<223> 3-TRbeta-C2 primer

<400> 4
ctagcctctg gaatcctttc tctt 24

<210> 5
<211> 177
<212> PRT
<213> Artificial

<220>
<223> human IL-7

<400> 5

```
Met Phe His Val Ser Phe Arg Tyr Ile Phe Gly Leu Pro Pro Leu Ile
1               5                   10                  15

Leu Val Leu Leu Pro Val Ala Ser Ser Asp Cys Asp Ile Glu Gly Lys
            20                  25                  30

Asp Gly Lys Gln Tyr Glu Ser Val Leu Met Val Ser Ile Asp Gln Leu
        35                  40                  45

Leu Asp Ser Met Lys Glu Ile Gly Ser Asn Cys Leu Asn Asn Glu Phe
    50                  55                  60

Asn Phe Phe Lys Arg His Ile Cys Asp Ala Asn Lys Glu Gly Met Phe
65                  70                  75                  80

Leu Phe Arg Ala Ala Arg Lys Leu Arg Gln Phe Leu Lys Met Asn Ser
                85                  90                  95

Thr Gly Asp Phe Asp Leu His Leu Leu Lys Val Ser Glu Gly Thr Thr
            100                 105                 110

Ile Leu Leu Asn Cys Thr Gly Gln Val Lys Gly Arg Lys Pro Ala Ala
        115                 120                 125

Leu Gly Glu Ala Gln Pro Thr Lys Ser Leu Glu Glu Asn Lys Ser Leu
    130                 135                 140

Lys Glu Gln Lys Lys Leu Asn Asp Leu Cys Phe Leu Lys Arg Leu Leu
145                 150                 155                 160

Gln Glu Ile Lys Thr Cys Trp Asn Lys Ile Leu Met Gly Thr Lys Glu
                165                 170                 175

His
```

<210> 6
<211> 98
<212> PRT

<213> Artificial

<220>
<223> human CCL19

<400> 6

```
Met Ala Leu Leu Leu Ala Leu Ser Leu Leu Val Leu Trp Thr Ser Pro
1               5                   10                  15

Ala Pro Thr Leu Ser Gly Thr Asn Asp Ala Glu Asp Cys Cys Leu Ser
            20                  25                  30

Val Thr Gln Lys Pro Ile Pro Gly Tyr Ile Val Arg Asn Phe His Tyr
        35                  40                  45

Leu Leu Ile Lys Asp Gly Cys Arg Val Pro Ala Val Val Phe Thr Thr
    50                  55                  60

Leu Arg Gly Arg Gln Leu Cys Ala Pro Pro Asp Gln Pro Trp Val Glu
65                  70                  75                  80

Arg Ile Ile Gln Arg Leu Gln Arg Thr Ser Ala Lys Met Lys Arg Arg
                85                  90                  95

Ser Ser
```

<210> 7
<211> 8
<212> PRT
<213> Artificial

<220>
<223> 2A peptide cleavage region

<220>
<221> MISC_FEATURE
<223> Val or Ile

<220>
<221> misc_feature
<222> (2)..(2)
<223> Xaa can be any naturally occurring amino acid

<220>
<221> MISC_FEATURE
<222> (4)..(4)
<223> any amino acid

<400> 7

```
Asp Xaa Glu Xaa Asn Pro Gly Pro
1               5
```

<210> 8
<211> 25
<212> PRT
<213> Artificial

<220>
<223> 2Apeptide(F2A)

<400> 8

Gly Ser Gly Val Lys Gln Thr Leu Asn Phe Asp Leu Leu Lys Leu Ala
1 5 10 15

Gly Asp Val Glu Ser Asn Pro Gly Pro
20 25

<210> 9
<211> 864
<212> DNA
<213> Artificial

<220>
<223> mouseIL-7-F2A-mouseCCL19

<400> 9
atgttccatg tttcttttag atatatcttt ggaattcctc cactgatcct tgttctgctg 60

cctgtcacat catctgagtg ccacattaaa gacaaagaag gtaaagcata tgagagtgta 120

ctgatgatca gcatcgatga attggacaaa atgacaggaa ctgatagtaa ttgcccgaat 180

aatgaaccaa acttttttag aaaacatgta tgtgatgata caaaggaagc tgcttttcta 240

aatcgtgctg ctcgcaagtt gaagcaattt cttaaaatga atatcagtga agaattcaat 300

gtccacttac taacagtatc acaaggcaca caaacactgg tgaactgcac aagtaaggaa 360

gaaaaaaacg taaaggaaca gaaaaagaat gacgcatgtt tcctaaagag actactgaga 420

gaaataaaaa cttgttggaa taaaattttg aagggcagta taggaagcgg agtgaaacag 480

actttgaatt ttgaccttct caagttggcg ggagacgtgg agtccaaccc tggacctatg 540

gcccccccgtg tgaccccact cctggccttc agcctgctgg ttctctggac cttcccagcc 600

ccaactctgg ggggtgctaa tgatgcggaa gactgctgcc tgtctgtgac ccagcgcccc 660

atccctggga acatcgtgaa agccttccgc taccttctta atgaagatgg ctgcagggtg 720

cctgctgttg tgttcaccac actaaggggc tatcagctct gtgcacctcc agaccagccc 780

tgggtggatc gcatcatccg aagactgaag aagtcttctg ccaagaacaa aggcaacagc 840

accagaagga gccctgtgtc ttga 864

<210> 10
<211> 1665
<212> DNA
<213> Artificial

<220>
<223> Mouse Il-7-F2A-mouseCCL19-F2A-eGFP

<400> 10
atggtccacg tctccttcag atacatcttc ggcatccccc ccctgatcct ggtcctcctg 60

cctgtcacct ccagcgaatg tcatatcaag gacaaagagg gcaaggctta tgagagcgtc 120

ctgatgatct ccattgatga gctggataag atgaccggca ccgacagcaa ctgtcccaac 180

aatgagccca acttctttag aaagcacgtg tgtgacgata ccaaggaggc tgccttcctg 240

aacagggccg ccagaaagct gaagcagttc ctgaagatga acatttccga ggagttcaac 300

gtgcacctcc tcaccgtgag ccagggcacc cagacactgg tcaattgcac ctccaaggag 360

gagaagaacg tgaaagagca gaaaaagaat gatgcttgtt tcctcaagag gctgctgagg 420

gagatcaaga cctgttggaa taagatcctg aaaggcagca tcggcagcgg agtcaagcaa 480

accctgaact tcgacctgct gaaactggcc ggagatgtgg agagcaatcc cggccctatg 540

gcccccagag tcacccctct gctggccttc agcctgctcg tgctgtggac cttccccgct 600

cccaccctgg gcggcgccaa tgatgctgag gactgttgcc tctccgtgac ccagaggccc 660

atccctggaa acatcgtcaa agccttcagg tacctgctca acgaagacgg atgtagggtg 720

cctgccgtgg tgttcacaac actgagaggc taccagctct gcgcccctcc tgatcagccc 780

tgggtcgaca gaatcatcag aaggctgaag aagtccagcg ccaagaacaa aggcaatagc 840

acaaggagaa gccctgtgag cgaattcgga agcggagtga aacagacttt gaattttgac 900

cttctcaagt tggcgggaga cgtggagtcc aaccctggac catgcatggt gagcaagggc 960

gaggagctgt tcaccggggt ggtgcccatc ctggtcgagc tggacggcga cgtaaacggc 1020

cacaagttca gcgtgtccgg cgagggcgag ggcgatgcca cctacggcaa gctgaccctg 1080

aagttcatct gcaccaccgg caagctgccc gtgccctggc ccaccctcgt gaccaccctg 1140

acctacggcg tgcagtgctt cagccgctac cccgaccaca tgaagcagca cgacttcttc 1200

aagtccgcca tgcccgaagg ctacgtccag gagcgcacca tcttcttcaa ggacgacggc 1260

aactacaaga cccgcgccga ggtgaagttc gagggcgaca ccctggtgaa ccgcatcgag 1320

ctgaagggca tcgacttcaa ggaggacggc aacatcctgg ggcacaagct ggagtacaac 1380

tacaacagcc acaacgtcta tatcatggcc gacaagcaga agaacggcat caaggtgaac 1440

ttcaagatcc gccacaacat cgaggacggc agcgtgcagc tcgccgacca ctaccagcag 1500

aacaccccca tcggcgacgg ccccgtgctg ctgcccgaca accactacct gagcacccag 1560

tccgccctga gcaaagaccc caacgagaag cgcgatcaca tggtcctgct ggagttcgtg 1620

accgccgccg ggatcactct cggcatggac gagctgtaca agtaa 1665

<210> 11

<211> 554
<212> PRT
<213> Artificial

<220>
<223> Mouse Il-7-F2A-mouse CCL19-2A-eGFP amino acid

<400> 11

```
Met Val His Val Ser Phe Arg Tyr Ile Phe Gly Ile Pro Pro Leu Ile
1               5                   10                  15

Leu Val Leu Leu Pro Val Thr Ser Ser Glu Cys His Ile Lys Asp Lys
            20                  25                  30

Glu Gly Lys Ala Tyr Glu Ser Val Leu Met Ile Ser Ile Asp Glu Leu
        35                  40                  45

Asp Lys Met Thr Gly Thr Asp Ser Asn Cys Pro Asn Asn Glu Pro Asn
    50                  55                  60

Phe Phe Arg Lys His Val Cys Asp Asp Thr Lys Glu Ala Ala Phe Leu
65                  70                  75                  80

Asn Arg Ala Ala Arg Lys Leu Lys Gln Phe Leu Lys Met Asn Ile Ser
                85                  90                  95

Glu Glu Phe Asn Val His Leu Leu Thr Val Ser Gln Gly Thr Gln Thr
            100                 105                 110

Leu Val Asn Cys Thr Ser Lys Glu Glu Lys Asn Val Lys Glu Gln Lys
        115                 120                 125

Lys Asn Asp Ala Cys Phe Leu Lys Arg Leu Leu Arg Glu Ile Lys Thr
    130                 135                 140

Cys Trp Asn Lys Ile Leu Lys Gly Ser Ile Gly Ser Gly Val Lys Gln
145                 150                 155                 160

Thr Leu Asn Phe Asp Leu Leu Lys Leu Ala Gly Asp Val Glu Ser Asn
                165                 170                 175

Pro Gly Pro Met Ala Pro Arg Val Thr Pro Leu Leu Ala Phe Ser Leu
            180                 185                 190

Leu Val Leu Trp Thr Phe Pro Ala Pro Thr Leu Gly Gly Ala Asn Asp
        195                 200                 205

Ala Glu Asp Cys Cys Leu Ser Val Thr Gln Arg Pro Ile Pro Gly Asn
    210                 215                 220
```

Ile Val Lys Ala Phe Arg Tyr Leu Leu Asn Glu Asp Gly Cys Arg Val
225 230 235 240

Pro Ala Val Val Phe Thr Thr Leu Arg Gly Tyr Gln Leu Cys Ala Pro
245 250 255

Pro Asp Gln Pro Trp Val Asp Arg Ile Ile Arg Arg Leu Lys Lys Ser
260 265 270

Ser Ala Lys Asn Lys Gly Asn Ser Thr Arg Arg Ser Pro Val Ser Glu
275 280 285

Phe Gly Ser Gly Val Lys Gln Thr Leu Asn Phe Asp Leu Leu Lys Leu
290 295 300

Ala Gly Asp Val Glu Ser Asn Pro Gly Pro Cys Met Val Ser Lys Gly
305 310 315 320

Glu Glu Leu Phe Thr Gly Val Val Pro Ile Leu Val Glu Leu Asp Gly
325 330 335

Asp Val Asn Gly His Lys Phe Ser Val Ser Gly Glu Gly Glu Gly Asp
340 345 350

Ala Thr Tyr Gly Lys Leu Thr Leu Lys Phe Ile Cys Thr Thr Gly Lys
355 360 365

Leu Pro Val Pro Trp Pro Thr Leu Val Thr Thr Leu Thr Tyr Gly Val
370 375 380

Gln Cys Phe Ser Arg Tyr Pro Asp His Met Lys Gln His Asp Phe Phe
385 390 395 400

Lys Ser Ala Met Pro Glu Gly Tyr Val Gln Glu Arg Thr Ile Phe Phe
405 410 415

Lys Asp Asp Gly Asn Tyr Lys Thr Arg Ala Glu Val Lys Phe Glu Gly
420 425 430

Asp Thr Leu Val Asn Arg Ile Glu Leu Lys Gly Ile Asp Phe Lys Glu
435 440 445

Asp Gly Asn Ile Leu Gly His Lys Leu Glu Tyr Asn Tyr Asn Ser His
450 455 460

Asn Val Tyr Ile Met Ala Asp Lys Gln Lys Asn Gly Ile Lys Val Asn

465 470 475 480

Phe Lys Ile Arg His Asn Ile Glu Asp Gly Ser Val Gln Leu Ala Asp
485 490 495

His Tyr Gln Gln Asn Thr Pro Ile Gly Asp Gly Pro Val Leu Leu Pro
500 505 510

Asp Asn His Tyr Leu Ser Thr Gln Ser Ala Leu Ser Lys Asp Pro Asn
515 520 525

Glu Lys Arg Asp His Met Val Leu Leu Glu Phe Val Thr Ala Ala Gly
530 535 540

Ile Thr Leu Gly Met Asp Glu Leu Tyr Lys
545 550

<210> 12
<211> 9
<212> PRT
<213> Artificial

<220>
<223> P1A peptide

<400> 12

Leu Pro Tyr Leu Gly Trp Leu Val Phe
1 5

<210> 13
<211> 20
<212> RNA
<213> Artificial

<220>
<223> Guide RNA for PD-1 Gene

<400> 13
ucugggcaug ugggucggc 20

<210> 14
<211> 20
<212> RNA
<213> Artificial

<220>
<223> Guide RNA for ROSA26

<400> 14
cuccagucuu ucuagaagau 20

<210> 15
<211> 2559

<212> DNA
<213> Artificial

<220>
<223> anti-hCD20 CAR and anti-mPD-antibody

<400> 15
atggactgga cctggcggat cctgttcctg gtggctgctg ctacaggcgc ccacagccag 60

atcgtgctgt ctcagtctcc cgccatcctg tctgctagcc ctggcgagaa agtgaccatg 120

acctgcagag ccagcagcag cgtgtcctac atccactggt tccagcagaa gcccggcagc 180

agccccaagc cttggatcta cgccacaagc aacctggcct ctggcgtgcc agtgcggttt 240

agcggctctg gctctggcac cagctacagc ctgaccatca gcagagtgga agccgaggac 300

gccgccacct actactgtca gcagtggacc agcaaccccc ccacattcgg cggaggcacc 360

aagctggaaa tcaagggcgg aggcggatct ggcggcggag gatctggggg aggcggctct 420

caggtgcagc tgcagcagcc tggcgctgag ctcgtgaaac ctggcgcctc cgtgaagatg 480

agctgcaagg ccagcggcta caccttcaca agctacaaca tgcactgggt caagcagacc 540

cctggcagag gcctggaatg gatcggcgct atctaccccg gcaacggcga cacctcctac 600

aaccagaagt tcaagggcaa ggccaccctg accgccgaca agagcagcag cacagcctac 660

atgcagctgt cctccctgac cagcgaggac agcgccgtgt actactgcgc cagatctacc 720

tactacggcg gcgactggta cttcaacgtg tggggcgctg gcaccaccgt gaccgtgtct 780

gctgcggccg cagtcgtgcc agtccttcag aaagtgaact ctactactac caagccagtg 840

ctgcgaactc cctcacctgt gcaccctacc gggacatctc agccccagag accagaagat 900

tgtcggcccc gtggctcagt gaaggggacc ggattggact tcgcctgtga tatttacatc 960

tgggcaccct tggccggaat ctgcgtggcc cctctgctgt ccttgatcat cactctcatc 1020

tgctaccaca ggagccgaaa tagtagaagg aacagactcc ttcaaagtga ctacatgaac 1080

atgactcccc ggaggcctgg gctcactcga aagccttacc agccctacgc ccctgccaga 1140

gactttgcag cgtaccgccc caaatggatc aggaaaaaat tcccccacat attcaagcaa 1200

ccatttaaga agaccactgg agcagctcaa gaggaagatg cttgtagctg ccgatgtcca 1260

caggaagaag aaggaggagg aggaggctat gagctgagag caaaattcag caggagtgca 1320

gagactgctg ccaacctgca ggaccccaac cagctctaca atgagctcaa tctagggcga 1380

agagaggaat atgacgtctt ggagaagaag cgggctcggg atccagagat gggaggcaaa 1440

cagcagagga ggaggaaccc ccaggaaggc gtatacaatg cactgcagaa agacaagatg 1500

gcagaagcct acagtgagat cggcacaaaa ggcgagaggc ggagaggcaa ggggcacgat 1560

ggcctttacc agggtctcag cactgccacc aaggacacct atgatgccct gcatatgcag 1620

accctggccc ctcgcgaatt cggctccggc gtgaagcaga cactgaactt cgacctgctc 1680

aagctggctg gagacgtgga gagcaacccc ggccctatga catgggcccc tctgctgctg 1740

attttcctgc agcacctgag aggcagctgc gcccagtttg tgctgaccca gcccaaaaca 1800

gtgtccgaga gcctgggcag aaccgtgaca atcagctgca aaaggagcag cggctccgtg 1860

ggcgattact acgtgagctg gcaccagcag aggtttggca gcagccctaa gaccgtcatc 1920

tacctggacg atgagaggcc ttccggcgtc cccaaaaggt tctccggctc catcgacagc 1980

tccagcaata gcgctagcct caccatcacc gacctccaga ccgacgatga ggccgattac 2040

ttctgcctca gctacgacag caacaaccac ttcgtcttcg gctccggaac ccacctgaca 2100

gtgctgggcg gaggaggatc cggaggagga ggaagcggag gaggcggaag cgaggtgcag 2160

ctggtggagt ccggaggagg actggctcaa cccggaaaga gcctgaagct gtcctgcgag 2220

gccagcggct tcacctttag cgactacggc atgaactggt tcaggcaggc ccccggaaag 2280

ggcctggaat gggtggccta catcagcagc ggctccaaca agatcaccta cgccgacaca 2340

gtcaagggca ggtttaccgt gagcagggat aacggcaaga accagctgtt cctgcagatg 2400

aataacctga agagcgagga taccgccatc tactattgcg tggattccgg cttcaactcc 2460

tatagcgacg tctggggcca gggcatccag gtcaccgtga gcagcgacta caaggacgac 2520

gacgacaagt tcgtggagca tcaccaccat caccactga 2559

<210> 16
<211> 852
<212> PRT
<213> Artificial

<220>
<223> anti-hCD20 CAR and anti-mPD-antibody

<400> 16

```
Met Asp Trp Thr Trp Arg Ile Leu Phe Leu Val Ala Ala Ala Thr Gly
1               5                   10                  15

Ala His Ser Gln Ile Val Leu Ser Gln Ser Pro Ala Ile Leu Ser Ala
            20                  25                  30

Ser Pro Gly Glu Lys Val Thr Met Thr Cys Arg Ala Ser Ser Ser Val
        35                  40                  45

Ser Tyr Ile His Trp Phe Gln Gln Lys Pro Gly Ser Ser Pro Lys Pro
    50                  55                  60

Trp Ile Tyr Ala Thr Ser Asn Leu Ala Ser Gly Val Pro Val Arg Phe
65                  70                  75                  80

Ser Gly Ser Gly Ser Gly Thr Ser Tyr Ser Leu Thr Ile Ser Arg Val
                85                  90                  95
```

Glu Ala Glu Asp Ala Ala Thr Tyr Tyr Cys Gln Gln Trp Thr Ser Asn
100 105 110

Pro Pro Thr Phe Gly Gly Gly Thr Lys Leu Glu Ile Lys Gly Gly Gly
115 120 125

Gly Ser Gly Gly Gly Gly Ser Gly Gly Gly Gly Ser Gln Val Gln Leu
130 135 140

Gln Gln Pro Gly Ala Glu Leu Val Lys Pro Gly Ala Ser Val Lys Met
145 150 155 160

Ser Cys Lys Ala Ser Gly Tyr Thr Phe Thr Ser Tyr Asn Met His Trp
165 170 175

Val Lys Gln Thr Pro Gly Arg Gly Leu Glu Trp Ile Gly Ala Ile Tyr
180 185 190

Pro Gly Asn Gly Asp Thr Ser Tyr Asn Gln Lys Phe Lys Gly Lys Ala
195 200 205

Thr Leu Thr Ala Asp Lys Ser Ser Ser Thr Ala Tyr Met Gln Leu Ser
210 215 220

Ser Leu Thr Ser Glu Asp Ser Ala Val Tyr Tyr Cys Ala Arg Ser Thr
225 230 235 240

Tyr Tyr Gly Gly Asp Trp Tyr Phe Asn Val Trp Gly Ala Gly Thr Thr
245 250 255

Val Thr Val Ser Ala Ala Ala Ala Val Val Pro Val Leu Gln Lys Val
260 265 270

Asn Ser Thr Thr Thr Lys Pro Val Leu Arg Thr Pro Ser Pro Val His
275 280 285

Pro Thr Gly Thr Ser Gln Pro Gln Arg Pro Glu Asp Cys Arg Pro Arg
290 295 300

Gly Ser Val Lys Gly Thr Gly Leu Asp Phe Ala Cys Asp Ile Tyr Ile
305 310 315 320

Trp Ala Pro Leu Ala Gly Ile Cys Val Ala Pro Leu Leu Ser Leu Ile
325 330 335

Ile Thr Leu Ile Cys Tyr His Arg Ser Arg Asn Ser Arg Arg Asn Arg

```
            340                 345                 350

Leu Leu Gln Ser Asp Tyr Met Asn Met Thr Pro Arg Arg Pro Gly Leu
        355                 360                 365

Thr Arg Lys Pro Tyr Gln Pro Tyr Ala Pro Ala Arg Asp Phe Ala Ala
    370                 375                 380

Tyr Arg Pro Lys Trp Ile Arg Lys Lys Phe Pro His Ile Phe Lys Gln
385                 390                 395                 400

Pro Phe Lys Lys Thr Thr Gly Ala Ala Gln Glu Glu Asp Ala Cys Ser
                405                 410                 415

Cys Arg Cys Pro Gln Glu Glu Glu Gly Gly Gly Gly Gly Tyr Glu Leu
            420                 425                 430

Arg Ala Lys Phe Ser Arg Ser Ala Glu Thr Ala Ala Asn Leu Gln Asp
        435                 440                 445

Pro Asn Gln Leu Tyr Asn Glu Leu Asn Leu Gly Arg Arg Glu Glu Tyr
    450                 455                 460

Asp Val Leu Glu Lys Lys Arg Ala Arg Asp Pro Glu Met Gly Gly Lys
465                 470                 475                 480

Gln Gln Arg Arg Arg Asn Pro Gln Glu Gly Val Tyr Asn Ala Leu Gln
                485                 490                 495

Lys Asp Lys Met Ala Glu Ala Tyr Ser Glu Ile Gly Thr Lys Gly Glu
            500                 505                 510

Arg Arg Arg Gly Lys Gly His Asp Gly Leu Tyr Gln Gly Leu Ser Thr
        515                 520                 525

Ala Thr Lys Asp Thr Tyr Asp Ala Leu His Met Gln Thr Leu Ala Pro
    530                 535                 540

Arg Glu Phe Gly Ser Gly Val Lys Gln Thr Leu Asn Phe Asp Leu Leu
545                 550                 555                 560

Lys Leu Ala Gly Asp Val Glu Ser Asn Pro Gly Pro Met Thr Trp Ala
                565                 570                 575

Pro Leu Leu Leu Ile Phe Leu Gln His Leu Arg Gly Ser Cys Ala Gln
            580                 585                 590
```

Phe Val Leu Thr Gln Pro Lys Thr Val Ser Glu Ser Leu Gly Arg Thr
595 600 605

Val Thr Ile Ser Cys Lys Arg Ser Ser Gly Ser Val Gly Asp Tyr Tyr
610 615 620

Val Ser Trp His Gln Gln Arg Phe Gly Ser Ser Pro Lys Thr Val Ile
625 630 635 640

Tyr Leu Asp Asp Glu Arg Pro Ser Gly Val Pro Lys Arg Phe Ser Gly
645 650 655

Ser Ile Asp Ser Ser Ser Asn Ser Ala Ser Leu Thr Ile Thr Asp Leu
660 665 670

Gln Thr Asp Asp Glu Ala Asp Tyr Phe Cys Leu Ser Tyr Asp Ser Asn
675 680 685

Asn His Phe Val Phe Gly Ser Gly Thr His Leu Thr Val Leu Gly Gly
690 695 700

Gly Gly Ser Gly Gly Gly Gly Ser Gly Gly Gly Gly Ser Glu Val Gln
705 710 715 720

Leu Val Glu Ser Gly Gly Gly Leu Ala Gln Pro Gly Lys Ser Leu Lys
725 730 735

Leu Ser Cys Glu Ala Ser Gly Phe Thr Phe Ser Asp Tyr Gly Met Asn
740 745 750

Trp Phe Arg Gln Ala Pro Gly Lys Gly Leu Glu Trp Val Ala Tyr Ile
755 760 765

Ser Ser Gly Ser Asn Lys Ile Thr Tyr Ala Asp Thr Val Lys Gly Arg
770 775 780

Phe Thr Val Ser Arg Asp Asn Gly Lys Asn Gln Leu Phe Leu Gln Met
785 790 795 800

Asn Asn Leu Lys Ser Glu Asp Thr Ala Ile Tyr Tyr Cys Val Asp Ser
805 810 815

Gly Phe Asn Ser Tyr Ser Asp Val Trp Gly Gln Gly Ile Gln Val Thr
820 825 830

Val Ser Ser Asp Tyr Lys Asp Asp Asp Asp Lys Phe Val Glu His His
835 840 845

His His His His
    850

<210>  17
<211>  3501
<212>  DNA
<213>  Artificial

<220>
<223>  anti-hCD20 CAR, mil7, mccl19, and anti-mPD-antibody

<400>  17
atggactgga cctggcggat cctgttcctg gtggctgctg ctacaggcgc ccacagccag       60

atcgtgctgt ctcagtctcc cgccatcctg tctgctagcc ctggcgagaa agtgaccatg      120

acctgcagag ccagcagcag cgtgtcctac atccactggt tccagcagaa gcccggcagc      180

agccccaagc cttggatcta cgccacaagc aacctggcct ctggcgtgcc agtgcggttt      240

agcggctctg gctctggcac cagctacagc ctgaccatca gcagagtgga agccgaggac      300

gccgccacct actactgtca gcagtggacc agcaaccccc ccacattcgg cggaggcacc      360

aagctggaaa tcaagggcgg aggcggatct ggcggcggag gatctggggg aggcggctct      420

caggtgcagc tgcagcagcc tggcgctgag ctcgtgaaac ctggcgcctc cgtgaagatg      480

agctgcaagg ccagcggcta caccttcaca agctacaaca tgcactgggt caagcagacc      540

cctggcagag gcctggaatg gatcggcgct atctaccccg gcaacggcga cacctcctac      600

aaccagaagt tcaagggcaa ggccaccctg accgccgaca agagcagcag cacagcctac      660

atgcagctgt cctccctgac cagcgaggac agcgccgtgt actactgcgc cagatctacc      720

tactacggcg gcgactggta cttcaacgtg tggggcgctg gcaccaccgt gaccgtgtct      780

gctgcggccg cagtcgtgcc agtccttcag aaagtgaact ctactactac caagccagtg      840

ctgcgaactc cctcacctgt gcaccctacc gggacatctc agccccagag accagaagat      900

tgtcggcccc gtggctcagt gaaggggacc ggattggact tcgcctgtga tatttacatc      960

tgggcaccct tggccggaat ctgcgtggcc cctctgctgt ccttgatcat cactctcatc     1020

tgctaccaca ggagccgaaa tagtagaagg aacagactcc ttcaaagtga ctacatgaac     1080

atgactcccc ggaggcctgg gctcactcga aagccttacc agccctacgc ccctgccaga     1140

gactttgcag cgtaccgccc caaatggatc aggaaaaaat tcccccacat attcaagcaa     1200

ccatttaaga agaccactgg agcagctcaa gaggaagatg cttgtagctg ccgatgtcca     1260

caggaagaag aaggaggagg aggaggctat gagctgagag caaaattcag caggagtgca     1320

gagactgctg ccaacctgca ggaccccaac cagctctaca atgagctcaa tctagggcga     1380

agagaggaat atgacgtctt ggagaagaag cgggctcggg atccagagat gggaggcaaa     1440

cagcagagga ggaggaaccc ccaggaaggc gtatacaatg cactgcagaa agacaagatg     1500

gcagaagcct acagtgagat cggcacaaaa ggcgagaggc ggagaggcaa ggggcacgat 1560
ggcctttacc agggtctcag cactgccacc aaggacacct atgatgccct gcatatgcag 1620
accctggccc ctcgcgaatt cggaagcgga gtgaaacaga ctttgaattt tgaccttctc 1680
aagttggcgg gagacgtgga gtccaaccct ggaccatgca tgttccatgt ttcttttaga 1740
tatatctttg gaattcctcc actgatcctt gttctgctgc ctgtcacatc atctgagtgc 1800
cacattaaag acaaagaagg taaagcatat gagagtgtac tgatgatcag catcgatgaa 1860
ttggacaaaa tgacaggaac tgatagtaat tgcccgaata atgaaccaaa ctttttttaga 1920
aaacatgtat gtgatgatac aaaggaagct gcttttctaa atcgtgctgc tcgcaagttg 1980
aagcaatttc ttaaaatgaa tatcagtgaa gaattcaatg tccacttact aacagtatca 2040
caaggcacac aaacactggt gaactgcaca agtaaggaag aaaaaaacgt aaaggaacag 2100
aaaaagaatg acgcatgttt cctaaagaga ctactgagag aaataaaaac ttgttggaat 2160
aaaattttga agggcagtat aggaagcgga gtgaaacaga ctttgaattt tgaccttctc 2220
aagttggcgg gagacgtgga gtccaaccct ggacctatgg ccccccgtgt gaccccactc 2280
ctggccttca gcctgctggt tctctggacc ttcccagccc caactctggg gggtgctaat 2340
gatgcggaag actgctgcct gtctgtgacc cagcgcccca tccctgggaa catcgtgaaa 2400
gccttccgct accttcttaa tgaagatggc tgcagggtgc ctgctgttgt gttcaccaca 2460
ctaaggggct atcagctctg tgcacctcca gaccagccct gggtggatcg catcatccga 2520
agactgaaga agtcttctgc caagaacaaa ggcaacagca ccagaaggag ccctgtgtct 2580
cgaggctccg gcgtgaagca gacactgaac ttcgacctgc tcaagctggc tggagacgtg 2640
gagagcaacc ccggccctat gacatgggcc cctctgctgc tgattttcct gcagcacctg 2700
agaggcagct gcgcccagtt tgtgctgacc cagcccaaaa cagtgtccga gagcctgggc 2760
agaaccgtga caatcagctg caaaaggagc agcggctccg tgggcgatta ctacgtgagc 2820
tggcaccagc agaggtttgg cagcagccct aagaccgtca tctacctgga cgatgagagg 2880
ccttccggcg tccccaaaag gttctccggc tccatcgaca gctccagcaa tagcgctagc 2940
ctcaccatca ccgacctcca gaccgacgat gaggccgatt acttctgcct cagctacgac 3000
agcaacaacc acttcgtctt cggctccgga acccacctga cagtgctggg cggaggagga 3060
tccggaggag gaggaagcgg aggaggcgga agcgaggtgc agctggtgga gtccggagga 3120
ggactggctc aacccggaaa gagcctgaag ctgtcctgcg aggccagcgg cttcaccttt 3180
agcgactacg gcatgaactg gttcaggcag gcccccggaa agggcctgga atgggtggcc 3240
tacatcagca gcggctccaa caagatcacc tacgccgaca cagtcaaggg caggtttacc 3300
gtgagcaggg ataacggcaa gaaccagctg ttcctgcaga tgaataacct gaagagcgag 3360

gataccgcca tctactattg cgtggattcc ggcttcaact cctatagcga cgtctggggc 3420

cagggcatcc aggtcaccgt gagcagcgac tacaaggacg acgacgacaa gttcgtggag 3480

catcaccacc atcaccactg a 3501

<210> 18
<211> 1166
<212> PRT
<213> Artificial

<220>
<223> anti-hCD20 CAR, mil7, mccl19, and anti-mPD-antibody

<400> 18

Met Asp Trp Thr Trp Arg Ile Leu Phe Leu Val Ala Ala Ala Thr Gly
1 5 10 15

Ala His Ser Gln Ile Val Leu Ser Gln Ser Pro Ala Ile Leu Ser Ala
20 25 30

Ser Pro Gly Glu Lys Val Thr Met Thr Cys Arg Ala Ser Ser Ser Val
35 40 45

Ser Tyr Ile His Trp Phe Gln Gln Lys Pro Gly Ser Ser Pro Lys Pro
50 55 60

Trp Ile Tyr Ala Thr Ser Asn Leu Ala Ser Gly Val Pro Val Arg Phe
65 70 75 80

Ser Gly Ser Gly Ser Gly Thr Ser Tyr Ser Leu Thr Ile Ser Arg Val
85 90 95

Glu Ala Glu Asp Ala Ala Thr Tyr Tyr Cys Gln Gln Trp Thr Ser Asn
100 105 110

Pro Pro Thr Phe Gly Gly Gly Thr Lys Leu Glu Ile Lys Gly Gly Gly
115 120 125

Gly Ser Gly Gly Gly Gly Ser Gly Gly Gly Gly Ser Gln Val Gln Leu
130 135 140

Gln Gln Pro Gly Ala Glu Leu Val Lys Pro Gly Ala Ser Val Lys Met
145 150 155 160

Ser Cys Lys Ala Ser Gly Tyr Thr Phe Thr Ser Tyr Asn Met His Trp
165 170 175

Val Lys Gln Thr Pro Gly Arg Gly Leu Glu Trp Ile Gly Ala Ile Tyr
180 185 190

Pro Gly Asn Gly Asp Thr Ser Tyr Asn Gln Lys Phe Lys Gly Lys Ala
195 200 205

Thr Leu Thr Ala Asp Lys Ser Ser Ser Thr Ala Tyr Met Gln Leu Ser
210 215 220

Ser Leu Thr Ser Glu Asp Ser Ala Val Tyr Tyr Cys Ala Arg Ser Thr
225 230 235 240

Tyr Tyr Gly Gly Asp Trp Tyr Phe Asn Val Trp Gly Ala Gly Thr Thr
245 250 255

Val Thr Val Ser Ala Ala Ala Ala Val Val Pro Val Leu Gln Lys Val
260 265 270

Asn Ser Thr Thr Thr Lys Pro Val Leu Arg Thr Pro Ser Pro Val His
275 280 285

Pro Thr Gly Thr Ser Gln Pro Gln Arg Pro Glu Asp Cys Arg Pro Arg
290 295 300

Gly Ser Val Lys Gly Thr Gly Leu Asp Phe Ala Cys Asp Ile Tyr Ile
305 310 315 320

Trp Ala Pro Leu Ala Gly Ile Cys Val Ala Pro Leu Leu Ser Leu Ile
325 330 335

Ile Thr Leu Ile Cys Tyr His Arg Ser Arg Asn Ser Arg Arg Asn Arg
340 345 350

Leu Leu Gln Ser Asp Tyr Met Asn Met Thr Pro Arg Arg Pro Gly Leu
355 360 365

Thr Arg Lys Pro Tyr Gln Pro Tyr Ala Pro Ala Arg Asp Phe Ala Ala
370 375 380

Tyr Arg Pro Lys Trp Ile Arg Lys Lys Phe Pro His Ile Phe Lys Gln
385 390 395 400

Pro Phe Lys Lys Thr Thr Gly Ala Ala Gln Glu Glu Asp Ala Cys Ser
405 410 415

Cys Arg Cys Pro Gln Glu Glu Glu Gly Gly Gly Gly Gly Tyr Glu Leu
420 425 430

Arg Ala Lys Phe Ser Arg Ser Ala Glu Thr Ala Ala Asn Leu Gln Asp

435 440 445

Pro Asn Gln Leu Tyr Asn Glu Leu Asn Leu Gly Arg Arg Glu Glu Tyr
450 455 460

Asp Val Leu Glu Lys Lys Arg Ala Arg Asp Pro Glu Met Gly Gly Lys
465 470 475 480

Gln Gln Arg Arg Arg Asn Pro Gln Glu Gly Val Tyr Asn Ala Leu Gln
485 490 495

Lys Asp Lys Met Ala Glu Ala Tyr Ser Glu Ile Gly Thr Lys Gly Glu
500 505 510

Arg Arg Arg Gly Lys Gly His Asp Gly Leu Tyr Gln Gly Leu Ser Thr
515 520 525

Ala Thr Lys Asp Thr Tyr Asp Ala Leu His Met Gln Thr Leu Ala Pro
530 535 540

Arg Glu Phe Gly Ser Gly Val Lys Gln Thr Leu Asn Phe Asp Leu Leu
545 550 555 560

Lys Leu Ala Gly Asp Val Glu Ser Asn Pro Gly Pro Cys Met Phe His
565 570 575

Val Ser Phe Arg Tyr Ile Phe Gly Ile Pro Pro Leu Ile Leu Val Leu
580 585 590

Leu Pro Val Thr Ser Ser Glu Cys His Ile Lys Asp Lys Glu Gly Lys
595 600 605

Ala Tyr Glu Ser Val Leu Met Ile Ser Ile Asp Glu Leu Asp Lys Met
610 615 620

Thr Gly Thr Asp Ser Asn Cys Pro Asn Asn Glu Pro Asn Phe Phe Arg
625 630 635 640

Lys His Val Cys Asp Asp Thr Lys Glu Ala Ala Phe Leu Asn Arg Ala
645 650 655

Ala Arg Lys Leu Lys Gln Phe Leu Lys Met Asn Ile Ser Glu Glu Phe
660 665 670

Asn Val His Leu Leu Thr Val Ser Gln Gly Thr Gln Thr Leu Val Asn
675 680 685

Cys Thr Ser Lys Glu Glu Lys Asn Val Lys Glu Gln Lys Lys Asn Asp
690 695 700

Ala Cys Phe Leu Lys Arg Leu Leu Arg Glu Ile Lys Thr Cys Trp Asn
705 710 715 720

Lys Ile Leu Lys Gly Ser Ile Gly Ser Gly Val Lys Gln Thr Leu Asn
725 730 735

Phe Asp Leu Leu Lys Leu Ala Gly Asp Val Glu Ser Asn Pro Gly Pro
740 745 750

Met Ala Pro Arg Val Thr Pro Leu Leu Ala Phe Ser Leu Leu Val Leu
755 760 765

Trp Thr Phe Pro Ala Pro Thr Leu Gly Gly Ala Asn Asp Ala Glu Asp
770 775 780

Cys Cys Leu Ser Val Thr Gln Arg Pro Ile Pro Gly Asn Ile Val Lys
785 790 795 800

Ala Phe Arg Tyr Leu Leu Asn Glu Asp Gly Cys Arg Val Pro Ala Val
805 810 815

Val Phe Thr Thr Leu Arg Gly Tyr Gln Leu Cys Ala Pro Pro Asp Gln
820 825 830

Pro Trp Val Asp Arg Ile Ile Arg Arg Leu Lys Lys Ser Ser Ala Lys
835 840 845

Asn Lys Gly Asn Ser Thr Arg Arg Ser Pro Val Ser Arg Gly Ser Gly
850 855 860

Val Lys Gln Thr Leu Asn Phe Asp Leu Leu Lys Leu Ala Gly Asp Val
865 870 875 880

Glu Ser Asn Pro Gly Pro Met Thr Trp Ala Pro Leu Leu Leu Ile Phe
885 890 895

Leu Gln His Leu Arg Gly Ser Cys Ala Gln Phe Val Leu Thr Gln Pro
900 905 910

Lys Thr Val Ser Glu Ser Leu Gly Arg Thr Val Thr Ile Ser Cys Lys
915 920 925

Arg Ser Ser Gly Ser Val Gly Asp Tyr Tyr Val Ser Trp His Gln Gln
930 935 940

```
Arg Phe Gly Ser Ser Pro Lys Thr Val Ile Tyr Leu Asp Asp Glu Arg
945                 950                 955                 960

Pro Ser Gly Val Pro Lys Arg Phe Ser Gly Ser Ile Asp Ser Ser Ser
                965                 970                 975

Asn Ser Ala Ser Leu Thr Ile Thr Asp Leu Gln Thr Asp Asp Glu Ala
            980                 985                 990

Asp Tyr Phe Cys Leu Ser Tyr Asp  Ser Asn Asn His Phe  Val Phe Gly
        995                 1000                 1005

Ser Gly  Thr His Leu Thr Val  Leu Gly Gly Gly Gly  Ser Gly Gly
    1010                 1015                 1020

Gly Gly  Ser Gly Gly Gly Gly  Ser Glu Val Gln Leu  Val Glu Ser
    1025                 1030                 1035

Gly Gly  Gly Leu Ala Gln Pro  Gly Lys Ser Leu Lys  Leu Ser Cys
    1040                 1045                 1050

Glu Ala  Ser Gly Phe Thr Phe  Ser Asp Tyr Gly Met  Asn Trp Phe
    1055                 1060                 1065

Arg Gln  Ala Pro Gly Lys Gly  Leu Glu Trp Val Ala  Tyr Ile Ser
    1070                 1075                 1080

Ser Gly  Ser Asn Lys Ile Thr  Tyr Ala Asp Thr Val  Lys Gly Arg
    1085                 1090                 1095

Phe Thr  Val Ser Arg Asp Asn  Gly Lys Asn Gln Leu  Phe Leu Gln
    1100                 1105                 1110

Met Asn  Asn Leu Lys Ser Glu  Asp Thr Ala Ile Tyr  Tyr Cys Val
    1115                 1120                 1125

Asp Ser  Gly Phe Asn Ser Tyr  Ser Asp Val Trp Gly  Gln Gly Ile
    1130                 1135                 1140

Gln Val  Thr Val Ser Ser Asp  Tyr Lys Asp Asp Asp  Asp Lys Phe
    1145                 1150                 1155

Val Glu  His His His His His  His
    1160                 1165
```

## Description

Field of Invention

The present disclosure relates to a drug for use in treatment of a cancer, a combination drug, a pharmaceutical composition, an immunoresponsive cell, a nucleic acid delivery vehicle, and a product.

Background Art

Cancer is also referred to as "malignant neoplasm", and treatment thereof is one big target in medical science. Treatment using a radiation or a chemotherapeutic anticancer drug has been carried out so far, but the efficacy thereof greatly varies with the types of cancers, and high efficacy is not obtained against all cancers.

CAR-T cell-based therapy has recently been developed which uses CAR-T cells obtained by modifying T cells so as to enable the T cells to produce a special protein referred to as "Chimeric Antigen Receptor (CAR)", using a genetic medicine technique. For example, CAR-T cell-based therapy has been shown to be effective against recurrent or refractory CD19-positive B-cell acute lymphoblastic leukemia (B-ALL) and recurrent or refractory CD19-positive diffuse large B-cell lymphoma (DLBCL). International Publication (WO) No. 2017/159736 describes that interleukine-7 (IL-7) and chemokine (C-C motif) ligand 19 (CCL19) are allowed to express in immunocompetent cells expressing a cell surface molecule that specifically recognizes a cancer antigen, such as CAR-T cells, thereby enhancing the antitumor activity thereof. International Publication (WO) No. 2019/073973 describes enhanced T cells or B cells that contain a nucleic acid delivery vehicle, a nucleic acid encoding IL-7, and a nucleic acid encoding CCL19 and that have a memory function in a subject to which administration is performed, and an inducer that induces a memory function of T cells or B cells in a subject to which administration is performed.

In the treatment of a cancer, plural anticancer drugs are often used together with a view to enhancing anticancer effects and to alleviating side-effects by reducing the dose.

For example, Japanese Patent National-Phase Publication (JP-A) No. 2018-538339 describes a composition and a method for treatment of a disease associated with mesothelin expression, including administering cells that express a mesothelin-specific CAR in combination with an anti-PD-L1 antibody, from the viewpoint of improving the efficacy of treatment in which CAR-T cells are used.

SUMMARY OF INVENTION

Problem to be Solved by Invention

Combined use of anti-cancer drugs produces a synergistic effect in some cases, but mutually inhibit their effects in other cases, depending on the combination. Therefore, a useful combination for combined use is being searched for.

In consideration of the foregoing circumstances, the present disclosure aims to provide a drug for use in treatment of a cancer, a combination drug, a pharmaceutical composition, an immunoresponsive cell, a nucleic acid delivery vehicle, and a product, with which a high anti-cancer effect is obtained.

Means for Solving the Problem

The present disclosure includes the following aspect.

A combination drug for use in treatment of a cancer in a subject, including:

(a) an immunoresponsive cell expressing interleukine-7 (IL-7), chemokine (C-C motif) ligand 19 (CCL19), and a cell surface molecule that specifically recognizes a cancer antigen; and
(b) an immunosuppression inhibitor.

The present disclosure also includes the following aspect.

A combination drug for use in treatment of a cancer in a subject, including:

(a) one or more kinds of cells, one or more kinds of nucleic acid delivery vehicles, or a combination thereof, which cooperatively include a nucleic acid encoding interleukine-7 and a nucleic acid encoding CCL19, and a cell surface molecule that specifically recognizes a cancer antigen; and
(b) an immunosuppression inhibitor.

The present disclosure also includes the following aspect.

An immunoresponsive cell expressing interleukine-7, CCL19, an immunosuppression inhibiting polypeptide, and a cell surface molecule that specifically recognizes a cancer antigen.

Advantageous Effect of Invention

According to the present disclosure, a drug for use in treatment of a cancer, a combination drug, a pharmaceutical composition, an immunoresponsive cell, a nucleic acid delivery vehicle, and a product, with which a high anti-cancer effect is obtained, are provided.

BRIEF DESCRIPTION OF THE DRAWINGS

Fig. 1A is a figure showing a map of pMSGV vector that includes eGFP but includes neither IL-7 nor CCL19, and that is described in Examples (hereinafter also referred to as "eGFP-Conv. vector"; a vector obtainable by removing an IL-7 encoding region and a CCL19-encoding region from pMSGV vector containing IL-7-F2A-CCL19-F2A-eGFP DNA fragment (SEQ ID NO:. 10)).

Fig. 1B is a figure showing a map of pMSGV vector that includes IL-7-F2A-CCL19-F2A-eGFP DNA fragment (SEQ ID NO: 10), and that is described in Examples (hereinafter also referred to as "7×19 expression vector").

Fig. 2 is a scatter diagram showing results of measurement of eGFP expression amount and CD8 expression amount by flow cytometry in each of: TCR-T cells to which a vector has not been introduced (specifically, mouse T cells that have been obtained from spleen cells, and that express a TCR specific to P815 tumor antigen P1A, and to which a vector has not been introduced; hereinafter referred to as "vector unintroduced P1A-TCRT cells". Mouse T cells expressing PIA-specific TCR will be collectively referred to as "PIA-TCRT cells", regardless of whether a vector has been introduced or not.); P1A-TCRT cells to which eGFP-Conv. vector has been introduced (hereinafter also referred to as "eGFP-expressing P1A-TCRT cells"); and P1A-TCRT cells to which 7×19 expression vector has been introduced (hereinafter also referred to as "eGFP-expressing P1A-7×19 TCRT cells").

Fig. 3A is a graph showing results of measurement of IL-7 expression amount by ELISA in each of: vector unintroduced P1A-TCRT cells, eGFP-expressing P1A-TCRT cells, and eGFP-expressing P1A-7×19 TCRT cells.

Fig. 3B is a graph showing results of measurement of CCL19 expression amount by ELISA in each of: vector unintroduced P1A-TCRT cells, eGFP-expressing P1A-TCRT cells, and eGFP-expressing P1A-7×19 TCRT cells.

Fig 4 is a graph showing a relationship between lapsed days and viability in the case of subcutaneously injecting mastocytoma cells (P815) into DBA/2 mice, irradiating the DBA/2 mice with a radiation, and thereafter administering vector unintroduced P1A-TCRT cells or eGFP-expressing P1A-TCRT cells, and/or anti-PD-1 monoclonal antibody to the DBA/2 mice in an Example.

Fig. 5A is a graph showing a relationship between lapsed days and tumor volume in the case of subcutaneously injecting mastocytoma cells (P815) into DBA/2 mice, irradiating the DBA/2 mice with a radiation, and thereafter performing no treatment in an Example.

Fig. 5B is a graph showing a relationship between lapsed days and tumor volume in the case of subcutaneously injecting mastocytoma cells (P815) into DBA/2 mice, irradiating the DBA/2 mice with a radiation, and thereafter administering an anti-PD-1 monoclonal antibody to the DBA/2 mice in an Example.

Fig. 5C is a graph showing a relationship between lapsed days and tumor volume in the case of subcutaneously injecting mastocytoma cells (P815) into DBA/2 mice, irradiating the DBA/2 mice with a radiation, and thereafter administering eGFP-expressing P1A-TCRT cells to the DBA/2 mice in an Example.

Fig. 5D is a graph showing a relationship between lapsed days and tumor volume in the case of subcutaneously injecting mastocytoma cells (P815) into DBA/2 mice, irradiating the DBA/2 mice with a radiation, and thereafter administering eGFP-expressing P1A-TCRT cells and an anti-PD-1 monoclonal antibody to the DBA/2 mice in an Example.

Fig. 5E is a graph showing a relationship between lapsed days and tumor volume in the case of subcutaneously injecting mastocytoma cells (P815) into DBA/2 mice, irradiating the DBA/2 mice with a radiation, and thereafter administering eGFP-expressing P1A-7×19 TCRT cells to the DBA/2 mice in an Example.

Fig. 5F is a graph showing a relationship between lapsed days and tumor volume in the case of subcutaneously injecting mastocytoma cells (P815) into DBA/2 mice, irradiating the DBA/2 mice with a radiation, and thereafter administering eGFP-expressing P1A-7×19 TCRT cells and an anti-PD-1 monoclonal antibody to the DBA/2 mice in an Example.

Fig 6A is a graph showing a relationship between lapsed days and viability in the case of subcutaneously injecting mastocytoma cells (P815) into DBA/2 mice, irradiating the DBA/2 mice with a radiation, and thereafter administering/not administering eGFP-expressing P1A-7×19 TCRT cells of which PD-1 gene or ROSA26 gene has been knocked down (disrupted) to the DBA/2 mice and administering/not administering an anti-PD-1 monoclonal antibody to the DBA/2 mice in Examples.

Fig. 6B is a graph showing a relationship between lapsed days and tumor volume in the case of administering neither the P1A-TCRT cells nor the anti-PD-1 monoclonal antibody in the experiment of Fig. 6A.

Fig. 6C is a graph showing a relationship between lapsed days and tumor volume in the case of administering eGFP-expressing P1A-7×19 TCRT cells of which ROSA26 gene has been knocked down in the experiment of Fig. 6A.

Fig. 6D is a graph showing a relationship between lapsed days and tumor volume in the case of administering eGFP-expressing P1A-7×19 TCRT cells of which PD-1 gene has been knocked down in the experiment of Fig. 6A.

Fig. 6E is a graph showing a relationship between lapsed days and tumor volume in the case of administering eGFP-expressing P1A-7×19 TCRT cells of which PD-1 gene has been knocked down and an anti-PD-1 monoclonal antibody in the experiment of Fig. 6A.

Fig. 7 is a graph determining, based on flow cytometry measurement of CD8 expression amount and eGFP expression amount, whether or not eGFP-expressing P1A-TCRT cells or eGFP-expressing P1A-7×19 TCRT cells which express both of eGFP and CD8 are present in spleen cells after complete tumor regression following administration of eGFP-expressing P1A-TCRT cells or eGFP-expressing P1A-7×19 TCRT cells to DBA/2 mice that have been inoculated with cancer cells in Examples.

Fig. 8A is a graph showing the proportion of T cells that do not express CD8 but express eGFP, and the proportion of T cells that express both of CD8 and eGFP in spleen cells obtained in the experiment of Fig. 7.

Fig. 8B is a graph showing the number of T cells that do not express CD8 but express eGFP, and the number of T cells that express both of CD8 and eGFP in spleen cells obtained in the experiment of Fig. 7.

Fig. 8C is a graph showing a relationship between lapsed days and the number of T cells expressing both of CD8 and eGFP in the case of further co-culturing spleen cells obtained in the experiment of Fig. 7 with P815 tumor cells.

Fig. 8D is a graph showing a relationship between lapsed days and the concentration of IFN-γ in the conditioned medium in the case of further co-culturing spleen cells obtained in the experiment of Fig. 7 with P815 tumor cells.

Fig. 9 is a graph showing a relationship between lapsed days and tumor volume in each of the case of inoculating P815 tumor cells again after complete tumor regression in the experiment of Fig. 5F and the case of inoculating P815 tumor cells into naive DBA/2 mice.

Fig. 10A is a graph showing a relationship between lapse of time and viability in the case of inoculating P815 tumor cells (P815-hCD20 tumor cells) expressing human CD20 (hCD20) into DBA/2 mice, and then intraperitoneally administering cyclophosphamide, and then further performing/not performing intravenous injection of anti-hCD20-CAR-expressing T cells or anti-hCD20 CAR-IL-7/CCL19-expressing T cells, and then administering/not administering control IgG or an anti-PD-1 monoclonal antibody.

Fig. 10B is a graph showing a relationship between lapse of time and tumor volume in the case of performing neither the administration of the CAR-T nor the administration of the antibody in the experiment of Fig. 10A.

Fig. 10C is a graph showing a relationship between lapse of time and tumor volume in the case of not performing the administration of the CAR-T but administering the anti-PD-1 monoclonal antibody in the experiment of Fig. 10A.

Fig. 10D is a graph showing a relationship between lapse of time and tumor volume in the case of administering the anti-hCD20-CAR-expressing T cells and administering the anti-PD-1 antibody in the experiment of Fig. 10A.

Fig. 10E is a graph showing a relationship between lapse of time and tumor volume in the case of administering the anti-hCD20-CAR-IL-7/CCL19-expressing T cells and administering the control IgG in the experiment of Fig. 10A.

Fig. 10F is a graph showing a relationship between lapse of time and tumor volume in the case of administering the anti-hCD20-CAR-IL-7/CCL19-expressing T cells and administering the anti-PD-1 monoclonal antibody in the experiment of Fig. 10A.

Fig. 11A shows scatter diagrams showing results of analysis of the composition of tumor infiltrating lymphocytes (TIL) based on expressions of c-kit, CD11c, CD3, eGFP, CD4, and CD8 in the case of inoculating P815 tumor cells into DBA/2 mice, and intravenously injecting eGFP-expressing P1A-TCRT cells or eGFP-expressing P1A-7×19 TCRT cells on day 7.

Fig. 11B is a graph showing the number of cells of each type of immune cells obtained in the experiment of Fig. 11A.

Fig. 12 is a conceptual diagram illustrating a vector encoding a CAR and anti-mouse-PD-1 scFv and a vector encoding a CAR, IL-7, CCL19, and anti-mouse-PD-1 scFv, which are used in Examples.

Fig. 13 shows graphs showing forward scattering and side scattering obtained by flow cytometry of T cells that have not undergone transduction and T cells transduced with various CAR-containing constructs, which are used in Examples, and graphs showing the proportion of CAR-expressing cells measured using protein L-bio and sav-apc.

Fig. 14 shows graphs showing results of measurement by ELISA of IL-7 and CCL19 concentrations in conditioned medium of T cells that have not undergone transduction and in conditioned media of T cells transduced with various CAR-containing constructs, which are used in Examples.

Fig. 15 shows graphs showing results of measurement by ELISA of anti-PD-1 antibody concentration in conditioned medium of T cells that have not undergone transduction and in conditioned media of T cells transduced with various CAR-containing constructs, which are used in Examples.

Fig. 16 shows a graph showing a relationship between lapsed days and viability in the case of subcutaneously injecting P815 tumor cells expressing human CD20 into DBA/2 mice, intraperitoneally administering cyclophosphamide, and then administering T cells that have not undergone transduction and T cells transduced with various CAR-containing constructs in Examples, and a table indicating P values among experimental groups according to log-rank test.

Fig. 17 shows graphs showing a relationship between lapsed days and tumor volume in respective experimental groups in the experiment of Fig. 16.

## MODES FOR CARRYING OUT THE INVENTION

The contents of the present disclosure will be described in detail below. In the following, explanations of constituent elements may be made based on representative embodiments of the present disclosure. However, the present disclosure is not limited to such embodiments.

For numerical ranges described in a stepwise manner in the present disclosure, the upper limit value or the lower limit value of one numerical range may be replaced by the upper limit value or the lower limit value of another numerical range in the stepwise description. The upper limit value or the lower limit value of any numerical range described in the present disclosure may also be replaced by a value described in Examples.

In a case in which plural substances corresponding to a component of interest are present in a composition, the amount of the component in the composition described in the present disclosure means the total amount of the plural substances present in the composition, unless otherwise specified.

In the present disclosure, the terms " mass%" and " weight%" are used synonymously, and the terms "parts by mass" and "parts by weight" are used synonymously.

In the present disclosure, two or more exemplary aspects may be combined with each other as long as contradiction does not arise.

According to an aspect of the present disclosure, a combination drug for use in treatment of a cancer in a subject is provided (hereinafter also referred to as "combination drug A according to the present disclosure") which includes: (a) an immunoresponsive cell expressing interleukine-7 (IL-7), CCL19, and a cell surface molecule that specifically recognizes a cancer antigen; and (b) an immunosuppression inhibitor.

The inventors of the present invention have carried out a study regarding a combination of anti-cancer drugs that is useful in a method of treating a cancer, and have found that a remarkably high therapeutic effect against cancer can be obtained using the combination drug A according to the present disclosure. Specifically, in the combination drug A according to the present disclosure, an immunoresponsive cell expressing IL-7, CCL19, and a cell surface molecule that specifically recognizes a cancer antigen (hereinafter also referred to as "immunoresponsive cell A according to the present disclosure") is used. The present inventors have found, for the first time, that a remarkably high synergistic effect is obtained by co-administration of the immunoresponsive cell A according to the present disclosure and an immunosuppression inhibitor.

The cell surface molecule that specifically recognizes a cancer antigen and that is present on a cell surface of the immunoresponsive cell A according to the present disclosure specifically binds to the cancer antigen expressed on a cancer cell. As a result of this binding, one or more events selected from tethering of the immunoresponsive cell to the cancer cell, triggering of intracellular signal transduction, or the like occur, to start an attack against the cancer cell in a person suffering from a cancer (hereinafter referred to as a "cancer-suffering person"). In the present disclosure, the term "recognize" is used interchangeably with the term "bind".

Since cancer cells have an immunosuppressive mechanism that suppresses immunoresponsive cells' action to attack against the cancer cells or to send an instruction to attack the cancer cells, attack against the cancer cells by the immune system of the cancer-suffering person himself/herself is suppressed. It is conceivable that the immunosuppression inhibitor, which is one component of the combination drug A according to the present disclosure, inhibits the immunosuppressive mechanism provided by the cancer cells, thereby making it easier for the immune system of the cancer-suffering person to attack the cancer cells.

In addition, since the immunoresponsive cell A according to the present disclosure also expresses IL-7 and CCL19, not only the immunoresponsive cell A according to the present disclosure, but also endogenous immunoresponsive cells of the cancer-suffering person accumulate around the cancer cells, as a result of which more effective attack against the cancer cells is enabled.

It is conceivable that, by including an immunoresponsive cell expressing IL-7, CCL19, and a cell surface molecule that specifically recognizes a cancer antigen, and an immunosuppression inhibitor, the combination drug A according to the present disclosure exerts a synergistic effect due to the combination of factors consisting of the immunoresponsive cell expressing a cell surface molecule that specifically recognizes a cancer antigen, secreted IL-7 and CCL19, and the immunosuppression inhibitor, and the combination drug A according to the present disclosure thereby exerts a remarkably improved cancer therapeutic effect. This synergistic effect is so superior as to be unable to predict from the individual effects of respective factors.

For example, even in the case of treatment of a cancer that is difficult to treat with an immunoresponsive cell expressing interleukin-7, CCL19, and a cell surface molecule that specifically recognizes a cancer antigen alone, or with an immunosuppression inhibitor alone, such a cancer can be treated with the combination drug A according to the present disclosure. This point is indicated by the experimental results concerning the immunoresponsive cell C according to the present disclosure that will be described later. Further, such a high therapeutic effect makes it possible to obtain a therapeutic effect even with a reduced cell dose, which makes it possible to obtain a therapeutic effect even in a case in which the number of immunoresponsive cells conventionally required for making use of autologous cells cannot be collected. These effects are not expected from solo administration of the immunoresponsive cell expressing interleukin-7, CCL19, and a cell surface molecule that specifically recognizes a cancer antigen or from solo administration of the immunosuppression inhibitor.

<Cell Surface Molecule That Specifically Recognizes Cancer Antigen>

The cell surface molecule that specifically recognizes a cancer antigen in the present disclosure is a molecule that specifically binds to a cancer antigen, and may be any of a polypeptide, a nucleic acid such as an aptamer, or another molecule as long as the molecule specifically binds to a cancer antigen. Here, the "cancer antigen" means a substance, such as a protein or a glycolipid, that exhibits higher expression in cancer cells than that in normal cells, or that are expressed specifically in cancer cells. Examples of the cancer antigen include tumor-associated antigens, cancer-testis antigens, angiogenesis-associated antigens, and epitope peptides of cancer neoantigens resulting from genetic mutations.

Specific examples of cancer antigens that are specifically recognized by cell surface molecules include proteins such as WT1, MART-1, NY-ESO-1, MAGE-A1, MAGE-A3, MAGE-A4, MAGE-A10, Glypican-3, KIF20A, Survivin, AFP, gp100, MUC1, DLL3, PRSS21, Nectin4, FAP, integrin β7, CT-83(KK-LC-1), KRAS (including mutants, i.e., mKRAS), Epha2, PRAME, HA-1, PAP-10, PAP-5, TRP2-1, SART-1, VEGFR1, VEGFR2, NEIL3, MPHOSPH1, DEPDC1, FOXM1, CDH3, TTK, TOMM34, URLC10, KOC1, UBE2T, TOPK, ECT2, MESOTHELIN, NKG2D, and P1A, and glycolipids such as GD2 and GM2. Further examples include, but are not limited to, CD20, EGFR (such as EGFRvIII), EGFR variants, FITC, CD19, CD22, CD33, PSMA, ROR1, c-Met, HER2, CEA, CD7, CD10, CD30, CD34, CD38, CD41, CD44, CD74, CD123, CD133, CD171, CD180, MUC16, CS1(CD319), IL-13Ra2, BCMA, LewisY, IgG κ chain, folate receptor α, PSCA, EpCAM, CAIX, CDS, CD49f, CD56, CD138, IGF1R, cytomegalovirus (CMV)-infected cell antigen, EGP-2, EGP-40, ERB-B2, ERB-B3, ERB-B4, FBP, fetal acetylcholine receptor, GD3, HER-2, hTERT, K-light chain, LeY, L1 cell adhesion molecule, NKG2D ligand, 5T4, and TAG-72. The cell surface molecule may include one or more of these cell surface molecules. The origin organism of these antigens may be the same organism as the organism to be treated with the combination drug A according to the present disclosure, and the organism is, for example, human.

Examples of the cell surface molecule that specifically recognizes a cancer antigen include cell surface receptors, artificial receptors, and adhesion factors that specifically recognize a cancer antigen. The cell surface molecule that specifically recognizes a cancer antigen may be a cell surface molecule that performs only a function of binding to a cancer antigen and thereby positioning the immunoresponsive cell A according to the present disclosure in the vicinity of a cancer cell, or may be a cell surface molecule that also has a function of triggering intracellular signal transduction that activates an immune response of the immunoresponsive cell so as to further enhance the therapeutic effect against cancer. The cell surface molecule that specifically recognizes a cancer antigen may be an antibody or antibody fragment that specifically recognizes a cancer antigen. The antibody or antibody fragment is not limited to IgM, IgD, IgG, IgA, IgE, or the like, and may be a low-molecular-weight antibody such as Fab or scFv.

The cell surface molecule that specifically recognizes a cancer antigen is, for example, a molecule that provides the cell with the ability to specifically recognize a cancer when the molecule is expressed on the cell surface, such as a T-cell receptor (TCR) that specifically recognize a cancer antigen, or a chimeric antigen receptor (CAR) that specifically recognize a cancer antigen. It can be said that a TCR is an example of the cell surface receptor, CAR is an example of the artificial receptor, and an antibody (including a low-molecular-weight antibody such as Fab, Fab', $F(ab')_2$, or scFv) is an example of the adhesion factor. Of course, the adhesion factor may alternatively be a molecule other than an antibody, such as a sugar chain or an aptamer, as far as the molecule specifically recognizes a cancer antigen. These cell surface molecules specifically recognize a cancer antigen, thereby enabling the immunoresponsive cell A according to the present disclosure to localize around a cancer cell.

A TCR is an antigen receptor molecule expressed on a cell membrane of a T cell. A TCR is present in the form of a heterodimer consisting of an alpha chain and a beta chain, or of a gamma chain and a delta chain, and it is known that a TCR activates a T cell upon recognition of an antigen molecule bonded to a major histocompatibility complex (MHC). The TCR may be either a heterodimer consisting of an alpha chain and a beta chain (an alpha-beta TCR) or a heterodimer consisting of a gamma chain and a delta chain (a gamma-delta TCR), as far as the TCR specifically recognizes a cancer antigen.

The TCR may be an endogenous TCR or an exogenous TCR (recombinant TCR). Examples of a source of a T cell that expresses an endogenous TCR or of a T cell to which an exogenous TCR is to be genetically introduced include, but are not limited to, a tumor infiltrating lymphocyte (TIL), a tumor regional lymph node, a peripheral blood lymphocyte, a lymphocyte in pleural fluid, and a lymphocyte in ascitic fluid.

Examples of methods for the separation of T cells that express a TCR having a given antigen binding property include, but are not limited to: density gradient centrifugation; resetting; coupling to a particle for changing the cell density; magnetic separation using antibody-coated magnetic beads; affinity chromatography (for example, affinity chromatography using negative selection); a cytotoxic agent (examples of which include, but are not limited to, complements and cytotoxins) linked to a monoclonal antibody or used in combination with a monoclonal antibody; panning using an antibody bound to a solid matrix such as a plate or a tip; elutriation; selective proliferation due to antigen stimuli; and separation utilizing a complex of MHC and an antigen.

Transgenic animals, such as transgenic mice, that have been modified to express a particular TCR have also been developed.

A CAR is an artificial chimeric protein obtainable by fusing a single chain antibody that recognizes a cell surface antigen of a cancer cell and a signaling region that induces activation of a T cell. The CAR may include, for example, a single chain antibody region that recognizes a cell surface antigen of a cancer cell, a cell membrane penetrating region, and a signaling region that induces activation of a T cell. When the cell surface molecule that specifically recognizes a cancer antigen in the combination drug A according to the present disclosure is a CAR, an equivalent or higher effect can be obtained even in a case in which the immunoresponsive cell A according to the present disclosure administered as a component of the combination drug A is present in a number that is smaller than the number of CAR-T cells (usually, at least $1 \times 10^6$ cells) administered in conventional methods in which CAR-T cells are used alone.

The single chain antibody (scFv) in the CAR is, for example, an oligopeptide or polypeptide that includes a light chain variable region and a heavy chain variable region derived from an antigen binding site of a monoclonal antibody, and in which a linker peptide is present between the light chain variable region and the heavy chain variable region.

The single chain antibody that recognizes a cancer antigen of interest can be prepared using known methods. For example, a lymphoid tissue may be collected after inoculating an antigen into a mouse or the like, a library of antibody genes may be prepared therefrom, a base sequence encoding an antibody that recognizes a cancer antigen may be obtained by antibody direct cloning, and a single chain antibody may be designed based on the base sequence. Alternatively, hybridomas may be prepared using the collected lymphoid tissue, a hybridoma encoding an antibody that recognizes a cancer antigen may be identified to obtain a monoclonal antibody, and a single chain antibody may be designed based on the sequence information of the monoclonal antibody. Alternatively, a library of single chain antibodies may be prepared based on, for example, a naive antibody library prepared from B cells of a normal person or an antibody library prepared from B cells of a cancer-suffering person having an antiserum exhibiting high neutralization activity against a cancer antigen, the library of single chain antibodies may be displayed by phage display, and a single chain antibody that recognizes a cancer antigen may be selected therefrom.

The immunoresponsive cell activating signaling region is a region capable of transducing a signal into the cell when the single chain antibody has recognized a cell surface antigen of a cancer cell. The immunoresponsive cell activating signaling region may include at least one, or two or more, selected from polypeptides of intracellular regions of CD28, 4-1BB (CD137), GITR, CD27, OX40, HVEM, CD3ζ, and Fc Receptor-associated γ chain, and may include polypeptides of three kinds of intracellular regions of CD28, 4-1BB, and CD3ζ.

Polypeptides of respective intracellular regions may be linked via an oligopeptide linker consisting of 2 to 10 amino acids or via a polypeptide linker. The sequence of the linker is, for example, a glycine-serine repeating sequence.

Activation of an immunoresponsive cell means initiation of an immune response based on intracellular signal transduction or induction of a change of protein expression. For example, a signal transduction cascade is formed when CD3 chains gather in response to ligand binding and immunoreceptor tyrosine-based inhibitory motif (ITAMs). Further, binding of an endogenous TCR or an exogenous CAR to an antigen may induce formation of an immunological synapse that includes assembly of a lot of molecules, in the vicinity of receptors (for example, CD4 or CD8, CD3γ/δ/ε/ζ, or the like) involved in the binding. Due to the assembly of the membrane-associated signaling molecules, the ITAM motif contained in a CD3 chain is phosphorylated. The phosphorylation, in turn, initiates immunoresponsive cell activation pathway, and may eventually activate transcriptional factors such as NF-κB and AP-1. These transcriptional factors trigger, for example, gross gene expression of the T cell to initiate a T-cell-mediated immune response, thereby increasing the production of IL-2 for the proliferation of main regulatory T cells and expression of proteins thereof.

Examples of the cell membrane penetrating region of the CAR include a polypeptide of a cell membrane penetrating region coming from any of CD8, α-chain or β-chain of a T cell receptor, CD28, CD3ε, CD45, CD4, CD5, CD8, CD9, CD16, CD22, CD33, CD37, CD64, CD80, CD86, CD134, CD137, CD154, or GITR. The cell membrane penetrating region may be, for example, the polypeptide of human CD8 cell membrane penetrating region. The cell membrane penetrating region fixes the CAR to the cell membrane of a T cell.

The cell membrane penetrating region may include a hinge region having a length of from 1 to 100 amino acid, more particularly 10 to 70 amino acids, and formed by a freely-selected oligopeptide or polypeptide. The hinge region is, for example, the hinge region of human CD8.

A spacer region consisting of a freely-selected oligopeptide or polypeptide may be provided between the single chain antibody that recognizes a cell surface antigen of a cancer cell and the cell membrane penetrating region, or between the cell membrane penetrating region and the immunoresponsive cell activating signaling region. The length of the spacer region is, for example, from 1 to 100 amino acids, more particularly from 10 to 50 amino acids. An example of the spacer region is a glycine-serine repeating sequence.

The amino acid sequence of the cell surface molecule that specifically recognizes a cancer antigen is, for example, an amino acid sequence from a mammalian animal, and may be an amino acid sequence from a human from the viewpoint of reducing rejection upon administration to a human. The amino acid sequence can be obtained, as desired, by searching known publications or database such as NCBI (http://www.ncbi.nlm.nih.gov/guide/). The cell surface molecule that specifically recognizes a cancer antigen may be a human TCR or a CAR in which the single chain antibody region is humanized.

The cell surface molecule that specifically recognizes a cancer antigen may be a cell surface molecule that indirectly recognize a cancer antigen as long as the recognition of the cancer antigen is specific. For example, in a case in which a molecule, such as an antibody, that specifically recognize a cancer antigen is administered to the subject simultaneously with or in series with administration of the immunoresponsive cell A according to the present disclosure, the immunoresponsive cell A according to the present disclosure may specifically recognize the cancer antigen in an indirect manner by recognizing the molecule such as an antibody or by recognizing a tag attached as a label to the molecule such as an antibody. In these cases, in an example in which the immunoresponsive cell A recognizes an antibody, the cell surface molecule is, for example, CD16, and the tag attached as a label to the molecule such as an antibody is, for example, FITC.

<IL-7>

IL-7 is a cytokine of about 25 kDa produced by a stroma cell derived from bone marrow or the thymus as structural origin. IL-7 causes emission of a signal that promotes, via an IL-7 receptor, differentiation from hematopoietic stem cells to lymphoid progenitor cells, to generate T cells, B, cells, and NK cells. The amino acid sequence of the IL-7 is, for example, an amino acid sequence from a mammalian animal, and may be an amino acid sequence from a human from the viewpoint of reducing the rejection. The amino acid sequence can be obtained, as desired, by searching known publications or database such as NCBI (http://www.ncbi.nlm.nih.gov/guide/).

<CCL19>

Chemokine (C-C motif) ligand 19 (CCL19) is a cytokine belonging to the CC chemokine family, and exhibits high expression in the thymus and lymph nodes. The amino acid sequence of the CCL19 is, for example, an amino acid sequence from a mammalian animal, and may be an amino acid sequence from a human from the viewpoint of reducing the rejection. The amino acid sequence can be obtained, as desired, by searching known publications or database such as NCBI (http://www.ncbi.nlm.nih.gov/guide/).

<Immunoresponsive Cell A according to the Present Disclosure>

Immunoresponsive cells refer to cells involved in immune responses. Examples of immunoresponsive cells include lymphocytic cells such as T cells, natural killer cells (NK cells), and B cells, antigen-presenting cells such as monocytes, macrophages, and dendritic cells, and granulocytes such as neutrophils, eosinophils, basophils, and mast cells. Examples of T cells include alpha-beta T cells, gamma-delta T cells, $CD8^+$ T cells, $CD4^+$ T cells, tumor infiltrating T cells, memory T cells, naive T cells, and natural killer T (NKT) cells.

Immunoresponsive cells can be obtained by isolation and purification from body fluid such as blood or bone marrow aspirate, or from immune cells that infiltrate into a tissue such as the spleen, the thymus, or lymph nodes, or from immune cells that infiltrate into a cancer tissue such as primary tumor, metastatic tumor, or cancerous ascites. It is also possible to use immunoresponsive cells prepared from pluripotent stem cells such as iPS cells or ES cells, or from somatic stem cells such as hematopoietic stem cells.

The immunoresponsive cell may be a T cell derived from a mammalian animal such as a human, a dog, a cat, a pig, or a mouse, and may be a T cell derived from a human.

The immunoresponsive cell A according to the present disclosure expresses IL-7, CCL19, and a cell surface molecule that specifically recognizes a cancer antigen. Here, the expression, "expresses IL-7, CCL19, and a cell surface molecule that specifically recognizes a cancer antigen", refers to a situation in which IL-7, CCL19, and a cell surface molecule that specifically recognizes a cancer antigen are produced by the immunoresponsive cell, so that at least some of cell surface molecules that specifically recognize a cancer antigen are located on a cell surface (a cell surface at the outer side of the cell), and so that IL-7 and CCL19 are secreted to the outside of the cell.

The immunoresponsive cell A according to the present disclosure can be obtained by introducing a gene encoding a cell surface molecule that specifically recognizes a cancer antigen, a gene encoding IL-7, and a gene encoding CCL19 into, for example, an immunoresponsive cell collected from a living body or an immunoresponsive cell induced from a pluripotent stem cell such as an iPS cell or an ES cell, or induced from a somatic stem cell such as a hematopoietic stem cell. The immunoresponsive cell A according to the present disclosure can alternatively be obtained by collecting an immunoresponsive cell that inherently expresses a cell surface molecule that specifically recognizes a cancer antigen (for example a TCR that specifically recognizes a cancer antigen) from a living body, and introducing a gene encoding IL-7 and a gene encoding CCL19. In the present disclosure, the expression, "gene encoding XX", and the expression, "nucleic acid encoding XX", are used interchangeably. In this case, the nucleic acid may be single-stranded or double-stranded, and may be DNA or RNA. The nucleic acid is preferably double-stranded DNA.

In the case of gene introduction into an immunoresponsive cell collected from a living body, rejection can be minimized by collecting an immunoresponsive cell of a cancer-suffering person to be treated with the combination drug A according to the present disclosure, (i.e., an autologous immunoresponsive cell). However, use of an allogenic immunoresponsive cell is not excluded. In other words, the immunoresponsive cell A according to the present disclosure may or may not be an immunoresponsive cell derived from the subject itself.

The gene encoding a cell surface molecule that specifically recognizes a cancer antigen, the gene encoding IL-7, and the gene encoding CCL19 each may be present in the genome of the immunoresponsive cell A according to the present disclosure or may be retained on a vector that is present outside the genome. For example, each gene may be allowed to be present in the genome from the viewpoint of the stability of gene retaining. The gene encoding a cell surface molecule that specifically recognizes a cancer antigen, the gene encoding IL-7, and the gene encoding CCL19 may be present in a gathered arrangement in the genome, or may be present in an ungathered manner (separately from one another). In a case in which the cell surface molecule that specifically recognizes a cancer antigen is a heterodimer, such as a TCR formed of an $\alpha\beta$-dimer or $\gamma\delta$-dimer, or a heteromultimer, genes encoding the respective molecules composing the heterodimer or heteromultimer may be present in a gathered arrangement in the genome, or may be present in an ungathered manner (separately from one another).

In one embodiment, the gene encoding IL-7 and the gene encoding CCL19 are exogenous, and both of the genes are integrated into the genome of the immunoresponsive cell A according to the present disclosure, or the gene encoding IL-7 and the gene encoding CCL19 are coded together in one vector or separately coded in plural vectors present in the immunoresponsive cell A according to the present disclosure. Whether or not the respective genes are present in the cell can easily be confirmed using a known technique such as PCR. In the present disclosure, the term "exogenous" means that the gene or nucleic acid is not a gene or nucleic acid that has been inherently present in the cell, but is a gene or nucleic acid that has been introduced from the outside.

With respect to TCRs, TCRs such as MARTI-specific TCR (Cancer Res.54, 5265-5268 (1994)), MAGE-A3-specific TCR (Anticancer Res., 20, 1793-1799 (2000)), gp100-specific TCR (J. Immunol. 170, 2186-2194 (2003)), NY-ESO-1-specific TCR (J. Immunol., 174, 4415-4423 (2005)), WT1-specific TCR (Blood, 106, 470-476 (2005)), MAGE-A1-specific TCR (Int. Immunol., 8, 1463-1466 (1996)), P1A-specific TCR (Sarma, S., Y. Guo, Y. Guilloux, C. Lee, X.-F. Bai, Y. Liu. 1999. Cytotoxic T lymphocytes to an unmutated tumor antigen P1A: normal development but restrained effector function. J. Exp. Med. 189:811.), MAGE-A10-specific TCR, AFP-specific TCR, CT-83-specific TCR, KRAS (including a mutant, i.e., mKRAS)-specific TCR, MAGE-A4-specific TCR, Epha2-specific TCR, BCMA-specific TCR, 5T4-specific TCR, PRAME-specific TCR, and HA-1-specific TCR, have been reported, and nucleic acid sequences encoding these TCRs are also reported in the respective documents. When the cell surface molecule that specifically recognizes a cancer antigen is a TCR, the base sequence of the TCR-encoding gene may have a sequence identity of, for example, 80% or more, more specifically 85% or more, more specifically 90% or more, more specifically 95% or more, and more specifically 98% or more, with respect to a TCR-encoding base sequence described in the foregoing documents, as far as the base sequence is capable of recognition of the target antigen molecule and activation of T cells. In the present disclosure, the sequence identity of amino acid sequences and the sequence identity of base sequences can be evaluated, for example, using BLAST (registered trademark, National Library of Medicine) program with default parameters.

Alternatively, the base sequence of the TCR-encoding gene may be a base sequence that retains the CDR-encoding base sequences in a TCR-encoding base sequence described in the foregoing documents, and of which the base sequence in regions other than the CDR-encoding base sequences has a sequence identity 60% or more, more specifically 70% or more, more specifically 80% or more, more specifically 90% or more, and more specifically 95% or more, with respect to the base sequence of the corresponding region in the TCR-encoding base sequence described in the foregoing documents.

Of course, the base sequence of the TCR varies with the antigen specificity of the TCR. A T cell expressing a TCR that binds to an antigen of interest may be isolated, and the base sequence of the TCR may be analyzed. For example, the base sequence of a gene encoding a TCR that specifically recognizes a specific antigen can be obtained by analyzing the base sequences encoding the alpha chain and the beta chain as TCR subunits of a cytotoxic T cell (CTL) induced with the specific antigen, using methods known in the art (International Publication (WO) No., 2007/032255 and Morgan et al., J. Immunol, 171, 3288 (2003). For analysis of the base sequence of the TCR, the analysis may be performed after base sequences encoding the respective chains are amplified using a PCR method. A PCR primer may be, for example, 5'R-primer as a 5'-side primer (5'-gtctaccaggcattcgcttcat-3': SEQ ID NO: 1), and 3-TRa-C primer (5'-tcagctggaccacagccgcagcgt-3': SEQ ID NO: 2) as a 3'-side primer that is specific to the TCR α-chain C region, 3-TRb-C1 primer (5'-tcagaaatcctttctcttgac-3': SEQ ID NO: 3) specific to TCR β-chain C1 region, or 3-TRbeta-C2 primer (5'-ctagcctctggaatcctttctctt-3': SEQ ID NO: 4) specific to TCR β-chain C2 region. However, the PCR primer is not limited thereto. An antigen-specific TCR can bind, with a high binding force, to a target cell presenting the antigen (for example, a peptide). By suitably selecting the kind of immunoresponsive cell, efficient killing of a target cell presenting an antigen peptide can be mediated.

The CAR-encoding base sequence is not particularly limited as long as the base sequence encodes a polypeptide configuring the CAR. The CAR-encoding base sequence includes base sequences encoding polypeptides of a single chain antibody that recognizes a cell surface antigen of a cancer cell, a cell membrane penetrating region, and a signaling region that induces T-cell activation.

The information of base sequences encoding polypeptides of a single chain antibody against a cell surface antigen of a cancer cell, a cell membrane penetrating region, and an immunoresponsive cell activating signaling region in the CAR can be obtained, as desired, by searching known publications or database such as NCBI (http://www.ncbi.nlm.nih.gov/guide/).

For example, information of base sequences encoding polypeptides of cell membrane penetrating regions of CD28, 4-1BB, and CD3ζ in the immunoresponsive cell activating signaling region can be obtained, as desired, by searching database such as NCBI. The base sequence registered with Genbank number NM_006139.2 (update date: May 10, 2014) can be exemplified for human CD28, the base sequence registered with Genbank number NM_001561.5 (update date: March 16, 2014) can be exemplified for human 4-1BB, and the base sequence registered with Genbank number NM_000734.3 (update date: August 12, 2014) can be exemplified for human CD3ζ.

The information of a base sequence encoding a polypeptide of a cell membrane penetrating region of human CD8 can be obtained, as desired, by searching database such as NCBI, examples of which include a base sequence registered with Genbank Number NM_001768.6 (update date: May 10, 2014).

Further, the information of a base sequence encoding a polypeptide of a single chain antibody can also be obtained based on an amino acid sequence that is obtained by producing a monoclonal antibody that recognizes the target cell surface antigen, and determining the amino acid sequence of the monoclonal antibody using a known method such as the Edman method. Examples of the method used for producing the monoclonal antibody include a production method using a hybridoma, a production method including transforming a host with an expression vector that contains an antibody gene, using a genetic engineering technique, and a production method including immunizing a transgenic animal with a desired antigen.

The information of a base sequence encoding IL-7 and a base sequence encoding CCL19 can be obtained, as desired, by searching known publications or database such as NCBI (http://www.ncbi.nlm.nih.gov/guide/).

The base sequence encoding IL-7 can be selected, as appropriate, in accordance with the type of the immunoresponsive cell A according to the present disclosure. The base sequence encoding IL-7 may be a base sequence encoding the amino acid sequence of human IL-7 (SEQ ID NO: 5), or a base sequence having a sequence identity of, for example, 80% or more, more specifically 85% or more, more specifically 90% or more, more specifically 95% or more, and more specifically 98% or more, with respect to the base sequence of SEQ ID NO: 5, as long as the effect of IL-7 in terms of promoting the cell proliferation rate is exerted.

The base sequence encoding CCL19 can be selected, as appropriate, in accordance with the type of the immunoresponsive cell A according to the present disclosure. The base sequence encoding CCL19 may be a base sequence encoding the amino acid sequence of human CCL19 (SEQ ID NO: 6), or a base sequence having a sequence identity of, for example, 80% or more, more specifically 85% or more, more specifically 90% or more, more specifically 95% or more, and more specifically 98% or more, with respect to the base sequence of SEQ ID NO: 6, as long as the activity of CCL19 on T-cell migration is exerted.

In the immunoresponsive cell A according to the present disclosure, the gene encoding a cell surface molecule that specifically recognizes a cancer antigen, the gene encoding IL-7, and the gene encoding CCL19 are arranged to be subject to control by an appropriate promoter.

The immunoresponsive cell A according to the present disclosure may further express one or more other immune function regulating factors such as IL-1, IL-2, IL-3, IL-4, IL-5, IL-6, IL-8, IL-9, IL-10, IL-11, IL-12, IL-13, IL-14, IL-15, IL-16, IL-17, IL-18, IL-23, IL-27, IP-10, CCL1, CCL2, CCL3, CCL4, CCL5, CCL7, CCL8, CCL11, CCL13, CCL14, CCL15, CCL16, CCL17, CCL18, CCL20, CCL21, CCL22, CCL23, CCL24, CCL25, CCL26, CCL27, CCL28, CXCL1, CXCL2, CXCL3, CXCL4, CXCL4L1, CXCL5, CXCL6, CXCL7, CXCL8, CXCL9, CXCL10, CXCL11, CXCL12, CXCL13, CXCL14, CXCL16, CXCL17, CX3CL1, XCL1, XCL2, CCL3L1, CCL3L3, CCL4L1, CCL4L2, Flt3L, Interferon-gamma, MIP-1 alpha, GM-CSF, M-CSF, TGF-beta, or TNF-alpha, in addition to IL-7, CCL19, and a cell surface molecule that specifically recognizes a cancer antigen.

Examples of methods for the separation of the immunoresponsive cell A according to the present disclosure include, but are not limited to: density gradient centrifugation; resetting; coupling to a particle for changing the cell density; magnetic separation using antibody-coated magnetic beads; affinity chromatography (for example, affinity chromatography using negative selection); a cytotoxic agent (examples of which include, but are not limited to, complements and cytotoxins) linked to a monoclonal antibody or used in combination with a monoclonal antibody; panning using an antibody bound to a solid matrix such as a plate or a tip; elutriation; selective proliferation due to antigen stimuli; and separation utilizing a complex of MHC and an antigen.

When the immunoresponsive cell inherently expresses a cell surface molecule that specifically recognizes a cancer antigen, for example, when a T cell expressing a TCR that specifically recognizes a given cancer antigen is to be isolated, it is not necessary to introduce a gene encoding a cell surface molecule that specifically recognizes a cancer antigen from the outside. However, in general, one or more of the gene encoding a cell surface molecule that specifically recognizes a cancer antigen, the gene encoding IL-7, or the gene encoding CCL19 are introduced from the outside.

A nucleic acid including a gene encoding a cell surface molecule that specifically recognizes a cancer antigen, a nucleic acid including a gene encoding IL-7, and a nucleic acid including a gene encoding CCL19, which are to be introduced into an immunoresponsive cell, can be prepared based on the information of base sequences encoding the respective molecules, using known techniques such as a chemical synthesis method or an amplification method using PCR. Codons in the coding region may be modified so as to optimize the expression of the gene in the immunoresponsive cell to which a nucleic acid including the gene is to be introduced.

The genes to be introduced may be introduced in a state in which the genes are retained on respectively different vectors, or in a state in which two or more genes are retained on the same vector. For example, in the case of introducing a gene encoding IL-7 and a gene encoding CCL19 into an immunoresponsive cell, the gene encoding IL-7 and the gene encoding CCL19 may be introduced using separate vectors, or both genes may be introduced while being retained on the same vector.

When a gene encoding a cell surface molecule that specifically recognizes a cancer antigen, a gene encoding IL-7, and a gene encoding CCL19 are to be introduced into an immunoresponsive cell,

(i) the introduction may be performed in a state in which the gene encoding a cell surface molecule that specifically recognizes a cancer antigen, the gene encoding IL-7, and the gene encoding CCL19 are retained on respectively separate vectors; or
(ii) the introduction may be performed in a state in which the gene encoding a cell surface molecule that specifically recognizes a cancer antigen and the gene encoding IL-7 are retained on the same vector, and in which the gene encoding CCL19 is retained on a separate vector; or
(iii) the introduction may be performed in a state in which the gene encoding a cell surface molecule that specifically recognizes a cancer antigen and the gene encoding CCL19 are retained on the same vector, and in which the gene encoding IL-7 is retained on a separate vector; or
(iv) the introduction may be performed in a state in which the gene encoding IL-7 and the gene encoding CCL19 are retained on the same vector, and in which the gene encoding a cell surface molecule that specifically recognizes a cancer antigen is retained on a separate vector; or
(v) the introduction may be performed in a state in which the gene encoding a cell surface molecule that specifically recognizes a cancer antigen, the gene encoding IL-7, and the gene encoding CCL19 are retained on the same vector.

The introduction may be performed in a state in which two or more genes are retained on the same vector, in consideration of introduction efficiency. In this case, the two or more genes will be present in a gathered arrangement in the immunoresponsive cell.

For example, the following vector or a group of vectors may be used.

(a) a vector containing a gene encoding a cell surface molecule that specifically recognizes a cancer antigen, a gene encoding IL-7, and a gene encoding CCL19;
(b) a group of vectors consisting of the following vector (b-1) and vector (b-2)

  (b-1) a vector containing a gene encoding a cell surface molecule that specifically recognizes a cancer antigen,
  (b-2) a vector containing a gene encoding IL-7 and a gene encoding CCL19;

(c) a group of vectors consisting of the following vector (c-1) and vector (c-2)

(c-1) a vector containing a gene encoding a cell surface molecule that specifically recognizes a cancer antigen and a gene encoding IL-7,
(c-2) a vector containing a gene encoding CCL19;

(d) a group of vectors consisting of the following vector (d-1) and vector (d-2)

(d-1) a vector containing a gene encoding IL-7;
(d-2) a vector containing a gene encoding a cell surface molecule that specifically recognizes a cancer antigen and a gene encoding CCL19,

(e) a group of vectors consisting of the following vector (e-1), vector (e-2), and vector (d-3)

(e-1) a vector containing a gene encoding a cell surface molecule that specifically recognizes a cancer antigen,
(e-2) a vector containing a gene encoding IL-7,
(e-3) a vector containing a gene encoding CCL19.

Such a group of vectors may be designed such that the genes are contained in a redundant manner. For example, a group of vectors consisting of the foregoing (c-1) and (d-2) may be designed. In this case, both of the vectors include a gene encoding a cell surface molecule that specifically recognizes a cancer antigen; the genes encoding a cell surface molecule that specifically recognizes a cancer antigen contained in both vectors may be either the same as each other or different from each other.

A gene or genes encoding one or more other immune function regulating factors such as IL-1, IL-2, IL-3, IL-4, IL-5, IL-6, IL-8, IL-9, IL-10, IL-11, IL-12, IL-13, IL-14, IL-15, IL-16, IL-17, IL-18, IL-23, IL-27, IP-10, CCL1, CCL2, CCL3, CCL4, CCL5, CCL7, CCL8, CCL11, CCL13, CCL14, CCL15, CCL16, CCL17, CCL18, CCL20, CCL21, CCL22, CCL23, CCL24, CCL25, CCL26, CCL27, CCL28, CXCL1, CXCL2, CXCL3, CXCL4, CXCL4L1, CXCL5, CXCL6, CXCL7, CXCL8, CXCL9, CXCL10, CXCL11, CXCL12, CXCL13, CXCL14, CXCL16, CXCL17, CX3CL1, XCL1, XCL2, CCL3L1, CCL3L3, CCL4L1, CCL4L2, Flt3L, Interferon-gamma, MIP-1 alpha, GM-CSF, M-CSF, TGF-beta, or TNF-alpha may be further incorporated into any of the foregoing vectors, or into a vector other than the foregoing vectors, and may be introduced into the immunoresponsive cell.

The method used for introducing a gene-retaining vector into an immunoresponsive cell is not particularly limited, and examples thereof include known methods such as a virus infection method, a transposon method, a calcium phosphate method, a lipofection method, a microinjection method, and an electroporation method. A method involving introduction using a virus infection method, which is capable of introducing a foreign gene into the genome, may provide stability of gene retention.

An example of the virus infection method is a method including transfecting a packaging cell, such as a GP2-293 cell (manufactured by Takara Bio Inc.), a Plat-GP cell (manufactured by Cosmo Bio Co., Ltd.), a PG13 cell (ATCC CRL-10686), or PA317 cell (ATCC CRL-9078) with a vector and a packaging plasmid to generate a recombinant virus, and infecting an immunoresponsive cell with the recombinant virus. This may be carried out using a commercial kit such as Retrovirus packaging kit Eco (manufactured by Takara Bio Inc.). Use of a MSCV retrovirus expression system or the like enables introduction of a foreign gene into the genome.

The integration of the gene encoding IL-7, the gene encoding CCL19, and, if necessary, the gene encoding a cell surface molecule that specifically recognizes a cancer antigen, into the genome can also be performed using a known gene editing technique. Examples of known gene editing techniques include a technique using an endonuclease such as zinc finger nuclease, TALEN (transcription activator-like effector nuclease), or a CRISPR (clustered regulatory interspaced short palindromic repeat)-Cas system. Integration of a gene encoding another foreign protein, which is optionally introduced, into the genome can be performed using such methods.

In the case of integrating any of these genes into the genome of an immunoresponsive cell, the gene may be integrated, together with an upstream promotor for regulating the gene, into a non-coding region or the like of the genome in an operable manner (i.e., to be capable of expression under control of the promotor), or may be integrated, without a promoter, into the downstream of a promoter that is already present in the genome in an operable manner. Examples of the promoter that is already present in the genome include a promoter of TCRa or TCR$\beta$.

When two or more of a gene encoding a cell surface molecule that specifically recognizes a cancer antigen, a gene encoding IL-7, a gene encoding CCL19, or an optionally introduced gene encoding an additional foreign protein are present adjacent to each other, those two or more genes may be expressed under a control exerted by a common promoter. In a case in which the genes are expressed under a control exerted by a common promoter, transcription and/or translation may be split by using a 2A peptide, an IRES peptide, or the like, to enable expression of individual polypeptides.

In a case in which a vector carrying two or more of a gene encoding a cell surface molecule that specifically recognizes a cancer antigen, a gene encoding IL-7, or a gene encoding CCL19 is introduced into an immunoresponsive cell, the alignment order of the two or more genes in the vector is not particularly limited. For example, in the vector (a) containing a gene encoding a cell surface molecule that specifically recognizes a cancer antigen, a gene encoding IL-7, and a gene encoding CCL19, the alignment order of these three genes is not limited. Specifically, with respect to the order from the upstream (5'-terminal side) to the downstream (3'-terminal side), the order may be any of the following:

an order in which a gene encoding a cell surface molecule that specifically recognizes a cancer antigen, a gene encoding IL-7, and a gene encoding CCL19 are arranged in this order;
an order in which a gene encoding a cell surface molecule that specifically recognizes a cancer antigen, a gene encoding CCL19, and a gene encoding IL-7 are arranged in this order;
an order in which a gene encoding IL-7, a gene encoding CCL19, and a gene encoding a cell surface molecule that specifically recognizes a cancer antigen are arranged in this order;
an order in which a gene encoding IL-7, a gene encoding a cell surface molecule that specifically recognizes a cancer antigen, and a gene encoding CCL19 are arranged in this order;
an order in which a gene encoding CCL19, a gene encoding a cell surface molecule that specifically recognizes a cancer antigen, and a gene encoding IL-7 are arranged in this order; or
an order in which a gene encoding CCL19, a gene encoding IL-7, and a gene encoding a cell surface molecule that specifically recognizes a cancer antigen are arranged in this order.

In the vector (b-2) containing a gene encoding IL-7 and a gene encoding CCL19, the alignment order of a gene encoding IL-7 and a gene encoding CCL19 is not particularly limited, and the gene encoding CCL19 may be located either at upstream or downstream of the gene encoding IL-7.

In the vector (c-1) containing a gene encoding a cell surface molecule that specifically recognizes a cancer antigen and a gene encoding IL-7, the alignment order of the gene encoding a cell surface molecule that specifically recognizes a cancer antigen and the gene encoding IL-7 is not particularly limited, and the gene encoding IL-7 may be located either at upstream or downstream of the gene encoding a cell surface molecule that specifically recognizes a cancer antigen.

In the vector (d-2) containing a gene encoding a cell surface molecule that specifically recognizes a cancer antigen and a gene encoding CCL19, the alignment order of the gene encoding a cell surface molecule that specifically recognizes a cancer antigen and the gene encoding CCL19 is not particularly limited, and the gene encoding CCL19 may be located either at upstream or downstream of the gene encoding a cell surface molecule that specifically recognizes a cancer antigen.

The gene encoding a cell surface molecule that specifically recognizes a cancer antigen, the gene encoding IL-7, and the gene encoding CCL19 may be transcribed due to the action of respectively different promoters, or may be transcribed due to the action of one promoter, using internal ribozyme entry site (IRES) or a self-cleaving 2A peptide.

In a case in which plural genes are transcribed due to the action of one promoter, a base sequence stretching between the respective genes may include a freely-selected base sequence as long as expression of individual genes is possible. The base sequence stretching between the respective genes may include a base sequence encoding a self-cleaving peptide (2A peptide) or an IRES-encoding base sequence, or may include a base sequence encoding a 2A peptide. Efficient expression of respective genes is enabled by linking plural genes with such a base sequence. The intergenic base sequence, which may include a base sequence encoding a self-cleaving peptide (2A peptide) or IRES, may be a base sequence stretching between a gene encoding IL-7 and a gene encoding CCL19, or a base sequence stretching between a gene encoding a cell surface molecule that specifically recognizes a cancer antigen and a gene encoding IL-7, or a base sequence stretching between a gene encoding a cell surface molecule that specifically recognizes a cancer antigen and a gene encoding CCL19, or a base sequence stretching between a gene encoding an alpha chain and a gene encoding a beta chain in an alpha-beta TCR, or a base sequence stretching between a gene encoding a gamma chain and a gene encoding a delta chain in a gamma-delta TCR. That is, these intergenic regions each may include a base sequence encoding a self-cleaving peptide (2A peptide) or IRES, if desired.

A 2A peptide is a self-cleaving peptide from a virus. In the case of an amino acid sequence represented by SEQ ID NO: 7, the peptide has a feature such that the bond between G and P (at a position that is one base distant from the C terminal) in the amino acid sequence is cleaved in the endoplasmic reticulum (Szymczak et al., Expert Opin. Biol. Ther. 5(5):627-638 (2005)). Thus, the nucleic acids located before and after the 2A peptide will be independently expressed in the cell.

The 2A peptide may be a 2A peptide from picornavirus, rotavirus, an insect virus, aphthovirus, or Trypanosoma virus, or a 2A peptide (F2A) from picornavirus indicated in SEQ ID NO: 8.

The vector used for introducing a gene into an immunoresponsive cell may be linear or circular, and may be a non-virus vector such as a plasmid, a virus vector, or a transposon vector. The vector used for introducing a gene into the immunoresponsive cell may include one or more of a regulatory sequence such as a promoter or a terminator, or a selection marker sequence such as a drug-resistant gene or a reporter gene. Expression of the gene may utilize a promoter contained in the vector even after the gene has been introduced into the immunoresponsive cell. For example, by disposing one or more of the gene encoding a cell surface molecule that specifically recognizes a cancer antigen, the gene encoding IL-7, or the gene encoding CCL19 at the downstream of a promoter sequence in the vector in an operable manner, the genes can efficiently be transcribed.

Examples of the promoter include promoters from viruses such as a LTR promoter of a retrovirus, the SV40 early promoter, the cytomegalovirus promoter, and the thymidine kinase promoter of the herpes simplex virus, and promoters from mammals such as the phosphoglycerate kinase (PGK) promoter, the Xist promoter, the β-actin promoter, the RNA polymerase II promoter, and the polypeptide chain elongation factor gene promoter. It is also possible to use a tetracycline-responsive promoter induced by tetracycline, a M×1 promoter induced by interferon, or the like. By using a promoter that is induced by a specific substance, the expression of a gene subject to transcription regulation by the promoter (for example, one or more of a gene encoding a cell surface molecule that specifically recognizes a cancer antigen, a gene encoding IL-7, or a gene encoding CCL19) can be regulated in accordance with the course of treatment of the cancer.

Examples of the virus vector include a retrovirus vector, a lentivirus vector, an adenovirus vector, and an adeno-associated virus vector. Examples of the retrovirus vector include a pMSGV vector (Tamada K. et al., Clin. Cancer Res. 18:6436-6445 (2002)) and pMSCV vector (manufactured by Takara Bio Inc.). Use of a retrovirus vector enables the introduced gene to be stably expressed for a long time since the introduced gene is integrated into the genome of a host cell.

The expression of the cell surface molecule that specifically recognizes a cancer antigen IL-7, and CCL19 in the immunoresponsive cell can be determined, for example, by flow cytometry, ELISA, or Western Blotting. Introduction of genes encoding these molecules can be confirmed by checking for the expression products as described above, or by using, for example, Northern Blotting, Southern Blotting, or PCR such as RT-PCR. When the vector used for gene introduction includes a marker gene, the introduction of the gene can be confirmed by checking for the expression of the marker gene inserted in the expression vector.

The immunoresponsive cell A according to the present disclosure may further express a suicide gene, such as herpes simplex virus thymidine kinase (HSV-TK) or inducible caspace 9, so as to enable apoptosis induction. The genes of these enzymes can be introduced into the immunoresponsive cell (for example, into the genome of the immunoresponsive cell) according to a method such as those described above. For example, a nucleic acid encoding a suicide gene may be incorporated into a vector retaining at least one of a gene encoding a cell surface molecule that specifically recognizes a cancer antigen, a gene encoding IL-7, or a gene encoding CCL19, or into a separate vector from the foregoing vector, and introduced into the immunoresponsive cell.

A suicide gene means a gene having a function such that, when the gene is expressed, the gene directly or secondarily induces a substance having a cytotoxic activity, and causes the cell that has expressed the suicide gene to die. When a nucleic acid encoding a suicide gene is allowed to be included in the immunoresponsive cell, an agent that activates the function of the suicide gene can be administered in accordance with the course of treatment of the cancer (for example, administered when the tumor has disappeared), and immunoresponsive cells A according to the present disclosure that are present in the living body can be reduced or eliminated.

Examples of the suicide gene include a gene encoding herpes simplex virus thymidine kinase (HSV-TK) or inducible caspace 9 described in the following documents, and the agent that activates the function of the suicide gene is, for example, ganciclovir for HSV-TK, or AP1903, which is a dimerization-inducing compound (chemical induction of dimerization (CID)), for inducible caspace 9 (see, Cooper L. J, et. al., Cytotherapy, 2006; 8(2): 105-17, Jensen M. C. et. al., Biol BloodMarrow Transplant, 2010 Sep; 16(9): 1245-56, Jones B. S. Front Pharmacol. 2014 Nov 27; 5: 254., Minagawa K., Pharmaceuticals (Basel) 2015 May 8; 8(2): 230-49, Bole-Richard E., Front Pharmacol. 2015 Aug 25; 6: 174).

<Immunosuppression Inhibitor>

An immunosuppression inhibitor refers to a substance that cancels or reduces suppression of activation of immunoresponsive cells. Suppression of activation of immunoresponsive cells occurs due to, for example: suppression of association of a cytotoxic T cell or helper T cell with a dendrite cell caused by binding of a regulatory T cell (Treg) to a dendrite cell,; suppression of activation of a cytotoxic T cell, a helper T cell, or the like caused by secretion of a suppressive cytokine such as TGF-β or IL-10 or a cytotoxic substance such as perforin or granzyme from a Treg; or suppression of activation of cytotokic T cells caused by an immune checkpoint performed by, for example, an interaction between PD-1 and PD-L1 or an interaction between CTLA-4 and CD80/CD86. The immunosuppression inhibitor is a substance that cancels the suppression of activation of immunoresponsive cells, such as those described above, and thereby enables activation of the immunoresponsive cell.

Examples of the immunosuppression inhibitor include an immune checkpoint inhibitor, a molecular target drug that inhibits infiltration, survival, or function of an immunosuppressive cell such as Treg or a myeloid-derived suppressor cell (MDSC), a CCR4 inhibitor, an indoleamine-2,3-dioxygenase (IDO) inhibitor, a prostaglandin E2 [PGE2] inhibitor, and a cytotoxic anticancer agent. The immunosuppression inhibitor may be an antibody as far as the antibody has the foregoing function, and may be, for example, an IgG monoclonal antibody or an antibody fragment.

The immune checkpoint inhibitor is typically a substance that cancels or relaxes an immunosuppressive mechanism that works via an immune checkpoint molecule that is expressed on a surface of a T cell. The immune checkpoint inhibitor can reduce a suppressive reaction on an immune response, for example, by binding to an immune checkpoint molecule (for example, PD-1, CTLA-4, BTLA, TIM-3, TIGIT, or LAG-3) or a ligand of an immune checkpoint molecule (for example, PD-L1, PD-L2, CD80/CD86, or Siglec-15), to inhibit initiation of signal transduction from the immune checkpoint molecule triggered by the ligand.

The molecular target drug that inhibits infiltration, survival, or function of an immunosuppressive cell such as Treg or a MDSC can relax immunosuppression in a cancer microenvironment, for example, by inhibiting a tyrosine kinase and thereby reducing Tregs infiltrating into the cancer tissue.

The CCR4 inhibitor can relax immunosuppression in a cancer microenvironment by inhibiting the function of chemokine receptor CCR4 in terms of bringing together Tregs.

The IDO inhibitor can reduce kynurenine-induced Treg activation by inhibiting the enzymatic activity of IDO, or inhibiting expression of IDO, and thereby reducing production of kynurenine.

The PGE2 inhibitor can relax immunosuppression in a cancer microenvironment by suppressing an increase in Treg immunosuppressive action caused by binding of PGE2 to a prostaglandin EP4 receptor present on the Treg surface.

The cytotoxic anticancer agent can reduce suppression of an immune response, by decreasing the number of immunosuppressive cells such as Tregs.

Examples of the immune checkpoint inhibitor include a PD-1 inhibitor, a PD-L1 inhibitor, a CTLA-4 inhibitor, a CD47 inhibitor, a SIRPα inhibitor, a BTLA inhibitor, a TIM-3 inhibitor, a TIGIT inhibitor, a LAG-3 inhibitor, a Siglec-15 inhibitor, and a galectin-9 inhibitor. Examples of the molecular target drug that inhibits infiltration, survival, or function of an immunosuppressive cell such as Treg or a MDSC include sorafenib and sunitinib. Examples of the CCR4 inhibitor include an anti-CCR4 antibody (for example, Mogamulizumab). Examples of the IDO inhibitor include epacadostat. Examples of the prostaglandin E2 [PGE2] inhibitor include aspirin. Examples of the cytotoxic anticancer agent include cyclophosphamide and gemcitabine.

The immunosuppression inhibitor may include at least one selected from the group consisting of a PD-1 inhibitor, a PD-L1 inhibitor, a PD-L2 inhibitor, a CTLA-4 inhibitor, a BTLA (B- and T-lymphocyte attenuator) inhibitor, a TIM-3 (T-cell immunoglobulin and mucin domain 3) inhibitor, a TIGIT (T-cell immunoreceptor with Ig and ITIM domains) inhibitor, a LAG-3 (Lymphocyte Activation Gene-3) inhibitor, and a Siglec-15 inhibitor.

The immunosuppression inhibitor in the combination drug A according to the present disclosure may be an immune checkpoint inhibitor, more specifically a PD-1 inhibitor or a PD-L1 inhibitor, and still more specifically an anti-PD-1 antibody or an anti-PD-Ll antibody. Examples of the anti-PD-1 antibody include Nivolumab, Pembrolizumab, Toripalimab, Cemiplimab-rwlc, and Sintilima. Examples of the anti-PD-Ll antibody include Atezolizumab, Durvalumab, and Avelumab. Examples of an anti-CTLA-4 antibody include Ipilimumab. Further examples include an antibody to CD47 and an antibody to SIRPα.

In the combination drug A according to the present disclosure, it is conceivable that expression of IL-7 and CCL19 in addition to a cell surface molecule that specifically recognizes a cancer antigen performed by the action of the immunoresponsive cell A according to the present disclosure, and further inclusion of an immunosuppression inhibitor not only exerts a cancer suppression effect due to the cell surface molecule that specifically recognizes a cancer antigen, but also produces an effect with respect to reduction of immunosuppression in a cancer microenvironment based on the combination of IL-7, CCL19, and the immunosuppression inhibitor, and, further, with respect to induction of endogenous cytotoxic T cells and the like to the vicinity of cancer cells and activation of the endogenous cytotoxic T cells and the like. For this reason, the combination drug A according to the present disclosure produces an improved therapeutic effect against cancer that cannot be obtained in the case of administration of the immunoresponsive cell A according to the present disclosure alone without administration of the immunosuppression inhibitor, or in the case of co-administration of the immunosuppression inhibitor and an immunoresponsive cell that expresses a cell surface molecule that specifically recognizes a cancer antigen but does not express IL-7 or CCL19.

The therapeutic effect against cancer can be evaluated, for example, by a decrease in the number of tumor cells, a decrease in tumor size, disappearance of tumor, or a decrease in tumor burden in a subject that is, for example, a mammalian animal such as a human.

Since the immunosuppression inhibitor activates the action of immunoresponsive cells in general in an immunosuppressive microenvironment around a cancer, the immunosuppression inhibitor improves cancer cell attacking activity of endogenous immunoresponsive cells as well as of the immunoresponsive cell A according to the present disclosure. Therefore, when the immunosuppression inhibitor is, for example, an immune checkpoint inhibitor targeted to a specific molecule, it is not necessary for the immunoresponsive cell A according to the present disclosure to express the target molecule on the cell surface thereof; the immunoresponsive cell A according to the present disclosure may or may not express the target molecule.

In the present disclosure, the term "antibody" refers not only to a complete antibody molecule, but may also refer to a fragment of an antibody molecule that retains the ability to bind to the antigen. Such an antibody fragment is also known in the art, and generally used *in vitro* as well as *in vivo*. Therefore, as used herein, the term "antibody" refers to a concept that encompasses not only a complete immunoglobulin molecule, but also $F(ab')_2$ and Fab, which are known functional fragments of antibodies. In the present disclosure, the scope of the antibody encompasses a complete natural antibody, a bispecific antibody, a chimeric antibody, Fab, Fab', a single chain antibody (scFv), a fused polypeptide, and a nonconventional antibody.

In the present disclosure, a single chain antibody (scFv) is a fusion protein of a heavy chain (VH) variable region and a light chain (VL) variable region of immunoglobulin that are covalently bonded to form a VH::VL heterodimer. The heavy chain (VH) and the light chain (VL) are directly bonded, or the N terminal of VH and the C terminal of VL are bonded via a peptide linker, or the C terminal of VH and the N terminal of VL are bonded via a peptide linker. The length of the peptide linker is, for example, 10 amino acids, 15 amino acids, 20 amino acids, or 25 amino acids. The peptide linker is usually rich in glycine, which contributes to flexibility, and serine or threonine, which contribute to solubility. Even though a constant region is removed and a peptide linker is incorporated, the scFv protein still has the antigen binding specificity that the original immunoglobulin had. As described in Huston et al., (Proc. Nat. Acad. Sci. USA, 85:5879-5883, 1988), a scFv can be expressed from a nucleic acid that includes a VH-encoding sequence and a VL-encoding sequence. With respect to scFv, U.S. Patent Nos. 5,091,513, 5,132,405, and 4,956,778, and U.S. Patent Application Publication Nos. 2005/0196754 and 2005/0196754, can also be referenced.

An antibody for use as an immune checkpoint inhibitor may be an IgG monoclonal antibody, a Fab fragment, a scFv, or another antibody or antibody fragment as long as the antibody or antibody fragment has the required antigen binding property. The scFv can be obtained, for example, by a method including preparing a mouse hybridoma clone and then converting the complete IgG (or IgM) to a scFv, a method including preparing an immunized phage display scFv, and then screening the library, using the antigen, or a method including directly obtaining a scFv by screening a ready-made scFv phage display library, using the antigen.

An antibody obtained from an animal (for example, a mouse) other than a human may raise an immune response upon administration to a human. In consideration of this, such an antibody may be used after being modified to a chimeric antibody prepared by replacing a gene of a non-variable part of the antibody by a human antibody gene by recombination, or to a humanized antibody prepared by replacing a part other than complementary determining regions (CDRs) by a human antibody gene by recombination. Further, a completely humanized antibody may be obtained using a phage display or a genetically modified mouse that produces a human antibody, rather than making a mouse or the like produce an antibody.

<Administration Composition>

In the combination drug A according to the present disclosure, an administration composition that includes the immunoresponsive cell A according to the present disclosure (hereinafter also referred to as a "first administration composition") may further include a pharmaceutically acceptable additive, and examples of the additive include physiological saline, buffered saline, cell culture medium, dextrose, water for injection, glycerol, ethanol, and combinations thereof, a stabilizer, a solubilizer, a surfactant, a buffering agent, a preservative, an isotonization agent, a filler, and a lubricant.

In the combination drug A according to the present disclosure, an administration composition that includes the immunosuppression inhibitor (hereinafter also referred to as a "second administration composition") may further include a pharmaceutically acceptable additive, and examples of the additive include physiological saline, buffered saline, cell culture medium, dextrose, water for injection, glycerol, ethanol, and combinations thereof, a stabilizer, a solubilizer, a surfactant, a buffering agent, a preservative, an isotonization agent, a filler, and a lubricant.

The second administration composition may be the same composition as the first administration composition, in which case one administration composition includes both of the immunoresponsive cell A according to the present disclosure and the immunosuppression inhibitor.

When the second administration composition is a composition that is separate from the first administration composition, the first administration composition and the second administration composition may be administered together, or may be administered at different times (different points in time), as described above. In other words, the immunoresponsive cell A according to the present disclosure and the immunosuppression inhibitor may be administered together, or may be separately administered at different times.

The amount of the immunoresponsive cells A according to the present disclosure contained in the first administration composition may be adjusted, as appropriate, in accordance with, for example, the type, position, or severity of the cancer, or the age, body weight, or condition of the subject to be treated. The amount of the immunoresponsive cells A according to the present disclosure contained in the first administration composition is, for example, from $1\times10^4$ to $1\times10^{11}$ cells, more specifically from $1\times10^5$ to $1\times10^{10}$ cells, and still more specifically from $1\times10^6$ to $1\times10^9$ cells, per one administration. Further the amount of the immunoresponsive cells A according to the present disclosure may be small, such as less than $1\times10^6$ cells, for example, from $1\times10^5$ to $5\times10^5$ cells, and more specifically from $1.5\times10^5$ to $4\times10^5$ cells, per one administration.

As described above, a cancer that is difficult to treat in the case of using the immunoresponsive cells A according to the present disclosure alone, or in the case of using the immunosuppression inhibitor alone, may be treated using the combination drug A according to the present disclosure. Therefore, the amount of the immunoresponsive cells A according to the present disclosure contained in the first administration composition may be so small an amount at which solo use of the same amount (same cell number) of immunoresponsive cells A according to the present disclosure would not exert an anticancer effect. The lower limit of the amount of the immunoresponsive cells A is not particularly limited as long as the amount of the immunoresponsive cells A is capable of exerting an anticancer effect based on a synergistic effect with the immunosuppression inhibitor.

The first administration composition may be administered at a frequency of four times daily, three times daily, twice daily, once daily, once per every other days, once in every third days, once in every fourth day, once in every fifth day, once in every sixth day, once weekly, once in every eighth day, once in every ninth day, once in every tenth day, twice weekly, once monthly, or twice monthly. Further, the number of administrations may be from, for example, 1 to 10 in total, specifically, 1, 2, 3, 4, 5, 6, 7, 8, 9, or 10 in total. Administration for more than 10 times is also permissible.

The amount of the immunosuppression inhibitor contained in the second administration composition may be adjusted, as appropriate, in accordance with, for example, the type, position, or severity of the cancer, or the age, body weight, or condition of the subject to be treated. The dose of the immunosuppression inhibitor is, for example, from 0.1 to 500mg/kg, more specifically from 0.5 to 250mg/kg, and more specifically from 1 to 100mg/kg, per one administration.

The second administration composition may be administered at a frequency of four times daily, three times daily, twice daily, once daily, once per every other days, once in every third days, once in every fourth day, once in every fifth day, once in every sixth day, once weekly, once in every eighth day, once in every ninth day, once in every tenth day, twice weekly, once monthly, or twice monthly. Further, the number of administrations may be, for example, from 1 to 30 in total, specifically, 1, 2, 3, 4, 5, 6, 7, 8, 9, 10, or 11 to 30 in total. Administration for more than 30 times is also permissible.

The relationship between the timing of administration of the first administration composition and the timing of administration of the second administration composition is not particularly limited. Administration of the first administration composition may be started first, or administration of the second administration composition may be started first, or administration of the first administration composition and administration of the second administration composition may be started at the same time. There is not particular limitation on the relationship between the numbers or frequency of administrations of the respective administration compositions.

The first administration composition and the second administration composition may be the same composition, in which case a pharmaceutical composition that includes both of the immunoresponsive cell A according to the present disclosure and the immunosuppression inhibitor (a compound drug) is provided. When the first administration composition and the second administration composition in the combination drug A according to the present disclosure are different compositions, the first administration composition and the second administration composition may be administered at the same time, or may be administered at different times (i.e., with an interval). From the viewpoint of effectively obtaining the synergistic effect of the immunoresponsive cell A according to the present disclosure and the immunosuppression inhibitor, the interval between the timing of administration of the first administration composition and the timing of administration of the second administration composition (the interval between the timing of administration of one of the administration compositions and the timing of administration of the other administration composition which timing is temporally closest to the foregoing timing) may be 3 months or less, 2 months or less, 1 month or less, or 2 weeks or less. The interval may be 1 week or less, or 3 days or less. Since the immunoresponsive cell A according to the present disclosure is maintained for a long time in a living body, as demonstrated in the later-described Examples, the interval needs not be overly short.

In the combination drug A according to the present disclosure, the immunoresponsive cell A according to the present disclosure and the immunosuppression inhibitor may be administered at independent points in time (for example, at different times), as described above. Further, in the present disclosure, the meaning of the term "co-administration" and the term "combined use" is intended to encompass a case in which plural agents are included in the same composition and administered, a case in which plural agents are included in separate compositions but administered at the same time, and a case in which plural agents are included in separate compositions and administered at different times.

As described above, the combination drug A according to the present disclosure exerts a surprisingly improved therapeutic effect against cancer, due to a synergistic effect exerted by the combination of factors of secreted IL-7 and CCL19, the immunoresponsive cell expressing a cell surface molecule that specifically recognizes a cancer antigen, and the immunosuppression inhibitor.

The first administration composition can be administered to a subject in need of treatment of a cancer, using a method known to those skilled in the art, and may be administered, for example, by oral administration, topical infusion or injection (including catheter infusion), systemic infusion or injection, intravenous infusion or injection, or parenteral administration (for example, transdermal administration or transmucosal administration, more specifically, nasal, ophthalmic, sublingual, by a suppository, by a patch, or the like). The first administration composition may be formulated into a form (solution, suspension liquid, or emulsion liquid) capable of infusion or injection of a unit dose, from the viewpoint of handleability. More specific examples of administration methods include intravenous injection, intratumor injection, intradermal injection, subcutaneous injection, intramuscular injection, intraperitoneal injection, intra-arterial injection, intramedullary injection, intracardiac injection, intra-articular injection, intrasynovial injection, intracranial injection, intrathecal injection, and subarchnoidal injection (injection to cerebrospinal fluid).

The second administration composition may be administered to a subject in need of treatment of a cancer, using a method known to those skilled in the art, and may be administered, for example, by topical infusion or injection (including catheter infusion), systemic infusion or injection, intravenous infusion or injection, or parenteral administration (for example, transdermal administration or transmucosal administration, more specifically, nasal, ophthalmic, sublingual, by a suppository, by a patch, or the like). The second administration composition may be formulated into a form (solution, suspension liquid, or emulsion liquid) capable of infusion or injection of a unit dose, from the viewpoint of handleability. More specific examples of administration methods include intravenous injection, intratumor injection, intradermal injection, subcutaneous injection, intramuscular injection, intraperitoneal injection, intra-arterial injection, intramedullary injection, intracardiac injection, intra-articular injection, intrasynovial injection, intracranial injection, intrathecal injection, and subarchnoidal injection (injection to cerebrospinal fluid).

After an immunoresponsive cell or precursor cell thereof as a starting point is obtained from a patient, who is the subject of treatment, and requisite genes for converting the cell into the immunoresponsive cell A according to the present disclosure are introduced thereto, the immunoresponsive cell A according to the present disclosure may be administered to the same patient (autologous administration) or to a different patient (allogenic administration). Alternatively, the immunoresponsive cell or precursor cell thereof as a starting point may be prepared from a pluripotent stem cell such as an iPS cell or an ES cell, or from somatic stem cell such as a hematopoietic stem cell.

The first administration composition and the second administration composition may be administered in the form of a sterile liquid preparation, which may be buffered to a prescribed pH, such as an isotonic aqueous solution, a suspension liquid, an emulsion liquid, a dispersion liquid, or a viscous composition. The liquid preparation may be a liquid preparation for injection. The liquid preparation may be in the form of a viscous composition having a viscosity within an appropriate viscosity range so as to elongate the duration of contact with a specified tissue. The liquid preparation may include a solvent or a dispersion medium selected from, for example, water, physiological saline, phosphate-buffered saline, a polyol (for example, glycerol, propylene glycol, or liquid polyethylene glycol), or a combination thereof.

The liquid preparation may be prepared by adding the immunoresponsive cell A according to the present disclosure and/or the immunosuppression inhibitor to an appropriate amount of an appropriate solvent, together with various amounts of other components. The liquid preparation may include a suitable carrier, diluent, or excipient. The liquid preparation may be lyophilized. The liquid preparation may further include various auxiliary agents, depending on the desired administration route, and examples of the auxiliary agents include moisturizing agents, dispersants or emulsifiers (for example, methyl cellulose), pH buffering agents, gelling agents or viscosity enhancing agents, preservatives, flavoring agents, and coloring agents. In regard to components that may be included in the liquid preparation, "REMINGTON'S PHARMACEUTICAL SCIENCE", 17th edition (1985) may be referenced.

The liquid preparation may further include various additives that enhance the stability and sterility of the liquid preparation, examples of which include antimicrobial preservatives, antioxidants, chelating agents, and buffering agents. Various antibacterial agents and antifungal agents, such as parabens, chlorobutanol, phenol, and sorbic acid, may be used in order to prevent actions of microorganisms. Vehicles, diluents, and additives for use in the liquid preparation should have compatibility with the immunoresponsive cell A according to the present disclosure and/or immunosuppression inhibitor contained in the liquid preparation.

The liquid preparation may be isotonic with blood. The isotonicity can be achieved by allowing the liquid preparation to include sodium chloride or another pharmaceutically acceptable osmolarity regulating substance (for example, dextrose, boric acid, sodium tartrate, propylene glycol, or another inorganic or organic solute).

The combination drug A according to the present disclosure may further include another anticancer agent, in addition to the immunoresponsive cell A according to the present disclosure and the immunosuppression inhibitor. Examples of the other anticancer agent include alkylating agents such as bendamustin, ifosfamide, and dacarbazine, antimetabolites such as pentostatin, fludarabin, cladribin, methotrexate, 5-fluorouracil, 6-mercaptopurine, and enocitabine, molecular target drugs such as rituximab, cetuximab, and trastuzumab, kinase inhibitors such as imatinib, gefitinib, erlotinib, afatinib, dasatinib, sunitinib, and trametinib, proteasome inhibitors such as bortezomib, calcineurin inhibitors such as cyclosporine and tacrolimus, anticancer antibiotics such as idarubicin, doxorubicin, and mitomycin C, plant alkaloids such as irinotecan and etoposide, platinum-containing drugs such as cisplatin, oxaliplatin, and carboplatin, hormone therapy agents such as tamoxifen and bicalutamide, and immune control agents such as interferon. The other anticancer agent may include at least one of an alkylating agent or an antimetabolite.

<Treatment of Cancer Using Combination Drug A according to the Present Disclosure>

In the case of using the combination drug A according to the present disclosure in treatment of a cancer, the subject to be treated may be, for example, any mammalian animal. The subject to be treated is, for example, a primate animal, and can more specifically be a human. The subject to be treated may alternatively be a pet animal or a farm animal, examples of which include a dog, a cat, a pig, cattle, a horse, a sheep, and a goat.

The cancer to be treated may be either a solid cancer or a blood cancer, and examples thereof include: cancers such as adenocarcinoma, squamous cell carcinoma, adenosquamous carcinoma, undifferentiated carcinoma, large cell carcinoma, small cell carcinoma, skin cancer, breast cancer, prostate cancer, bladder cancer, vaginal cancer, cervical cancer, uterine cancer, liver cancer, renal cancer, pancreatic cancer, spleen cancer, lung cancer, tracheal cancer, bronchial carcinoma, colon cancer, small intestine cancer, gastric cancer, esophageal cancer, gallbladder cancer, testicular cancer, and ovarian cancer; cancers of a bone tissue, a cartilage tissue, an adipose tissue, a muscle tissue, a vascular tissue, and a hematopoietic tissue; sarcomas such as chondrosarcoma, Ewing's sarcoma, malignant vascular endothelial sarcoma, malignant schwannoma, osteosarcoma, and soft tissue sarcoma; blastomas such as hepatoblastoma, medulloblastoma, nephroblastoma, neuroblastoma, pancreatoblastoma, pleuropulmonary blastoma, and retinoblastoma; germ cell tumor; lymphoma; and leukemia.

Since the combination drug A according to the present disclosure is capable of reducing immunosuppression in a cancer microenvironment, the cancer to be treated is not limited to a hemocyte cancer, and the combination drug A also exerts a therapeutic effect against a solid cancer. Therefore, the combination drug A exerts a high efficacy even against a solid cancer, which was difficult to treat by conventional methods.

In a situation in which the presence of cancer cells in a subject is suspected, the combination drug A according to the present disclosure may be prophylactically administered to the subject before a definitive diagnosis of cancer is made. In the present disclosure, such a mode of use is also included in the concept of use in treatment of a cancer.

<Method of Treating Cancer in Subject>

According to an aspect of the present disclosure, a method of treating a cancer in a subject that includes administering, in combination:

(a) an immunoresponsive cell expressing IL-7, CCL19, and a cell surface molecule that specifically recognizes a cancer antigen, and
(b) an immunosuppression inhibitor,

is provided (hereinafter also referred to as the "cancer treatment method A according to the present disclosure").

The immunoresponsive cell in the cancer treatment method A according to the present disclosure is the immunoresponsive cell A according to the present disclosure, and the above-described explanation regarding the immunoresponsive cell A according to the present disclosure applies, as it is, to detailed configuration and examples of the immunoresponsive cell in the cancer treatment method A according to the present disclosure. Also, the above-described explanation regarding the immunosuppression inhibitor in the combination drug A according to the present disclosure applies, as it is, to detailed configuration and examples of the immunosuppression inhibitor in the cancer treatment method A according to the present disclosure.

In addition, the explanation regarding the combination drug A according to the present disclosure applies, as it is, to the specifics of the treatment method in the cancer treatment method A according to the present disclosure, such as the subject, cancer type, dose, and administration schedule. For example, the (a) immunoresponsive cell and the (b) immunosuppression inhibitor may be administered at the same time, or administered at different times. The (a) immunoresponsive cell and the immunosuppression inhibitor (b) each may be administered in a therapeutically effective amount.

Due to a synergistic effect exerted by the combination of factors of the immunosuppression inhibitor and the immunoresponsive cell expressing IL-7, CCL19, and a cell surface molecule that specifically recognizes a cancer antigen, the cancer treatment method A according to the present disclosure produces a surprisingly improved therapeutic effect against cancer.

Further, according to the present disclosure, use of (a) an immunoresponsive cell expressing IL-7, CCL19, and a cell surface molecule that specifically recognizes a cancer antigen, and (b) an immunosuppression inhibitor in the manufacture of a drug for treating a cancer is provided. Also in this use, the explanation regarding the combination drug A according to the present disclosure applies, as it is, to the specifics of the immunoresponsive cell, the immunosuppression inhibitor, the administration compositions, the cancer treatment, and the like.

In addition, a drug for combined use with an immunosuppression inhibitor in treatment of a cancer in a subject is provided according to the present disclosure, the drug including an immunoresponsive cell expressing IL-7, CCL19, and a cell surface molecule that specifically recognizes a cancer antigen. The drug including the immunoresponsive cell A according to the present disclosure produces a synergistic effect and provides a surprising improved therapeutic effect against cancer when used in combination with an immunosuppression inhibitor. The above-described explanation regarding the combination drug A according to the present disclosure applies, as it is, to the specifics of the immunoresponsive cell, the immunosuppression inhibitor, the administration compositions, the cancer treatment, and the like. Further, an immunoresponsive cell for combined use with an immunosuppression inhibitor in treatment of a cancer in a subject is provided, the immunoresponsive cell expressing IL-7, CCL19, and a cell surface molecule that specifically recognizes a cancer antigen.

According to the present disclosure, a drug including an immunosuppression inhibitor for combined use with an immunoresponsive cell expressing IL-7, CCL19, and a cell surface molecule that specifically recognizes a cancer antigen, in treatment of a cancer in a subject is also provided. The drug including an immunosuppression inhibitor produces a synergistic effect and provides a surprising improved therapeutic effect against cancer when used in combination with the immunoresponsive cell A according to the present disclosure. The above-described explanation regarding the combination drug A according to the present disclosure applies, as it is, to the specifics of the immunoresponsive cell, the immunosuppression inhibitor, the administration compositions, the cancer treatment, and the like. An immunosuppression inhibitor for combined use with an immunoresponsive cell expressing IL-7, CCL19, and a cell surface molecule that specifically recognizes a cancer antigen in treatment of a cancer in a subject is also provided.

According to the present disclosure, a drug which includes an immunoresponsive cell expressing IL-7, CCL19, and a cell surface molecule that specifically recognizes a cancer antigen, and which drug is contained in a container carrying an indication of instruction for combined use with an immunosuppression inhibitor, is also provided. The above-described explanation regarding the combination drug A according to the present disclosure applies, as it is, to the specifics of the immunoresponsive cell, the immunosuppression inhibitor, and the like. The explanation regarding the aforementioned first administration composition applies, as it is, to the specifics of the drug configuration. The container carrying an indication of instruction for combined use with an immunosuppression inhibitor may be a container, such as a vial, an intravenous injection bag, a cell preservation bag, or an infusion bag, to which an indication of usage method is attached, or a container, such as an Eppendorf tube, to which an indication of usage method is attached. In such a container, the drug including the immunoresponsive cell A according to the present disclosure is contained, for example, in a state described in the foregoing explanation regarding the first administration composition. The indication of instruction for combined use with an immunosuppression inhibitor may be attached to any face of the container, and may be attached to an outer face of the container in consideration of visibility. A configuration is also contemplated in which the indication of instruction is attached to a case, such as a box, that accommodates one or more containers, instead of being attached to the container itself. Further, the indication of instruction for combined use with an immunosuppression inhibitor is not limited to an indication expressly instructing combined use with an immunosuppression inhibitor, and may be any indication that refers to the possibility of combined use with an immunosuppression inhibitor.

According to the present disclosure, a product including (i) a label describing an instruction for combined use with an immunosuppression inhibitor, and (ii) a container containing a drug including an immunoresponsive cell expressing IL-7, CCL19, and a cell surface molecule that specifically recognizes a cancer antigen, is also provided. The above-described explanation regarding the combination drug A according to the present disclosure applies, as it is, to the specifics of the immunoresponsive cell, the immunosuppression inhibitor, and the like. The explanation regarding the aforementioned first administration composition applies, as it is, to the specifics of the drug configuration. The container containing the immunoresponsive cell A according to the present disclosure may be a container, such as a vial, an intravenous injection bag, a cell preservation bag, or an infusion bag, to which an indication of usage method is attached, or a container, such as an Eppendorf tube, to which an indication of usage method is attached. In such a container, the drug including the immunoresponsive cell A according to the present disclosure is contained, for example, in a state described in the foregoing explanation regarding the first administration composition. The instruction for combined use with an immunosuppression inhibitor provided in the label is not limited to an express instruction for combined use with an immunosuppression inhibitor, and may be any instruction that refers to the possibility of combined use with an immunosuppression inhibitor.

As described above, according to an aspect of the present disclosure, a surprisingly improved therapeutic effect against cancer can be obtained due to a synergistic effect exerted by the combination of the immunosuppression inhibitor and the immunoresponsive cell expressing IL-7, CCL19, and a cell surface molecule that specifically recognizes a cancer antigen.

Embodiments according to the present disclosure include the following embodiments:

<1> A combination drug for use in treatment of a cancer in a subject, including:

(a) an immunoresponsive cell expressing interleukine-7, CCL19, and a cell surface molecule that specifically recognizes a cancer antigen; and
(b) an immunosuppression inhibitor.

<2> The combination drug according to <1>, wherein the immunoresponsive cell and the immunosuppression inhibitor are separately administered at different times.
<3> The combination drug according to <1> or <2>, wherein a gene encoding interleukin-7 and a gene encoding CCL19 are exogenous, and both of the genes are integrated into a genome of the immunoresponsive cell, or encoded together or separately in one or more vectors present in the immunoresponsive cell.
<4> The combination drug according to any one of <1> to <3>, wherein the cell surface molecule that specifically recognizes a cancer antigen is a chimeric antigen receptor (CAR) or a T-cell receptor (TCR).
<5> The combination drug according to any one of <1> to <4>, wherein the immunosuppression inhibitor includes at least one selected from the group consisting of a PD-1 inhibitor, a PD-L1 inhibitor, a PD-L2 inhibitor, a CTLA-4 inhibitor, a BTLA (B- and T-lymphocyte attenuator) inhibitor, a TIM-3 (T-cell immunoglobulin and mucin domain 3) inhibitor, a TIGIT (T-cell immunoreceptor with Ig and ITIM domains) inhibitor, a LAG-3 (Lymphocyte Activation Gene-3) inhibitor, and a Siglec-15 inhibitor.
<6> The combination drug according to any one of <1> to <5>, wherein the immunosuppression inhibitor is an antibody.
<7> The combination drug according to <6>, wherein the antibody is an IgG monoclonal antibody or an antibody fragment.
<8> The combination drug according to any one of <1> to <7>, wherein the cancer is a solid cancer.
<9> The combination drug according to any one of <1> to <8>, wherein the immunoresponsive cell is derived from the subject itself.
<10> The combination drug according to any one of <1> to <9>, wherein the immunoresponsive cell is selected from the group consisting of lymphocytic cells such as T cells, natural killer cells (NK cells), and B cells, antigen-presenting cells such as monocytes, macrophages, and dendritic cells, and neutrophils, eosinophils, basophils, and mast cells.
<11> A drug for combined use with an immunosuppression inhibitor in treatment of a cancer in a subject, the drug including an immunoresponsive cell expressing interleukin-7, CCL19, and a cell surface molecule that specifically recognizes a cancer antigen.
<12> A drug for combined use with an immunoresponsive cell expressing interleukin-7, CCL19, and a cell surface molecule that specifically recognizes a cancer antigen, in treatment of a cancer in a subject, the drug including an immunosuppression inhibitor.
<13> The drug according to <11> or <12> for use in a mode in which the immunosuppression inhibitor and the immunoresponsive cell are administered separately at different times.
<14> A drug including an immunoresponsive cell expressing interleukin-7, CCL19, and a cell surface molecule that specifically recognizes a cancer antigen, the drug being contained in a container carrying an indication of instruction for combined use with an immunosuppression inhibitor.
<15> A product including:

a label describing an instruction for combined use with an immunosuppression inhibitor, and
a container containing a drug including an immunoresponsive cell expressing interleukin-7, CCL19, and a cell surface molecule that specifically recognizes a cancer antigen.

<16> A pharmaceutical composition for use in treatment of a cancer in a subject, the pharmaceutical composition including:

(a) an immunoresponsive cell expressing interleukine-7, CCL19, and a cell surface molecule that specifically recognizes a cancer antigen; and
(b) an immunosuppression inhibitor.

<17> The pharmaceutical composition according to <16>, wherein the cell surface molecule that specifically recognizes a cancer antigen is a chimeric antigen receptor (CAR) or a T-cell receptor (TCR).
<18> A method of treating a cancer in a subject, the method including administering, to the subject, the following (a) and (b) in combination:

(a) an immunoresponsive cell expressing IL-7, CCL19, and a cell surface molecule that specifically recognizes a cancer antigen, and

(b) an immunosuppression inhibitor.

<19> Use of (a) an immunoresponsive cell expressing IL-7, CCL19, and a cell surface molecule that specifically recognizes a cancer antigen, and (b) an immunosuppression inhibitor in the manufacture of a drug for treating a cancer.

Further aspects according to the present disclosure are described below.

According to an aspect of the present disclosure, a combination drug for use in treatment of a cancer in a subject which includes:

(a) one or more kinds of cells, one or more kinds of nucleic acid delivery vehicles, or a combination thereof, which cooperatively include a nucleic acid encoding interleukine-7 and a nucleic acid encoding CCL19; and
(b) an immunosuppression inhibitor

is provided (hereinafter also referred to as "combination drug B according to the present disclosure").

Here, the specifics of the interlukin-7, the CCL19, the nucleic acid encoding interleukin-7, the nucleic acid encoding CCL19, and the immunosuppression inhibitor, such as definitions, examples, amino acid sequences, base sequences, and preferable embodiments, in the combination drug B are respectively the same as the specifics of the interlukin-7, the CCL19, the nucleic acid encoding interleukin-7, the nucleic acid encoding CCL19, and the immunosuppression inhibitor, such as definitions, examples, amino acid sequences, base sequences, and preferable embodiments, in the immunoresponsive cell A according to the present disclosure and the combination drug A according to the present disclosure.

In the present disclosure, the expression, "cooperatively include", means that, in a case in which there are plural elements (for example, plural cells, plural nucleic acid delivery vehicles, or one or more cells and one or more nucleic acid delivery vehicles), the substance described as a substance to be included (for example, a nucleic acid encoding a specified polypeptide) is included in at least one of the plural elements, and it is not necessary for all of the plural elements to include the substance. In other words, "cooperatively include" can also be expressed as "include as a whole". The scope of the meaning of the expression, "cooperatively include", encompasses a case in which all of plural substances described as substances to be included are included in a single cell or nucleic acid delivery vehicle, and this configuration is also a preferable configuration.

Therefore, in a case in which the combination drug B according to the present disclosure includes a cell I and a cell II, a configuration may be adopted in which the cell I includes a nucleic acid encoding interleukin-7 but does not include a nucleic acid encoding CCL19, and in which the cell II includes a nucleic acid encoding CCL19 but does not include a nucleic acid encoding interleukin-7. Similarly, in a case in which the combination drug B according to the present disclosure includes a nucleic acid delivery vehicle I and a nucleic acid delivery vehicle II, a configuration may be adopted in which the nucleic acid delivery vehicle I includes a nucleic acid encoding interleukin-7 but does not include a nucleic acid encoding CCL19, and in which the nucleic acid delivery vehicle II includes a nucleic acid encoding CCL19 but does not include a nucleic acid encoding interleukin-7.

Alternatively, the combination drug B according to the present disclosure may include a single cell or nucleic acid delivery vehicle that include both of a nucleic acid encoding interleukin-7 and a nucleic acid encoding CCL19.

The term, "combination thereof", in the expression, "one or more kinds of cells, one or more kinds of nucleic acid delivery vehicles, or a combination thereof, which cooperatively include a nucleic acid encoding interleukine-7 and a nucleic acid encoding CCL19", refers to a combination which is a combination of one or more kinds of cells and one or more kinds of nucleic acid delivery vehicles, and in which a nucleic acid encoding interleukin-7 is included in at least one of the one or more kinds of cells or one or more kinds of nucleic acid delivery vehicles, and a nucleic acid encoding CCL19 is included in at least one of the one or more kinds of cells or one or more kinds of nucleic acid delivery vehicles.

In the present disclosure, the term "polypeptide" generally refers to a polymer in which amino acid residues are connected via peptides bonds, and a so-called protein is also included as an example of the polypeptide. The number of amino acid residues in the polypeptide may be 10 or more, 20 or more, 30 or more, 40 or more, 50 or more, 70 or more, or 100 or more. The upper limit of the number of amino acid residues is not particularly limited. The number of amino acid residues may be 10,000 or less, 5000 or less, 2000 or less, 1000 or less, 500 or less, 200 or less, 100 or less, 70 or less, 50 or less, 40 or less, 30 or less, or 20 or less. The foregoing lower limit values and the upper limit values may freely be combined to form ranges, as far as contradiction does not occur.

In the combination drug B according to the present disclosure, the cell is not particularly limited as long as the cell is capable of expressing the nucleic acid that is described as a nucleic acid to be included (for example, a nucleic acid encoding interleukin-7 and/or a nucleic acid encoding CCL19). The cell is preferably a cell that accumulates around cancer cells or infiltrates into a region around cancer cells, when the cell has been introduced into the body. When two or more nucleic acids that are described as nucleic acids to be included are present in a cell, each of these nucleic acids may be independently included in a state of being integrated into the genome or being retained on a plasmid in the cell, or these nucleic acids may be linked to each other and included in a state of being integrated into the genome or being retained on a plasmid in the cell. Examples of the cell include immunoresponsive cells, cells of anaerobic microorganisms, and mesenchymal stem cells (MSCs).

In the combination drug B according to the present disclosure, the nucleic acid delivery vehicle is not particularly limited as long as it is a nucleic acid delivery vehicle capable of delivering a nucleic acid that is described as a nucleic acid to be included (for example, a nucleic acid encoding interleukin-7 and/or a nucleic acid encoding CCL19) into a cell, to cause expression of the nucleic acid. The cell is preferably a human cell, more preferably a cell in a human body, and is still more preferably a cancer cell or an immunoresponsive cell in a human body. In other words, the nucleic acid delivery vehicle is preferably a nucleic acid delivery vehicle that delivers a nucleic acid into a cancer cell or an immunoresponsive cell when the nucleic acid delivery vehicle has been introduced into the body.

Examples of the nucleic acid delivery vehicle include viruses, liposomes, and nanoparticles. Nucleic acid delivery vehicles known in the art may be used according to ordinary methods. It is also possible to use a virus vector as the nucleic acid delivery vehicle.

When two or more nucleic acids that are described as nucleic acids to be included are present in a nucleic acid delivery vehicle, each of these nucleic acids may be included in the nucleic acid delivery vehicle in a mutually independent state, or in a mutually linked state.

Since cancer cells have an immunosuppressive mechanism that suppresses immunoresponsive cells' actions to attack the cancer cells or to send instructions for attacking the cancer cells, attack by the cancer-suffering person's own immune system against the cancer cells is suppressed. It is conceivable that the immunosuppression inhibitor, which is one component of the combination drug B according to the present disclosure, makes it easier for the immune system of a cancer-suffering person to attack cancer cells, by means of suppressing the immunosuppressive mechanism exerted by the cancer cells.

In addition, it is conceivable that one or more kinds of cells, one or more kinds of nucleic acid delivery vehicles, or a combination thereof, which cooperatively include a nucleic acid encoding interleukine-7 and a nucleic acid encoding CCL19 express IL-7 in the vicinity of a cancer tissue or causes a cell (typically, a cell in the body) to express IL-7 in the vicinity of the cancer tissue via nucleic acid delivery, and further expresses CCL19 in the vicinity of the cancer tissue or causes a cell (typically, a cell in the body) to express CCL19 in the vicinity of the cancer tissue via nucleic acid delivery, as a result of which endogenous immunoresponsive cells in the cancer-suffering person accumulate around cancer cells, thereby enabling more efficient attack against the cancer cells. From this viewpoint, the cell toward which the nucleic acid delivery is performed is preferably a cancer cell in the body.

It is conceivable that the reason why the combination drug B according to the present disclosure exerts a significantly improved therapeutic effect against cancer is that the combination drug B exerts a synergistic effect exerted by the combination of factors of the immunosuppression inhibitor and the expressed IL-7 and CCL19, due to inclusion of the immunosuppression inhibitor and the one or more kinds of cells, one or more kinds of nucleic acid delivery vehicles, or combination thereof, which cooperatively include a nucleic acid encoding interleukine-7 and a nucleic acid encoding CCL19. This synergistic effect is superior to a degree that cannot be predicted from the individual effects of the respective factors.

In an embodiment, the one or more kinds of cells or one or more kinds of nucleic acid delivery vehicles in the combination drug B delivers the nucleic acids to a cell, such as an immunoresponsive cell, other than cancer cells. In this embodiment, the one or more kinds of cells, one or more kinds of nucleic acid delivery vehicles, or combination thereof in the combination drug B according to the present disclosure may further include a nucleic acid encoding a cell surface molecule that specifically recognizes a cancer antigen. In this case, the cell or nucleic acid delivery vehicle that includes the nucleic acid encoding a cell surface molecule that specifically recognizes a cancer antigen may or may not include the nucleic acid encoding interleukin-7, and may or may not include a nucleic acid encoding CCL19. That is, when the one or more kinds of cells, one or more kinds of nucleic acid delivery vehicles, or combination thereof in the combination drug B according to the present disclosure further includes a nucleic acid encoding a cell surface molecule that specifically recognizes a cancer antigen, it is sufficient for the one or more kinds of cells, one or more kinds of nucleic acid delivery vehicles, or combination thereof to cooperatively include (in other words, include as a whole) the nucleic acid encoding interleukin-7, the nucleic acid encoding CCL19, and the nucleic acid encoding the cell surface molecule that specifically recognizes a cancer antigen.

In a case in which the one or more kinds of cells, one or more kinds of nucleic acid delivery vehicles, or combination thereof in the combination drug B according to the present disclosure causes the cell surface molecule that specifically recognizes a cancer antigen to be further expressed by the cell to which the nucleic acids have been delivered, the therapeutic effect against cancer can be further enhanced. For example, in a case in which a nucleic acid encoding a cell surface molecule that specifically recognizes a cancer antigen is included in a nucleic acid delivery vehicle, the nucleic acid delivery vehicle may be administered to a subject to introduce the nucleic acid into a T cell in the subject's body, thereby causing expression of the cell surface molecule on the T cell.

In an embodiment, the one or more kinds of cells or one or more kinds of nucleic acid delivery vehicles in the combination drug B have, on a surface thereof, a molecule that specifically recognizes a cancer cell, in order to deliver the nucleic acids into cancer cells. The specifics of the molecule that specifically recognizes a cancer cell, such as examples and preferable embodiments, are the same as the specifics, such as examples and preferable embodiments, of the cell surface molecule that specifically recognizes a cancer cell.

When the one or more kinds of cells or one or more kinds of nucleic acid delivery vehicles in the combination drug B are one or more kinds of cells, for example, a nucleic acid encoding a cell surface molecule that specifically recognizes a cancer antigen may be introduced into a cell, and the cell may be cultured before administration of the combination drug B, whereby scFv or the like that specifically recognizes a cancer antigen can be expressed on a surface of the cell.

When the one or more kinds of cells or one or more kinds of nucleic acid delivery vehicles in the combination drug B are one or more kinds of viruses, for example, a nucleic acid encoding a cell surface molecule that specifically recognizes a cancer antigen may be incorporated into the genome of a virus, and the virus may be transfected into an appropriate cell to cause production and proliferation of the virus, whereby scFv or the like that specifically recognizes a cancer antigen can be expressed on a surface of the cell. An antibody such as scFv may be included in the envelop or capsid of the virus. For example, when herpes virus is used, envelope glycoprotein gD, which is responsible for invasion of herpes virus, may be modified so as to be unable to bind to an original receptor thereof, and an antibody, such as scFv, that specifically recognizes a cancer antigen may be inserted into the envelope glycoprotein gD.

Further, when the one or more kinds of cells or one or more kinds of nucleic acid delivery vehicles in the combination drug B are one or more kinds of liposomes or nanoparticles, a molecule that specifically recognizes a cancer cell may be attached, in advance, to the liposomes or nanoparticles using known methods or methods obvious therefrom.

The specifics, such as definitions, examples, amino acid sequences, base sequences, and preferable embodiments, of the cell surface molecule that specifically recognizes a cancer antigen and the nucleic acid encoding a cell surface molecule that specifically recognizes a cancer antigen are respectively the same as the specifics, such as definitions, examples, amino acid sequences, base sequences, and preferable embodiments, of the cell surface molecule that specifically recognizes a cancer antigen and the gene encoding a cell surface molecule that specifically recognizes a cancer antigen in the immunoresponsive cell A according to the present disclosure and the combination drug A according to the present disclosure.

The nucleic acid encoding a cell surface molecule that specifically recognizes a cancer antigen is preferably included in an immunoresponsive cell. In other words, the one or more kinds of cells, one or more kinds of nucleic acid delivery vehicles, or combination thereof in the combination drug B according to the present disclosure preferably include an immunoresponsive cell including a nucleic acid encoding a cell surface molecule that specifically recognizes a cancer antigen. The immunoresponsive cell may or may not include a nucleic acid encoding interleukin-7, and may or may not include a nucleic acid encoding CCL19. The cell surface molecule that specifically recognizes a cancer antigen is preferably a molecule that imparts, to a cell, a specific capability to recognize cancer, upon expression of the molecule on the cell surface, and the examples thereof include a T-cell receptor (TCR) that specifically recognize a cancer antigen, and a chimeric antigen receptor (CAR) that specifically recognize a cancer antigen.

In consideration of the foregoing, in an embodiment, it can be said that the combination drug B according to the present disclosure may be used in combination with treatment using a CAR-T or a TCR-T.

In one embodiment, the one or more kinds of cells, one or more kinds of nucleic acid delivery vehicles, or combination thereof, which cooperatively include a nucleic acid encoding interleukine-7 and a nucleic acid encoding CCL19 may include an immunoresponsive cell that expresses interleukin-7, CCL19, and a cell surface molecule that specifically recognizes a cancer antigen. The immunosuppression inhibitor may be an immunosuppression inhibiting polypeptide expressed by a cell, and the combination drug B according to the present disclosure may include a cell that expresses an immunosuppression inhibiting polypeptide. Examples of the cell include the later-described cells that are described as examples of a cell in the one or more kinds of cells, one or more kinds of nucleic acid delivery vehicles, or combination thereof, which cooperatively include a nucleic acid encoding interleukine-7 and a nucleic acid encoding CCL19. A configuration in which a nucleic acid delivery vehicle including a nucleic acid encoding an immunosuppression inhibiting polypeptide is included instead of the immunosuppression inhibitor in the combination drug B according to the present disclosure is also within the scope of embodiments according to the present disclosure. The nucleic acid delivery vehicle may be the same nucleic acid delivery vehicle as, or a different nucleic acid delivery vehicle from, a nucleic acid delivery vehicle or nucleic acid delivery vehicles that include the other nucleic acids.

Accordingly, in an embodiment of the present disclosure, the immunosuppression inhibitor is a polypeptide, and the one or more kinds of cells, one or more kinds of nucleic acid delivery vehicles, or combination thereof, cooperatively further include a nucleic acid encoding an immunosuppression inhibiting polypeptide.

When the combination drug B according to the present disclosure includes an immunoresponsive cell expressing interleukin-7, CCL19, and a cell surface molecule that specifically recognizes a cancer antigen, and a cell expressing an immunosuppression inhibiting polypeptide, these cells may be the same cell as each other or different cells from each other. When the immunoresponsive cell expressing interleukin-7, CCL19, and a cell surface molecule that specifically recognizes a cancer antigen and the cell expressing an immunosuppression inhibiting polypeptide are the same immunoresponsive cell, the combination drug B according to the present disclosure would include the one or more kinds of cells, one or more kinds of nucleic acid delivery vehicles, or combination thereof, which cooperatively include a nucleic acid encoding interleukine-7 and a nucleic acid encoding CCL19, and the immunosuppression inhibitor, by means of including the immunoresponsive cell. In the combination drug B according to the present disclosure, it is of course permissible that the immunosuppression inhibitor is included as a component independent from the one or more kinds of cells, one or more kinds of nucleic acid delivery vehicles, or combination thereof, which cooperatively include a nucleic acid encoding interleukine-7 and a nucleic acid encoding CCL19. In other words, the immunosuppression inhibitor may be included as a separately-added substance in the combination drug B according to the present disclosure, rather than as a substance expressed by the one or more kinds of cells, one or more kinds of nucleic acid delivery vehicles, or combination thereof.

As described above, the cells, nucleic acid delivery vehicles, or combination thereof in the combination drug B according to the present disclosure may be at least one selected from the group consisting of immunoresponsive cells, viruses, anaerobic microorganisms, liposomes, mesenchymal stem cells (MSCs), and nanoparticles, and it is also possible to use a mixture of two or more selected from these.

Here, the immunoresponsive cell is not particularly limited as long as it is a cell that is involved in immune responses, and that is capable of including and expressing a nucleic acid to be included (for example, a nucleic acid encoding interleukin-7 and/or a nucleic acid encoding CCL19). The nucleic acid delivered may be included in the genome of the immunoresponsive cell, or may be retained on a vector outside the genome. The nucleic acid delivered may be included in the genome from the viewpoint of the stability of gene retaining. The immunoresponsive cell is preferably an immunoresponsive cell collected from a living body, and examples thereof include lymphocytic cells such as T cells, natural killer cells (NK cells), and B cells, antigen-presenting cells such as monocytes, macrophages, and dendritic cells, and granulocytes such as neutrophils, eosinophils, basophils, and mast cells. Preferable examples thereof include a T cell collected from a mammalian animal such as a human, a dog, a cat, a pig, or a mouse, and more preferable examples include a T cell collected from a human. In the case of using a cell population, including T cells, that is collected from a living body, the cell population may include other cells in addition to T cells, and may include T cells in a proportion of 50% or more, 60% or more, 70% or more, 80% or more, or 90% or more with respect to the total number of cells, based on cell counts. Immunoresponsive cells, such as T cells, can be obtained by collecting a cell population that include immunoresponsive cells, from body fluid such as blood or bone marrow aspirate, or from immune cells that infiltrate into a tissue such as the spleen, the thymus, or lymph nodes, or from immune cells that infiltrate into a cancer tissue such as primary tumor, metastatic tumor, or cancerous ascites. The separated cell population may be subjected, if necessary, to an isolation step or a purification step according to an ordinary method, with a view to increasing the proportion of immunoresponsive cells, such as T cells, included in the cell population. The immunoresponsive cell may be an immunoresponsive cell prepared from an ES cell or an iPS cell. Examples of T cells, which are examples of immunoresponsive cells, include alpha-beta T cells, gamma-delta T cells, $CD8^+$ T cells, $CD4^+$ T cells, tumor infiltrating T cells, memory T cells, naive T cells, and NKT cells. The individual from which the immunoresponsive cell is obtained and the individual to which the combination drug is to be administered may be the same individual or different individuals, preferably the same individual. For example, when the subject to which administration is performed is a human, the immunoresponsive cell may be an autologous cell collected from the patient himself/herself to which administration is performed, or an allogenic cell collected from another person. That is, the donor and the recipient may be the same as each other or different from each other, and are preferably the same as each other.

The virus as a nucleic acid delivery vehicle is preferably a virus that is capable of encapsulating a nucleic acid to be delivered (for example, a nucleic acid encoding interleukin-7 and/or a nucleic acid encoding CCL19), and with which cancer cells can be infected. The virus is more preferably an oncolytic virus. The oncolytic virus means a virus that hardly proliferates when normal cells are infected therewith, but proliferates when cancer cells are infected therewith, thus having the ability to kill cancer cells (being cytotoxic against cancer cells). A review of the oncolytic virus is provided in Molecular Therapy vol. 18, no. 2 (February, 2010) pp. 233 to 234. The oncolytic virus is not particularly limited as long as it has the ability to kill cancer cells by infecting the cancer cells, and examples thereof include oncolytic vaccinia virus, oncolytic adenovirus, oncolytic herpes simplex virus, oncolytic reovirus, oncolytic measles virus, oncolytic Newcastle disease virus, oncolytic cowpox virus, oncolytic mumps virus, and oncolytic coxsackie virus.

Example of the oncolytic vaccinia virus include, but are not limited to, vaccinia viruses described in Kim MK et al., Science Translational Medicine, 2013 May 15; 5(185): 185ra63, Heo J, et al., Nature Medicine, 2013 (3): 329-36. doi: 10.1038/nm. 3089. Epub 2013 Feb 10, and WO 2012/094386. Examples of the oncolytic adenovirus include, but are not limited to, adenoviruses described in Tedcastle A et al. Mol. Ther. 2016; 24: 796-804, Marino N, Illingworth S, Kodialbail P, Patel A, Calderon H, Lear R, Fisher KD, Champion BR, Brown ACN. PLoS One 2017; 12(5): e0177810, Freedman JD, et al. EMBO Mol. Med. 9: 1067-1087 (2017), Lang FF et al., Journal of Clinical Oncology (2018), James M. et al., The Journal of Oncology, 188(6): 2391-7, 2012, and Japanese Patent Nos. 3867968 and 5574284. Examples of the oncolytic herpes simplex virus include, but are not limited to, herpes simplex viruses described in Mazzacurati et al., Mol. Ther., 2015 Jan; 23(1): 99-107, Hirooka Y, et al., BMC Cancer 2018, 18, 596, Nakatake R, et al., Cancer Sci. 2018 Mar, 109(3); 600-610, and Andtbacka RHI, et al., J. Clin. Oncol. 2015; 33: 2780-2788. Examples of the oncolytic reovirus include, but are not limited to, a reoviruse described in Mahalingam, et al., Cancers 2018, 10, 160. Examples of the oncolytic Newcastle disease virus include, but are not limited to, a Newcastle disease virus described in Journal of Virology, 2016 Jun; 90(11): 5343-5352. Examples of oncolytic vesicular stomatitis virus include, but are not limited to, a vesicular stomatitis virus described in Muik A. et al., Cancer Res; 74(13); 3567-78. Some oncolytic viruses have a protein expression function imparted by genetic modification. A polypeptide encoded by a nucleic acid to be delivered (for example, a nucleic acid encoding interleukin-7 and/or a nucleic acid encoding CCL19) may be expressed as a protein expressed based on the imparted protein expression function.

An aerobic microorganism as a cell in the "one or more kinds of cells, one or more kinds of nucleic acid delivery vehicles, or combination thereof, which cooperatively include a nucleic acid encoding interleukine-7 and a nucleic acid encoding CCL19" is not particularly limited as long as it is a cell of an aerobic microorganism capable of including and expressing a nucleic acid to be included (for example, a nucleic acid encoding interleukin-7 and/or a nucleic acid encoding CCL19). The anaerobic microorganism is preferably an anaerobic gram-negative bacterium having the ability to accumulate at cancer cells, and examples thereof include *Bifidobacterium* bacteria such as *Bifidobacterium bifidum, Lactobacillus* bacteria, and *Listeria* bacteria. Since anaerobic microorganisms more easily grow in a low-oxygen environment, it is known that anaerobic microorganisms have a tendency to accumulate at cancer cells. The anaerobic microorganism can be incorporated into a cell in the body of a subject to which the combination drug B according to the present disclosure is administered.

A liposome as a nucleic acid delivery vehicle is not particularly limited as long as it is a lipid nanocapsule that is formed from a phospholipid bilayer and is capable of encapsulating a nucleic acid to be delivered (for example, a nucleic acid encoding interleukin-7 and/or a nucleic acid encoding CCL19). The liposome may be a commercially available product, or a liposome synthesized using known methods. The liposome may be modified with PEG, or may have a targeting probe molecule such as a lectin or a protein (for example, an antibody) on a surface thereof, in order to improve accumulation at cancer cells.

A mesenchymal stem cell (MSC) as a cell in the "one or more kinds of cells, one or more kinds of nucleic acid delivery vehicles, or combination thereof, which cooperatively include a nucleic acid encoding interleukine-7 and a nucleic acid encoding CCL19" is not particularly limited as long as it is an MSC capable of including and expressing a nucleic acid to be included (for example, a nucleic acid encoding interleukin-7 and/or a nucleic acid encoding CCL19). The MSC is preferably a MSC that accumulates at cancer cells.

A nanoparticle as a nucleic acid delivery vehicle is not particularly limited as long as it is a particulate matter at a nanometer order, preferably having a diameter of from 5 to 800 nm, that is capable of delivering a nucleic acid to be delivered (for example, a nucleic acid encoding interleukin-7 and/or a nucleic acid encoding CCL19) to a cancer cell. Examples of the nanoparticle include metal nanoparticles such as gold nanoparticles, and silica nanoparticles. Such nanoparticles may be a commercially available product, or nanoparticles synthesized using ordinary methods. The nanoparticle can reach a cancer cell due to an enhanced permeability and retention effect (EPR effect). Depending on the material of the nanoparticle, the nucleic acid may be included in the nanoparticle, by being bound to a surface of the nanoparticle, or being encapsulated in the nanoparticle.

In the combination drug B according to the present disclosure, the nucleic acid encoding interleukin-7 and the nucleic acid encoding CCL19, each of which is included in a cell or a nucleic acid delivery vehicle,, and the nucleic acid encoding a cell surface molecule that specifically recognizes a cancer antigen, which is optionally included, are each preferably operably linked at the downstream of a promoter. The procedure for incorporating the nucleic acid into the cell or the nucleic acid delivery vehicle may be carried out using ordinary methods. In the case of introducing a nucleic acid into a cell, the introduction may be carried out using, for example, a method selected from the group consisting of an electroporation method (see, for example, Cytotechnology, 3, 133 (1990)), a calcium phosphate method (see, for example, Japanese Patent Application Laid-open (JP-A) No. H2-227075), a lipofection method (see, for example, Proc. Natl. Acad. Sci. U.S.A., 84, 7413 (1987)), and a virus infection method. The virus infection method may be a method including preparing a recombinant virus by transfecting a packaging cell, such as a GP-2-293 cell (manufactured by Takara Bio Inc.), a Plat-GP cell (manufactured by Cosmo Bio Co., Ltd.), a PG13 cell (ATCC CRL-10686), or a PA317 cell (ATCC CRL-9078), with a vector including a nucleic acid to be introduced and a packaging plasmid, and infecting a T cell with the recombinant virus (see, for example, International Publication (WO) No. 2017/159736).

The nucleic acid to be introduced may be integrated into the genome of a cell, using a known gene editing technique, such that the nucleic acid can be expressed under control exerted by an appropriate promoter. Examples of known gene editing techniques include a technique using an endonuclease such as zinc finger nuclease, TALEN (transcription activator-like effector nuclease), or a CRISPR (clustered regulatory interspaced short palindromic repeat)-Cas system.

In a case in which the nucleic acid to be included is included in a cell, the nucleic acid may be integrated into the genome of the cell, or may be retained on a vector within the cell. In a case in which two or more nucleic acids to be included are included in the same cell, those nucleic acids may be integrated, adjacent to each other, into the genome of the cell, or integrated separately into the genome of the cell, or included in the sane vector, or separately included in different vectors. A configuration may be adopted in which some of the nucleic acids to be included are included in the genome, and the other of the nucleic acids to be included are included in a vector or vectors.

In a case in which the nucleic acid to be included is included in a nucleic acid delivery vehicle, the nucleic acid may be included by itself, or may be retained on a vector. In a case in which two or more nucleic acids to be included are included in the same nucleic acid delivery vehicle, those nucleic acids may be included in the same vector or separately included in separate vectors, or may be included in the same virus or included in separate viruses. Different nucleic acids may be included in the same nucleic acid delivery vehicle, or in separate nucleic acid delivery vehicles.

The vector may be linear or circular, and may be a non-virus vector such as a plasmid, a virus vector, or a transposon vector. The vector may include one or more of a regulatory sequence such as a promoter or a terminator, or a selection marker sequence such as a drug-resistant gene or a reporter gene. Expression of the gene included in the vector may be performed utilizing a promoter contained in the vector.

The combination drug B according to the present disclosure exhibits a surprisingly improved therapeutic effect against cancer, due to a synergistic effect exerted by the combination of factors of IL-7, CCL19, and the immunosuppression inhibitor. The effect is more conspicuous in a case in which the combination drug B according to the present disclosure includes a cell or nucleic acid delivery vehicle that includes a nucleic acid encoding a cell surface molecule that specifically recognizes a cancer antigen, and still more conspicuous in a case in which the combination drug B includes an immunoresponsive cell that includes a nucleic acid encoding a cell surface molecule that specifically recognizes a cancer antigen.

In the combination drug B according to the present disclosure, an administration composition (hereinafter also referred to as the "third administration composition") including one or more kinds of cells, one or more kinds of nucleic acid delivery vehicles, or a combination thereof, which cooperatively include a nucleic acid encoding interleukine-7 and a nucleic acid encoding CCL19, may further include a pharmaceutically acceptable additive, and examples of the additive include physiological saline, buffered saline, cell culture medium, dextrose, water for injection, glycerol, ethanol, and combinations thereof, a stabilizer, a solubilizer, a surfactant, a buffering agent, a preservative, an isotonization agent, a filler, and a lubricant.

The description of the administration compositions in the combination drug A according to the present disclosure can be applied to the administration compositions for administration of the combination drug B according to the present disclosure, while replacing the first administration composition by the third administration composition. For example, the second administration composition may be the same composition as the third administration composition, in which case one administration composition would include both of (i) the one or more kinds of cells, one or more kinds of nucleic acid delivery vehicles, or combination thereof and (ii) the immunosuppression inhibitor. In this case, a pharmaceutical composition that includes both of (i) one or more kinds of cells, one or more kinds of nucleic acid delivery vehicles, or a combination thereof, which cooperatively include a nucleic acid encoding interleukine-7 and a nucleic acid encoding CCL19, and (ii) the immunosuppression inhibitor (a combined drug) is provided.

When the second administration composition is a composition separate from the third administration composition, the third administration composition and the second administration composition may be administered together, or may be administered at different times (different points in time), as described above.

Thus, the one or more kinds of cells, one or more kinds of nucleic acid delivery vehicles, or combination thereof and the immunosuppression inhibitor may be separately administered at different points in time.

Here, the description of the amount of the immunoresponsive cell A according to the present disclosure in the explanation of the administration compositions in the combination drug A according to the present disclosure will be applied, as the description of the cell amount in the third administration composition when the one or more kinds of cells, one or more kinds of nucleic acid delivery vehicles, or combination thereof include a cell, to the explanation of the administration compositions in the combination drug B according to the present disclosure.

Further, the explanation of the combination drug A according to the present disclosure can be applied to the combination drug B according to the present disclosure while replacing the combination drug A according to the present disclosure by the combination drug B according to the present disclosure, and replacing the immunoresponsive cell A according to the present disclosure and the "immunoresponsive cell expressing IL-7, CCL19, and a cell surface molecule that specifically recognizes a cancer antigen" by the one or more kinds of cells, one or more kinds of nucleic acid delivery vehicles, or combination thereof, which cooperatively include a nucleic acid encoding interleukine-7 and a nucleic acid encoding CCL19, unless contradiction arises. The applicable explanation includes the explanation regarding a mode of use of the combination drug, such as the organism species, individual, and type of disease to be treated, dose, and administration schedule.

When the one or more kinds of cells, one or more kinds of nucleic acid delivery vehicles, or combination thereof include a nucleic acid delivery vehicle, the amount of the nucleic acid delivery vehicle in the third administration composition may be adjusted, as appropriate, in accordance with, for example, the type, position, or severity of the cancer, or the age, body weight, or condition of the subject to be treated, and the amount of the nucleic acid delivery vehicle may be an amount appropriate for delivering a therapeutically effective amount of the nucleic acid to be delivered.

The explanation regarding the treatment of a cancer using the combination drug A according to the present disclosure can be applied to the treatment of a cancer using the combination drug B according to the present disclosure, while replacing the combination drug A according to the present disclosure by the combination drug B according to the present disclosure, and replacing the immunoresponsive cell A according to the present disclosure and the immunoresponsive cell expressing IL-7, CCL19, and a cell surface molecule that specifically recognizes a cancer antigen by the one or more kinds of cells, one or more kinds of nucleic acid delivery vehicles, or combination thereof, which cooperatively include a nucleic acid encoding interleukine-7 and a nucleic acid encoding CCL19. For example, according to an aspect of the present disclosure, a method of treating a cancer in a subject, including administering, to the subject, the following (a) and (b) in combination:

(a) one or more kinds of cells, one or more kinds of nucleic acid delivery vehicles, or combination thereof, which cooperatively include a nucleic acid encoding interleukine-7 and a nucleic acid encoding CCL19, and
(b) an immunosuppression inhibitor

is provided (hereinafter also referred to as the "cancer treatment method B according to the present disclosure").

Further, according to the present disclosure, use of (a) one or more kinds of cells, one or more kinds of nucleic acid delivery vehicles, or a combination thereof, which cooperatively include a nucleic acid encoding interleukine-7 and a nucleic acid encoding CCL19, and (b) an immunosuppression inhibitor in the manufacture of a drug for treating a cancer is provided. In addition, a drug for combined use with an immunosuppression inhibitor in treatment of a cancer in a subject is provided, the drug including one or more kinds of cells, one or more kinds of nucleic acid delivery vehicles, or a combination thereof, which cooperatively include a nucleic acid encoding interleukine-7 and a nucleic acid encoding CCL19. Further, one or more kinds of cells, one or more kinds of nucleic acid delivery vehicles, or a combination thereof, which cooperatively include a nucleic acid encoding interleukine-7 and a nucleic acid encoding CCL19, for combined use with an immunosuppression inhibitor in treatment of a cancer in a subject is provided. The one or more kinds of cells, one or more kinds of nucleic acid delivery vehicles, or combination thereof, which cooperatively include a nucleic acid encoding interleukine-7 and a nucleic acid encoding CCL19, and the immunosuppression inhibitor may be administered together, or administered separately at different times.

Further, according to the present disclosure, a drug including an immunosuppression inhibitor for combined use with one or more kinds of cells, one or more kinds of nucleic acid delivery vehicles, or a combination thereof, which cooperatively include a nucleic acid encoding interleukine-7 and a nucleic acid encoding CCL19, in treatment of a cancer in a subject is also provided. An immunosuppression inhibitor for combined use with one or more kinds of cells, one or more kinds of nucleic acid delivery vehicles, or a combination thereof, which cooperatively include a nucleic acid encoding interleukine-7 and a nucleic acid encoding CCL19, in treatment of a cancer in a subject is also provided.

According to the present disclosure, a drug that includes one or more kinds of cells, one or more kinds of nucleic acid delivery vehicles, or a combination thereof, which cooperatively include a nucleic acid encoding interleukine-7 and a nucleic acid encoding CCL19, is provided, the drug being contained in a container carrying an indication of instruction for combined use with an immunosuppression inhibitor. The specifics of the container, the indication, and the like are as described above. In addition, a product including (i) a label describing an instruction for combined use with an immunosuppression inhibitor, and (ii) a container containing a drug including one or more kinds of cells, one or more kinds of nucleic acid delivery vehicles, or a combination thereof, which cooperatively include a nucleic acid encoding interleukine-7 and a nucleic acid encoding CCL19, is also provided. The specifics of the container, the label, and the like are as described above.

As described above, according to an embodiment of the present disclosure, a surprisingly improved therapeutic effect against cancer can be obtained due to a synergistic effect exerted by the combination of the immunosuppression inhibitor and the one or more kinds of cells, one or more kinds of nucleic acid delivery vehicles, or combination thereof, which cooperatively include a nucleic acid encoding interleukine-7 and a nucleic acid encoding CCL19.

According to a further aspect of the present disclosure, an immunoresponsive cell expressing interleukin-7, CCL19, an immunosuppression inhibiting polypeptide, and a cell surface molecule that specifically recognizes a cancer antigen (hereinafter also referred to as "immunoresponsive cell C according to the present disclosure") is provided.

Here, the specifics, such as definitions, examples, amino acid sequences, base sequences, and preferable embodiments, of the cell surface molecule that specifically recognizes a cancer antigen, interleukin-7, CCL19, the nucleic acid encoding a cell surface molecule that specifically recognizes a cancer antigen, the nucleic acid encoding interleukin-7, the nucleic acid encoding CCL19, and the immunosuppression inhibitor in the immunoresponsive cell C according to the present disclosure are respectively the same as the specifics, such as definitions, examples, amino acid sequences, base sequences, and preferable embodiments, of the cell surface molecule that specifically recognizes a cancer antigen, interleukin-7, CCL19, the nucleic acid encoding a cell surface molecule that specifically recognizes a cancer antigen, the nucleic acid encoding interleukin-7, the nucleic acid encoding CCL19, and the immunosuppression inhibitor in the immunoresponsive cell A according to the present disclosure and the combination drug A according to the present disclosure.

The immunoresponsive cell C according to the present disclosure can be considered to be a cell that is an immunoresponsive cell A according to the present disclosure and that further expresses an immunosuppression inhibiting polypeptide. Therefore, the matters that have already explained in the explanation of the immunoresponsive cell A (such as the explanations regarding interleukin-7, CCL19, and the cell surface molecule that specifically recognizes a cancer antigen) are the same as those in the explanation of the immunoresponsive cell A, and duplicate explanations thereof are omitted.

The immunosuppression inhibiting polypeptide refers a substance that is a polypeptide and that is within the scope of the immunosuppression inhibitor in the combination drug A according to the present disclosure. The immunosuppression inhibiting polypeptide cancels or reduces suppression of activation of immunoresponsive cells.

Examples of the immunosuppression inhibiting polypeptide include an immune checkpoint inhibiting polypeptide, a polypeptide that inhibits infiltration, survival, or function of an immunosuppressive cell such as Treg or a myeloid-derived suppressor cell (MDSC), a CCR4 inhibiting polypeptide, an indoleamine-2,3-dioxygenase (IDO) inhibiting polypeptide, a prostaglandin E2 [PGE2] inhibiting polypeptide, and a cytotoxic anticancer polypeptide. The immunosuppression inhibiting polypeptide may be an antibody as far as the antibody has the foregoing function, and may be, for example, an IgG monoclonal antibody or an antibody fragment.

The immune checkpoint inhibiting polypeptide is typically a polypeptide that cancels or relaxes an immunosuppressive mechanism that works via an immune checkpoint molecule that is expressed on a surface of a T cell. The immune checkpoint inhibiting polypeptide can reduce a suppressive reaction on an immune response, for example, by binding to an immune checkpoint molecule (for example, PD-1, CTLA-4, BTLA, TIM-3, TIGIT, or LAG-3) or a ligand of an immune checkpoint molecule (for example, PD-L1, PD-L2, CD80/CD86, or Siglec-15), to suppress initiation of signal transduction from the immune checkpoint molecule triggered by the ligand.

Examples of the immune checkpoint inhibiting polypeptide include a PD-1 inhibiting polypeptide, a PD-L1 inhibiting polypeptide, a CTLA-4 inhibiting polypeptide, a CD47 inhibiting polypeptide, a SIRP$\alpha$ inhibiting polypeptide, a BTLA inhibiting polypeptide, a TIM-3 inhibiting polypeptide, a TIGIT inhibiting polypeptide, a LAG-3 inhibiting polypeptide, a Siglec-15 inhibiting polypeptide, and a galectin-9 inhibiting polypeptide. Examples of the CCR4 inhibiting polypeptide include an anti-CCR4 antibody (for example, Mogamulizumab).

The immunosuppression inhibiting polypeptide may include at least one selected from the group consisting of a PD-1inhibiting polypeptide, a PD-L1 inhibiting polypeptide, a PD-L2 inhibiting polypeptide, a CTLA-4 inhibiting polypeptide, a BTLA (B- and T-lymphocyte attenuator) inhibiting polypeptide, a TIM-3 (T-cell immunoglobulin and mucin domain 3) inhibiting polypeptide, a TIGIT (T-cell immunoreceptor with Ig and ITIM domains) inhibiting polypeptide, a LAG-3 (Lymphocyte Activation Gene-3) inhibiting polypeptide, and a Siglec-15 inhibiting polypeptide.

The immunosuppression inhibiting polypeptide may be an immune checkpoint inhibiting polypeptide, more specifically a PD-1 inhibiting polypeptide or a PD-L1 inhibiting polypeptide, and still more specifically an anti-PD-1 antibody or an anti-PD-L1 antibody. Examples of the anti-PD-1 antibody include Nivolumab, Pembrolizumab, Toripalimab, Cemiplimab-rwlc, and Sintilima. Examples of the anti-PD-L1 antibody include Atezolizumab, Durvalumab, and Avelumab. Examples of an anti-CTLA-4 antibody iclude Ipilimumab. Further examples include an antibody to CD47 and an antibody to SIRP$\alpha$.

The antibody may be an IgG monoclonal antibody, a Fab fragment, a scFv, or another antibody or antibody fragment as long as the antibody or antibody fragment has a prescribed antigen binding property. An example of an anti-PD-1 scFv as an immunosuppression inhibiting polypeptide is an amino acid sequence stretching from 592nd position to 835th position counted from the N-terminal in the SEQ ID NO: 16, and an example of a nucleic acid sequence encoding the amino acid sequence is a nucleic acid sequence stretching from 1717th residue to 2505th residue counted from the 5'-terminal in the SEQ ID NO: 15.

The single chain antibody (scFv) against a target of interest can be prepared using known methods. For example, a lymphoid tissue may be collected after inoculating an antigen into a mouse or the like, a library of antibody genes may be prepared therefrom, a base sequence encoding an antibody that recognizes a cancer antigen may be obtained by antibody direct cloning, and a single chain antibody may be designed based on the base sequence. Alternatively, hybridomas may be prepared using the collected lymphoid tissue, a hybridoma encoding an antibody that recognizes a cancer antigen may be identified to obtain a monoclonal antibody, and a single chain antibody may be designed based on the sequence information of the monoclonal antibody. Alternatively, a library of single chain antibodies may be prepared based on, for example, a naive antibody library prepared from B cells of a normal person or an antibody library prepared from B cells of a cancer-suffering person having an antiserum exhibiting high neutralization activity against a cancer antigen, the library of single chain antibodies may be displayed by phage display, and a single chain antibody that recognizes a cancer antigen may be selected therefrom.

The immunoresponsive cell C according to the present disclosure expresses IL-7, CCL19, an immunosuppression inhibiting polypeptide, and a cell surface molecule that specifically recognizes a cancer antigen. Here, the expression, "expresses IL-7, CCL19, an immunosuppression inhibiting polypeptide, and a cell surface molecule that specifically recognizes a cancer antigen", refers to a situation in which IL-7, CCL19, an immunosuppression inhibiting polypeptide, and a cell surface molecule that specifically recognizes a cancer antigen are produced by the immunoresponsive cell, so that at least some of cell surface molecules that specifically recognize a cancer antigen are located on a cell surface (a cell surface at the outer side of the cell), and so that IL-7, CCL19, and the immunosuppression inhibiting polypeptide are secreted to the outside of the cell.

Since cancer cells have an immunosuppressive mechanism that suppresses immunoresponsive cells' actions to attack the cancer cells or to send instructions for attacking the cancer cells, attack by the cancer-suffering person's own immune system against the cancer cells is suppressed. It is conceivable that the immunosuppression inhibiting polypeptide expressed by the immunoresponsive cell C according to the present disclosure facilitates attack by the immune system of a cancer-suffering person against cancer cells, by means of suppressing the immunosuppressive mechanism exerted by the cancer cells.

In addition, it is conceivable that the immunoresponsive cell C according to the present disclosure also expresses IL-7 and CCL19, not only the immunoresponsive cell C according to the present disclosure, but also endogenous immunoresponsive cells in the cancer-suffering person accumulate around cancer cells, thereby enabling more efficient attack against the cancer cells.

It is conceivable that the reason why the immunoresponsive cell C according to the present disclosure exerts a significantly improved therapeutic effect against cancer is that the immunoresponsive cell C according to the present disclosure produces a synergistic effect exerted by the combination of factors of secreted IL-7, CCL19, and the immunosuppression inhibiting polypeptide, and the immunoresponsive cell expressing the cell surface molecule that specifically recognizes a cancer antigen, by means of expressing Interleukin-7, CCL19, the immunosuppression inhibiting polypeptide, and the cell surface molecule that specifically recognizes a cancer antigen, This synergistic effect is superior to a degree that cannot be predicted from the individual effects of the respective factors.

For example, even in the case of treatment of a cancer that is difficult to treat with an immunoresponsive cell expressing interleukin-7, CCL19, and a cell surface molecule that specifically recognizes a cancer antigen, but not expressing an immunosuppression inhibiting polypeptide, or with an immunoresponsive cell expressing an immunosuppression inhibiting polypeptide and a cell surface molecule that specifically recognizes a cancer antigen, but not expressing interleukin-7 or CCL19, such a cancer can be treated with the immunoresponsive cell C according to the present disclosure. Further, such a high therapeutic effect makes it possible to obtain a therapeutic effect even with a reduced cell dose, which makes it possible to obtain a therapeutic effect even in a case in which a sufficient number of immunoresponsive cells for making use of autologous cells cannot be collected. These effects are not expected from co-expression of interleukin-7, CCL19, and a cell surface molecule that specifically recognizes a cancer antigen or from co-expression of an immunosuppression inhibiting polypeptide and a cell surface molecule that specifically recognizes a cancer antigen.

The immunoresponsive cell C according to the present disclosure can be obtained by introducing a nucleic acid encoding a cell surface molecule that specifically recognizes a cancer antigen, a nucleic acid encoding IL-7, a nucleic acid encoding CCL19, and a nucleic acid encoding an immunosuppression inhibiting polypeptide into, for example, an immunoresponsive cell collected from a living body or an immunoresponsive cell induced from a pluripotent stem cell such as an iPS cell or an ES cell, or induced from a somatic stem cell such as a hematopoietic stem cell. The immunoresponsive cell C according to the present disclosure can alternatively be obtained by collecting an immunoresponsive cell that inherently expresses a cell surface molecule that specifically recognizes a cancer antigen (for example a TCR that specifically recognizes a cancer antigen) from a living body, and introducing a nucleic acid encoding IL-7, a nucleic acid encoding CCL19, and a nucleic acid encoding an immunosuppression inhibiting polypeptide.

In the case of introduction of a nucleic acid into an immunoresponsive cell collected from a living body, rejection can be minimized by collecting an immunoresponsive cell of a cancer-suffering person to be treated with a drug including the immunoresponsive cell C according to the present disclosure (i.e., an autologous immunoresponsive cell). However, use of an allogenic immunoresponsive cell is not excluded. In other words, the immunoresponsive cell C according to the present disclosure may or may not be an immunoresponsive cell derived from the subject itself.

The nucleic acid encoding a cell surface molecule that specifically recognizes a cancer antigen, the nucleic acid encoding IL-7, the nucleic acid encoding CCL19, and the nucleic acid encoding an immunosuppression inhibiting polypeptide each may be present in the genome of the immunoresponsive cell C according to the present disclosure or may be retained on a vector that is present outside the genome. For example, each nucleic acid may be allowed to be present in the genome from the viewpoint of the stability of nucleic acid retaining. The nucleic acid encoding a cell surface molecule that specifically recognizes a cancer antigen, the nucleic acid encoding IL-7, the nucleic acid encoding CCL19, and the nucleic acid encoding an immunosuppression inhibiting polypeptide may be present in a gathered arrangement (for example, linked) in the genome, or may be present in an ungathered manner (separately from one another), or may be present such that only some of these nucleic acids are present in a gathered arrangement (for example, linked) while the remainder of these nucleic acids is/are present in an ungathered manner (separately from one another). In a case in which the cell surface molecule that specifically recognizes a cancer antigen is a heterodimer, such as a TCR formed of an αβ-dimer or γδ-dimer, or a heteromultimer, nucleic acids encoding the respective molecules composing the heterodimer or heteromultimer may be present in a gathered arrangement in the genome, or may be present in an ungathered manner (separately from one another).

In one embodiment, at least one of the nucleic acid encoding IL-7, the nucleic acid encoding CCL19, or the nucleic acid encoding an immunosuppression inhibiting polypeptide is exogenous, and it is permissible for all of these nucleic acids to be exogenous. Each of the nucleic acid encoding IL-7, the nucleic acid encoding CCL19, and the nucleic acid encoding an immunosuppression inhibiting polypeptide may independently be integrated into the genome, or integrated into a vector. In an embodiment, all of the nucleic acid encoding IL-7, the nucleic acid encoding CCL19, and the nucleic acid encoding an immunosuppression inhibiting polypeptide are integrated into the genome of the immunoresponsive cell C according to the present disclosure, or integrated together or separately into one or more vectors present in the immunoresponsive cell C. In another embodiment, some of the nucleic acid encoding IL-7, the nucleic acid encoding CCL19, and the nucleic acid encoding an immunosuppression inhibiting polypeptide are integrated into the genome of the immunoresponsive cell C according to the present disclosure, and the remainder of these nucleic acids is/are integrated into one or more vectors present in the immunoresponsive cell C according to the present disclosure.

In and embodiment, the nucleic acid encoding an immunosuppression inhibiting polypeptide is integrated into the genome of the immunoresponsive cell, or integrated into a vector that is the same as or different from one of one or more vectors that are present in the immunoresponsive cell and that cooperatively include a nucleic acid encoding IL-7 and a nucleic acid encoding CCL19.

Whether or not the respective nucleic acids are present in the cell can easily be confirmed using a known technique such as PCR.

Information regarding the amino acid sequence of the immunosuppression inhibiting polypeptide and the base sequence encoding the immunosuppression inhibiting polypeptide can be obtained, as desired, by searching known publications and database such as NCBI (http://www.ncbi.nlm.nih.gov/guide/).

When the immunoresponsive cell inherently expresses a cell surface molecule that specifically recognizes a cancer antigen, for example, when a T cell expressing a TCR that specifically recognizes a given cancer antigen is to be isolated, it is not necessary to introduce a nucleic acid encoding a cell surface molecule that specifically recognizes a cancer antigen from the outside. In the other cases, one or more of the nucleic acid encoding a cell surface molecule that specifically recognizes a cancer antigen, the nucleic acid encoding IL-7, the nucleic acid encoding CCL19, or the nucleic acid encoding an immunosuppression inhibiting polypeptide are introduced from the outside.

A nucleic acid encoding a cell surface molecule that specifically recognizes a cancer antigen, a nucleic acid encoding IL-7, a nucleic acid encoding CCL19, and a nucleic acid encoding an immunosuppression inhibiting polypeptide for introduction into an immunoresponsive cell can be prepared based on the information of base sequences encoding the respective molecules, using known techniques such as a chemical synthesis method or an amplification method using PCR. Codons in the coding region may be modified so as to optimize the expression of the gene in the immunoresponsive cell to which a gene-containing nucleic acid is to be introduced.

Introduction of the nucleic acids to be introduced may be performed by incorporating the nucleic acids into one or more nucleic acid delivery vehicles. Examples of the nucleic acid delivery vehicles include the aforementioned viruses, liposomes, and nanoparticles. The liposomes and the nanoparticles may include a nucleic acid that is in a state of being contained in a vector. Thus, according to the present disclosure, one or more nucleic acid delivery vehicles that cooperatively include a nucleic acid encoding IL-7, a nucleic acid encoding CCL19, and a nucleic acid encoding an immunosuppression inhibiting polypeptide are also provided. In a case in which the immunoresponsive cell does not inherently express a cell surface molecule that specifically recognizes a cancer antigen, the nucleic acid delivery vehicle may further include a nucleic acid encoding a cell surface molecule that specifically recognizes a cancer antigen.

In the following explanation, a vector or vectors that include the nucleic acids are described. However, the same explanation will apply to the case of the nucleic acid delivery vehicle or nucleic acid delivery vehicles, unless contradiction arises. Alternatively the nucleic acid delivery vehicle may include a vector such as those described below.

The nucleic acids to be introduced may be introduced in a state in which the nucleic acids are retained on respectively different vectors, or in a state in which two or more of the nucleic acids are retained on the same vector. For example, in the case of introducing a nucleic acid encoding IL-7, a nucleic acid encoding CCL19, and a nucleic acid encoding an immunosuppression inhibiting polypeptide into an immunoresponsive cell, the nucleic acid encoding IL-7 and the nucleic acid encoding CCL19 may be introduced by being retained on separate vectors, or both nucleic acids may be introduced by being retained on the same vector. The nucleic acid encoding CCL19 and the nucleic acid encoding an immunosuppression inhibiting polypeptide may be introduced by being retained on separate vectors, or both nucleic acids may be introduced by being retained on the same vector. The nucleic acid encoding IL-7 and the nucleic acid encoding an immunosuppression inhibiting polypeptide may be introduced by being retained on separate vectors, or both nucleic acids may be introduced by being retained on the same vector. The following explanation for a case in which a nucleic acid encoding a cell surface molecule that specifically recognizes a cancer antigen is further introduced can be applied to a case in which a nucleic acid encoding IL-7, a nucleic acid encoding CCL19, and a nucleic acid encoding an immunosuppression inhibiting polypeptide are introduced (i.e., a case in which introduction of a nucleic acid encoding a cell surface molecule that specifically recognizes a cancer antigen is not necessary), except that the "nucleic acid encoding a cell surface molecule that specifically recognizes a cancer antigen" should be omitted.

When a nucleic acid encoding a cell surface molecule that specifically recognizes a cancer antigen, a nucleic acid encoding IL-7, a nucleic acid encoding CCL19, and a nucleic acid encoding an immunosuppression inhibiting polypeptide are to be introduced into an immunoresponsive cell,

(i) the introduction may be performed in a state in which the nucleic acid encoding a cell surface molecule that specifically recognizes a cancer antigen, the nucleic acid encoding IL-7, the nucleic acid encoding CCL19, and the nucleic acid encoding an immunosuppression inhibiting polypeptide are retained on respectively different vectors; or

(ii) the introduction may be performed in a state in which the nucleic acid encoding a cell surface molecule that specifically recognizes a cancer antigen and the nucleic acid encoding IL-7 are retained on the same vector, and in which the nucleic acid encoding CCL19 is retained on a different vector, and in which the nucleic acid encoding an immunosuppression inhibiting polypeptide is retained on a further different vector; or

(iii) the introduction may be performed in a state in which the nucleic acid encoding a cell surface molecule that specifically recognizes a cancer antigen and the nucleic acid encoding CCL19 are retained on the same vector, and in which the nucleic acid encoding IL-7 is retained on a different vector, and in which the nucleic acid encoding an immunosuppression inhibiting polypeptide is retained on a further different vector; or

(iv) the introduction may be performed in a state in which the nucleic acid encoding a cell surface molecule that specifically recognizes a cancer antigen and the nucleic acid encoding an immunosuppression inhibiting polypeptide are retained on the same vector, and in which the nucleic acid encoding IL-7 is retained on a different vector, and in which the nucleic acid encoding CCL19 is retained on a further different vector; or

(v) the introduction may be performed in a state in which the nucleic acid encoding IL-7 and the nucleic acid encoding CCL19 are retained on the same vector, and in which the nucleic acid encoding a cell surface molecule that specifically recognizes a cancer antigen is retained on a different vector, and in which the nucleic acid encoding an immunosuppression inhibiting polypeptide is retained on a further different vector; or

(vi) the introduction may be performed in a state in which the nucleic acid encoding IL-7 and the nucleic acid encoding an immunosuppression inhibiting polypeptide are retained on the same vector, and in which the nucleic acid encoding a cell surface molecule that specifically recognizes a cancer antigen is retained on a different vector, and in which the nucleic acid encoding CCL19 is retained on a further different vector; or

(vii) the introduction may be performed in a state in which the nucleic acid encoding CCL19 and the nucleic acid encoding an immunosuppression inhibiting polypeptide are retained on the same vector, and in which the nucleic acid encoding a cell surface molecule that specifically recognizes a cancer antigen is retained on a different vector, and in which the nucleic acid encoding IL-7 is retained on a further different vector; or

(viii) the introduction may be performed in a state in which the nucleic acid encoding a cell surface molecule that specifically recognizes a cancer antigen and the nucleic acid encoding IL-7 are retained on the same vector, and in which the nucleic acid encoding CCL19 and the nucleic acid encoding an immunosuppression inhibiting polypeptide are together retained on a different vector; or

(ix) the introduction may be performed in a state in which the nucleic acid encoding a cell surface molecule that specifically recognizes a cancer antigen and the nucleic acid encoding CCL19 are retained on the same vector, and in which the nucleic acid encoding IL-7 and the nucleic acid encoding an immunosuppression inhibiting polypeptide are together retained on a different vector; or

(x) the introduction may be performed in a state in which the nucleic acid encoding a cell surface molecule that specifically recognizes a cancer antigen and the nucleic acid encoding an immunosuppression inhibiting polypeptide are retained on the same vector, and in which the nucleic acid encoding IL-7 and the nucleic acid encoding CCL19 are together retained on a different vector; or

(xi) the introduction may be performed in a state in which the nucleic acid encoding a cell surface molecule that specifically recognizes a cancer antigen, the nucleic acid encoding IL-7, and the nucleic acid encoding CCL19 are retained on the same vector, and in which the nucleic acid encoding an immunosuppression inhibiting polypeptide is retained on a different vector;

(xii) the introduction may be performed in a state in which the nucleic acid encoding a cell surface molecule that specifically recognizes a cancer antigen, the nucleic acid encoding IL-7, and the nucleic acid encoding an immunosuppression inhibiting polypeptide are retained on the same vector, and in which the nucleic acid encoding CCL19 is retained on a different vector; or

(xiii) the introduction may be performed in a state in which the nucleic acid encoding a cell surface molecule that specifically recognizes a cancer antigen, the nucleic acid encoding CCL19, and the nucleic acid encoding an immunosuppression inhibiting polypeptide are retained on the same vector, and in which the nucleic acid encoding IL-7 is retained on a different vector; or

(xiv) the introduction may be performed in a state in which the nucleic acid encoding IL-7, the nucleic acid encoding CCL19, and the nucleic acid encoding an immunosuppression inhibiting polypeptide are retained on the same vector, and in which the nucleic acid encoding a cell surface molecule that specifically recognizes a cancer antigen is retained on a different vector; or

(xv) the introduction may be performed in a state in which the nucleic acid encoding a cell surface molecule that specifically recognizes a cancer antigen, the nucleic acid encoding IL-7, the nucleic acid encoding CCL19, and the nucleic acid encoding an immunosuppression inhibiting polypeptide are retained on the same vector.

The introduction may be performed in a state in which two or more nucleic acids are retained on the same vector, in consideration of introduction efficiency. In this case, the two or more nucleic acids will be present in a gathered arrangement in the immunoresponsive cell.

For example, the following vector or a group of vectors may be used.

(f) a vector containing a nucleic acid encoding a cell surface molecule that specifically recognizes a cancer antigen, a nucleic acid encoding IL-7, a nucleic acid encoding CCL19, and a nucleic acid encoding an immunosuppression inhibiting polypeptide (corresponding to the foregoing case (xv));

(g) a group of vectors consisting of the following vector (g-1) and vector (g-2) (corresponding to the foregoing case (xiv))

(g-1) a vector containing a nucleic acid encoding a cell surface molecule that specifically recognizes a cancer antigen,

(g-2) a vector containing a nucleic acid encoding IL-7, a nucleic acid encoding CCL19, and a nucleic acid encoding an immunosuppression inhibiting polypeptide.

Appropriate vectors can also be designed in other cases in a similar manner.

Such a group of vectors may be designed such that the nucleic acids are contained in a redundant manner. In other words, a particular nucleic acid among a nucleic acid encoding a cell surface molecule that specifically recognizes a cancer antigen, a nucleic acid encoding IL-7, a nucleic acid encoding CCL19, and a nucleic acid encoding an immunosuppression inhibiting polypeptide may be included in two or more vectors belonging to a vector group.

One or more other immune function regulating factors such as IL-1, IL-2, IL-3, IL-4, IL-5, IL-6, IL-8, IL-9, IL-10, IL-11, IL-12, IL-13, IL-14, IL-15, IL-16, IL-17, IL-18, IL-23, IL-27, IP-10, CCL1, CCL2, CCL3, CCL4, CCL5, CCL7, CCL8, CCL11, CCL13, CCL14, CCL15, CCL16, CCL17, CCL18, CCL20, CCL21, CCL22, CCL23, CCL24, CCL25, CCL26, CCL27, CCL28, CXCL1, CXCL2, CXCL3, CXCL4, CXCL4L1, CXCL5, CXCL6, CXCL7, CXCL8, CXCL9, CXCL10, CXCL11, CXCL12, CXCL13, CXCL14, CXCL16, CXCL17, CX3CL1, XCL1, XCL2, CCL3L1, CCL3L3, CCL4L1, CCL4L2, Flt3L, Interferon-gamma, MIP-1 alpha, GM-CSF, M-CSF, TGF-beta, or TNF-alpha may be further incorporated into any of the foregoing vectors, or into a vector other than the foregoing vectors, and may be introduced into the immunoresponsive cell.

The method used for introducing a nucleic acid-retaining vector into an immunoresponsive cell is not particularly limited, and examples thereof include known methods such as a virus infection method, a transposon method, a calcium phosphate method, a lipofection method, a microinjection method, and an electroporation method. A method involving introduction using a virus infection method, which is capable of introducing a foreign nucleic acid into the genome, may provide stability of nucleic acid retention.

An example of the virus infection method is a method including transfecting a packaging cell, such as a GP2-293 cell (manufactured by Takara Bio Inc.), a Plat-GP cell (manufactured by Cosmo Bio Co., Ltd.), a PG13 cell (ATCC CRL-10686), or PA317 cell (ATCC CRL-9078) with a vector and a packaging plasmid to generate a recombinant virus, and infecting an immunoresponsive cell with the recombinant virus. This may be carried out using a commercial kit such as Retrovirus packaging kit Eco (manufactured by Takara Bio Inc.). Use of a MSCV retrovirus expression system or the like enables introduction of a foreign nucleic acid into the genome.

The integration of the nucleic acid encoding IL-7, the nucleic acid encoding CCL19, the nucleic acid encoding an immunosuppression inhibiting polypeptide, and, if necessary, the nucleic acid encoding a cell surface molecule that specifically recognizes a cancer antigen, into the genome can also be performed using a known gene editing technique. Examples of known gene editing techniques include a technique using an endonuclease such as zinc finger nuclease, TALEN (transcription activator-like effector nuclease), or a CRISPR (clustered regulatory interspaced short palindromic repeat)-Cas system. Integration of a nucleic acid encoding another foreign protein, which is optionally introduced, into the genome can be performed using such methods.

In the case of integrating these nucleic acids (genes) into the genome of an immunoresponsive cell, the nucleic acids may be integrated, together with an upstream promotor or upstream promoters for regulating the genes, into a non-coding region or the like of the genome in an operable manner (i.e., to be capable of expression under control exerted by the promotor), or may be operably integrated without, a promoter, into the downstream of a promoter that is already present in the genome. Examples of the promoter that is already present in the genome include a promoter of TCRα or TCRβ.

When two or more nucleic acids from among a nucleic acid encoding a cell surface molecule that specifically recognizes a cancer antigen, a nucleic acid encoding IL-7, a nucleic acid encoding CCL19, a nucleic acid encoding an immunosuppression inhibiting polypeptide, and an optionally introduced nucleic acid encoding an additional foreign protein are present in the proximity of each other, those two or more nucleic acids may be expressed under a control exerted by a common promoter. In a case in which the nucleic acids are expressed under a control exerted by a common promoter, transcription and/or translation may be split by using a 2A peptide, an IRES peptide, or the like, to enable expression of individual polypeptides.

In a case in which a vector retaining two or more of a nucleic acid encoding a cell surface molecule that specifically recognizes a cancer antigen, a nucleic acid encoding IL-7, a nucleic acid encoding CCL19, or a nucleic acid encoding an immunosuppression inhibiting polypeptide are introduced into an immunoresponsive cell, the alignment order of the two or more nucleic acids in the vector is not particularly limited. For example, in the vector (f) containing a nucleic acid encoding a cell surface molecule that specifically recognizes a cancer antigen, a nucleic acid encoding IL-7, a nucleic acid encoding CCL19, and a nucleic acid encoding an immunosuppression inhibiting polypeptide, the alignment order of these four nucleic acids is not limited. Specifically, with respect to the order from the upstream (5'-terminal side) to the downstream (3'-terminal side), the order may be:

(i) one of a nucleic acid encoding a cell surface molecule that specifically recognizes a cancer antigen, a nucleic acid encoding IL-7, a nucleic acid encoding CCL19, or a nucleic acid encoding an immunosuppression inhibiting polypeptide, followed by
(ii) one of the remainder (three nucleotides) of the foregoing four nucleic acids, followed by
(iii) one of the remainder (two nucleotides) of the foregoing four nucleic acids, followed by
(iv) the last remaining nucleic acid of the four nucleic acids.

Similar to the above, with respect to a vector including a nucleic acid encoding IL-7, a nucleic acid encoding CCL19, and a nucleic acid encoding an immunosuppression inhibiting polypeptide, which may be used in a case in which the immunoresponsive cell inherently expresses a cell surface molecule that specifically recognizes a cancer antigen, the order of the nucleic acids may be:

(i) one of a nucleic acid encoding IL-7, a nucleic acid encoding CCL19, or a nucleic acid encoding an immunosuppression inhibiting polypeptide, followed by
(ii) one of the remainder (two nucleic acids) of the foregoing three nucleic acids, followed by
(iii) the last remaining nucleic acid of the three nucleic acids.

The nucleic acid encoding a cell surface molecule that specifically recognizes a cancer antigen, the nucleic acid encoding IL-7, the nucleic acid encoding CCL19, and the nucleic acid encoding an immunosuppression inhibiting polypeptide may be transcribed due to the action of respectively different promoters, or may be transcribed due to the action of one promoter, using internal ribozyme entry site (IRES) or a self-cleaving 2A peptide.

In a case in which plural nucleic acids are transcribed due to the action of one promoter, a base sequence stretching between the respective nucleic acids may include a freely-selected base sequence as long as expression of individual nucleic acids is possible. The base sequence stretching between the respective nucleic acids may include a base sequence encoding a self-cleaving peptide (2A peptide) or an IRES-encoding base sequence, or may include a base sequence encoding a 2A peptide. Efficient expression of respective nucleic acids is enabled by linking plural nucleic acids with such a base sequence. The base sequence stretching between the respective nucleic acids, which may include a base sequence encoding a self-cleaving peptide (2A peptide) or IRES, may be a base sequence stretching between a nucleic acid encoding IL-7 and a nucleic acid encoding CCL19, or a base sequence stretching between a nucleic acid encoding IL-7 and a nucleic acid encoding an immunosuppression inhibiting polypeptide, or a base sequence stretching between a nucleic acid encoding a cell surface molecule that specifically recognizes a cancer antigen and a nucleic acid encoding IL-7, or a base sequence stretching between a nucleic acid encoding a cell surface molecule that specifically recognizes a cancer antigen and a nucleic acid encoding CCL19, or a base sequence stretching between a nucleic acid encoding a cell surface molecule that specifically recognizes a cancer antigen and a nucleic acid encoding an immunosuppression inhibiting polypeptide, or a base sequence stretching between a nucleic acid encoding CCL19 and a nucleic acid encoding an immunosuppression inhibiting polypeptide, or a base sequence stretching between a nucleic acid encoding an alpha chain and a nucleic acid encoding a beta chain in an alpha-beta TCR, or a base sequence stretching between a nucleic acid encoding a gamma chain and a nucleic acid encoding a delta chain in a gamma-delta TCR. That is, these intergenic regions each may include a base sequence encoding a self-cleaving peptide (2A peptide) or IRES, if desired.

A 2A peptide is a self-cleaving peptide from a virus. The specifics thereof are as described above.

The vector used for introducing a nucleic acid into an immunoresponsive cell may be linear or circular, and may be a non-virus vector such as a plasmid, a virus vector, or a transposon vector. The vector used for introducing a nucleic acid into the immunoresponsive cell may include one or more of a regulatory sequence such as a promoter or a terminator, or a selection marker sequence such as a drug-resistant nucleic acid or a reporter nucleic acid. Expression of the nucleic acid may utilize a promoter contained in the vector even after the nucleic acid has been introduced into the immunoresponsive cell. For example, by operably disposing one or more of the nucleic acid encoding a cell surface molecule that specifically recognizes a cancer antigen, the nucleic acid encoding IL-7, the nucleic acid encoding CCL19, or the nucleic acid encoding an immunosuppression inhibiting polypeptide at the downstream of a promoter sequence in the vector, the nucleic acids can efficiently be transcribed.

Examples of the promoter include promoters from viruses such as a LTR promoter of a retrovirus, the SV40 early promoter, the cytomegalovirus promoter, and the thymidine kinase promoter of the herpes simplex virus, and promoters from mammals such as the phosphoglycerate kinase (PGK) promoter, the Xist promoter, the β-actin promoter, the RNA polymerase II promoter, and the polypeptide chain elongation factor gene promoter. It is also possible to use a tetracycline responsive promoter induced by tetracycline, a Mx1 promoter induced by interferon, or the like. By using a promoter that is induced by a specific substance, the expression of a gene subject to transcription regulation by the promoter (for example, one or more of a gene encoding a cell surface molecule that specifically recognizes a cancer antigen, a gene encoding IL-7, a gene encoding CCL19, or a gene encoding an immunosuppression inhibiting polypeptide) can be regulated in accordance with the course of treatment of the cancer.

Examples of the virus vector include a retrovirus vector, a lentivirus vector, an adenovirus vector, and an adeno-associated virus vector. Examples of the retrovirus vector include a pMSGV vector (Tamada K. et al., Clin Cancer Res 18:6436-6445 (2002)) and pMSCV vector (manufactured by Takara Bio Inc.). Use of a retrovirus vector enables the introduced nucleic acid to be stably expressed for a long time since the introduced nucleic acid is integrated into the genome of the host cell.

The expression of IL-7, CCL19, the immunosuppression inhibiting polypeptide, and the cell surface molecule that specifically recognizes a cancer antigen in the immunoresponsive cell can be determined, for example, by flow cytometry, ELISA, or Western Blotting. Introduction of nucleic acids encoding these molecules can be confirmed by checking for the expression products as described above, or by using, for example, Northern Blotting, Southern Blotting, or PCR such as RT-PCR. When the vector used for nucleic acid introduction includes a marker gene, the introduction of the nucleic acid can be confirmed by checking for the expression of the marker gene inserted in the expression vector.

<Drug Including Immunoresponsive Cell C according to the Present Disclosure>

According to the present disclosure, a drug including an immunoresponsive cell expressing Interleukin-7, CCL19, an immunosuppression inhibiting polypeptide, and a cell surface molecule that specifically recognizes a cancer antigen (hereinafter also referred to as "drug C according to the present disclosure") is provided. In other words, the drug C according to the present disclosure is a drug that includes the immunoresponsive cell C according to the present disclosure.

The drug C according to the present disclosure may further include a pharmaceutically acceptable additive, and examples of the additive include physiological saline, buffered saline, cell culture medium, dextrose, water for injection, glycerol, ethanol, and combinations thereof, a stabilizer, a solubilizer, a surfactant, a buffering agent, a preservative, an isotonization agent, a filler, and a lubricant.

The amount of the immunoresponsive cells C according to the present disclosure contained in the drug C according to the present disclosure may be adjusted, as appropriate, in accordance with, for example, the type, position, or severity of the cancer, or the age, body weight, or condition of the subject to be treated. The amount of the immunoresponsive cells C according to the present disclosure contained in the drug C according to the present disclosure is, for example, from $1\times10^4$ to $1\times10^{11}$ cells, more specifically from $1\times10^5$ to $1\times10^{10}$ cells, and still more specifically from $1\times10^6$ to $1\times10^9$ cells, per one administration. Further the amount of the immunoresponsive cells C according to the present disclosure may be small, such as $1\times10^6$ cells or less, for example, from $1\times10^5$ to $5\times10^5$ cells, and more specifically from $1.5\times10^5$ to $4\times10^5$ cells, per one administration.

As described above, even in the case of treatment of a cancer that is difficult to treat with an immunoresponsive cell expressing interleukin-7, CCL19, and a cell surface molecule that specifically recognizes a cancer antigen, but not expressing an immunosuppression inhibiting polypeptide, or with an immunoresponsive cell expressing an immunosuppression inhibiting polypeptide and a cell surface molecule that specifically recognizes a cancer antigen, but not expressing interleukin-7 or CCL19, such a cancer can be treated with the immunoresponsive cell C according to the present disclosure. Therefore, the amount of the immunoresponsive cells C according to the present disclosure contained in the drug C may be so small an amount at which an anticancer effect would not be exerted in the case of using that amount of immunoresponsive cells expressing interleukin-7, CCL19, and a cell surface molecule that specifically recognizes a cancer antigen, but not expressing an immunosuppression inhibiting polypeptide, or using that amount of immunoresponsive cells expressing an immunosuppression inhibiting polypeptide and a cell surface molecule that specifically recognizes a cancer antigen, but not expressing interleukin-7 or CCL19. The lower limit of the amount of the immunoresponsive cells C is not particularly limited as long as the immunoresponsive cells C in that amount is capable of exerting an anticancer effect.

The drug C according to the present disclosure may be administered at a frequency of four times daily, three times daily, twice daily, once daily, once per every other days, once in every third days, once in every fourth day, once in every fifth day, once in every sixth day, once weekly, once in every eighth day, once in every ninth day, once in every tenth day, twice weekly, once monthly, or twice monthly. Further, the number of administrations may be from 1 to 10 in total, specifically, 1, 2, 3, 4, 5, 6, 7, 8, 9, or 10 in total. Administration for more than 10 times in total is also permissible.

As described above, the drug C according to the present disclosure exerts a surprisingly improved therapeutic effect against cancer, due to a synergistic effect exerted by the combination of factors of secreted IL-7 and CCL19, the immunosuppression inhibiting polypeptide, and the immunoresponsive cell that expresses a cell surface molecule that specifically recognizes a cancer antigen.

The drug C according to the present disclosure can be administered to a subject in need of treatment of a cancer, using a method known to those skilled in the art, and may be administered, for example, by topical infusion or injection (including catheter infusion), systemic infusion or injection, intravenous infusion or injection, or parenteral administration (for example, transdermal administration or transmucosal administration, more specifically, nasal, ophthalmic, sublingual, by a suppository, by a patch, or the like). The drug C according to the present disclosure may be formulated into a form (solution, suspension liquid, or emulsion liquid) capable of infusion or injection of a unit dose, from the viewpoint of handleability. More specific examples of administration methods include intravenous injection, intratumor injection, intradermal injection, subcutaneous injection, intramuscular injection, intraperitoneal injection, intra-arterial injection, intramedullary injection, intracardiac injection, intra-articular injection, intrasynovial injection, intracranial injection, intrathecal injection, and subarchnoidal injection (injection to cerebrospinal fluid).

After an immunoresponsive cell or precursor cell thereof as a starting point is obtained from a patient, who is the subject of treatment, and requisite genes for converting the cell into the immunoresponsive cell C according to the present disclosure are introduced thereto, the immunoresponsive cell C according to the present disclosure may be introduced to the same patient (autologous administration) or to a different patient (allogenic administration). Alternatively, the immunoresponsive cell or precursor cell thereof as a starting point may be prepared from a pluripotent stem cell such as an iPS cell or an ES cell, or from somatic stem cell such as a hematopoietic stem cell.

The drug C according to the present disclosure may be administered, for example, in the form of a sterile liquid preparation, which may be buffered to a predetermined pH, such as an isotonic aqueous solution, a suspension liquid, an emulsion liquid, a dispersion liquid, or a viscous composition. The liquid preparation may be a liquid preparation for injection. The liquid preparation may be in the form of a viscous composition having a viscosity within an appropriate viscosity range so as to elongate the duration of contact with a specified tissue. The liquid preparation may include a solvent or a dispersion medium selected from, for example, water, physiological saline, phosphate-buffered saline, a polyol (for example, glycerol, propylene glycol, or liquid polyethylene glycol), or a combination thereof.

The liquid preparation may be prepared by adding the immunoresponsive cell C according to the present disclosure to an appropriate amount of an appropriate solvent, together with various amounts of other components. The liquid preparation may include a suitable carrier, diluent, or excipient. The liquid preparation may be lyophilized. The liquid preparation may further include various auxiliary agents, depending on the desired administration route, and examples of the auxiliary agents include a moisturizing agent, a dispersant or emulsifier (for example, methyl cellulose), a pH buffering agent, a gelling agent or viscosity enhancing agent, a preservative, a flavoring agent, and a coloring agent. In regard to components that may be included in the liquid preparation, "REMINGTON'S PHARMACEUTICAL SCIENCE", 17th edition (1985) may be referenced.

The liquid preparation may further include various additives that enhance the stability and sterility of the liquid preparation, examples of which include antimicrobial preservatives, antioxidants, chelating agents, and buffering agents. Various antibacterial agents and antifungal agents, such as parabens, chlorobutanol, phenol, and sorbic acid, may be used in order to prevent actions of microorganisms. Vehicles, diluents, and additives for use in the liquid preparation should have compatibility with the immunoresponsive cell C according to the present disclosure, which is included in the liquid preparation.

The liquid preparation may be isotonic with blood. The isotonicity can be achieved by allowing the liquid preparation to include sodium chloride or another pharmaceutically acceptable osmolarity regulating substance (for example, dextrose, boric acid, sodium tartrate, propylene glycol, or another inorganic or organic solute).

The drug C according to the present disclosure may include another anticancer agent, in addition to the immunoresponsive cell C according to the present disclosure. Examples of the other anticancer agent include alkylating agents such as bendamustin, ifosfamide, and dacarbazine, antimetabolites such as pentostatin, fludarabin, cladribin, methotrexate, 5-fluorouracil, 6-mercaptopurine, and enocitabine, molecular target drugs such as rituximab, cetuximab, and trastuzumab, kinase inhibitors such as imatinib, gefitinib, erlotinib, afatinib, dasatinib, sunitinib, and trametinib, proteasome inhibitors such as bortezomib, calcineurin inhibitors such as cyclosporine and tacrolimus, anticancer antibiotics such as idarubicin, doxorubicin, and mitomycin C, plant alkaloids such as irinotecan and etoposide, platinum-containing drugs such as cisplatin, oxaliplatin, and carboplatin, hormone therapy agents such as tamoxifen and bicalutamide, and immune control agents such as interferon. The other anticancer agent may include at least one of an alkylating agent or an antimetabolite.

<Treatment of Cancer Using Drug C according to Present Disclosure>

In the case of using the drug C according to the present disclosure in treatment of a cancer, the subject to be treated may be, for example, any mammalian animal. The subject to be treated is, for example, a primate animal, and can more specifically be a human. The subject to be treated may alternatively be a pet animal or a farm animal, examples of which include a dog, a cat, a pig, cattle, a horse, a sheep, and a goat.

The cancer to be treated may be either a solid cancer or a blood cancer, and examples thereof include: cancers such as adenocarcinoma, squamous cell carcinoma, adenosquamous carcinoma, undifferentiated carcinoma, large cell carcinoma, small cell carcinoma, skin cancer, breast cancer, prostate cancer, bladder cancer, vaginal cancer, cervical cancer, uterine cancer, liver cancer, renal cancer, pancreatic cancer, spleen cancer, lung cancer, tracheal cancer, bronchial carcinoma, colon cancer, small intestine cancer, gastric cancer, esophageal cancer, gallbladder cancer, testicular cancer, and ovarian cancer; cancers of a bone tissue, a cartilage tissue, an adipose tissue, a muscle tissue, a vascular tissue, and a hematopoietic tissue; sarcomas such as chondrosarcoma, Ewing’s sarcoma, malignant vascular endothelial sarcoma, malignant schwannoma, osteosarcoma, and soft tissue sarcoma; blastomas such as hepatoblastoma, medulloblastoma, nephroblastoma, neuroblastoma, pancreatoblastoma, pleuropulmonary blastoma, and retinoblastoma; germ cell tumor; lymphoma; and leukemia.

Since the drug C according to the present disclosure is capable of reducing immunosuppression in a cancer microenvironment, the cancer to be treated is not limited to a hemocyte cancer, and the drug C also exerts a therapeutic effect against a solid cancer. Therefore, the drug C exerts a high efficacy even against a solid cancer, which was difficult to treat by conventional methods.

In a situation in which the presence of cancer cells in a subject is suspected, the drug C according to the present disclosure may be prophylactically administered to the subject before a definitive diagnosis of cancer is made. In the present disclosure, such a mode of use is also included in the concept of use in treatment of a cancer.

<Method of Treating Cancer in Subject>

According to an aspect of the present disclosure, a method of treating a cancer in a subject (hereinafter also referred to as the "cancer treatment method C according to the present disclosure") is provided, the method including administering, to a subject, (a) an immunoresponsive cell expressing interleukin-7, CCL19, an immunosuppression inhibiting polypeptide, and a cell surface molecule that specifically recognizes a cancer antigen.

Here, the immunoresponsive cell in the cancer treatment method C according to the present disclosure is the immunoresponsive cell C according to the present disclosure, and the above-described explanation regarding the immunoresponsive cell C according to the present disclosure applies, as it is, to detailed configuration and examples of the immunoresponsive cell in the cancer treatment method C according to the present disclosure.

In addition, the explanation regarding the drug C according to the present disclosure applies, as it is, to the specifics of the treatment method such as the subject, cancer type, dose, and administration schedule in the cancer treatment method C according to the present disclosure. The immunoresponsive cell C according to the present disclosure may be administered in a therapeutically effective amount.

Due to a synergistic effect exerted by the combination of factors of IL-7, CCL19, an immunosuppression inhibiting polypeptide, and a cell surface molecule that specifically recognizes a cancer antigen, which are expressed by the immunoresponsive cell, the cancer treatment method C according to the present disclosure produces a surprisingly improved therapeutic effect against cancer.

Further, according to the present disclosure, use of an immunoresponsive cell expressing interleukin-7, CCL19, an immunosuppression inhibiting polypeptide, and a cell surface molecule that specifically recognizes a cancer antigen in the manufacture of a drug for treating a cancer is provided. Also in this use, the explanation regarding the combination drug C according to the present disclosure applies, as it is, to the specifics of the immunoresponsive cell, the cancer treatment, and the like.

Further, according to the present disclosure, an immunoresponsive cell expressing interleukin-7, CCL19, an immunosuppression inhibiting polypeptide, and a cell surface molecule that specifically recognizes a cancer antigen for use in treatment of a cancer in a subject is provided.

The drug C according to the present disclosure may be contained in a container, such as those described in the explanation regarding the combination drug A according to the present disclosure. A product including a container containing the drug C is also provided.

As described above, according to one aspect of the present disclosure, a surprisingly improved cancer therapeutic effect can be obtained, owing to a synergistic effect exerted by the combination of IL-7, CCL19, an immunosuppression inhibiting polypeptide, and a cell surface molecule that specifically recognizes a cancer antigen, which are expressed by the immunoresponsive cell.

EXAMPLES

Embodiments are more specifically described below, based on examples. However, these examples do not limit the present disclosure in any way. In the following, % and ppm are both based on mass, unless otherwise specified.

[Preparation of IL-7 × CCL19 Expression Vector]

An IL-7-F2A-CCL19 DNA fragment (SEQ ID NO: 9), which encodes mouse IL-7 (without a stop codon) followed by F2A and mouse CCL19, was artificially synthesized. In order to prepare a vector that expresses IL-7, CCL19, and eGFP, the synthesized IL-7-F2A-CCL19 DNA fragment was inserted into a MCS of a pMSGV retrovirus expression vector having a F2A-eGFP sequence (Tamada k et al., Clin Cancer Res 18: 6436-6445 (2002)) through treatment with restriction enzymes (NcoI and EcoRI) and ligation, to obtain a pMSGV vector (7×19 expression vector), which includes a IL-7-F2A-CCL19-F2A-eGFP DNA fragment (SEQ ID NO: 10). The map of the vector obtained is shown in Fig. 1B. In addition, a pMSGV vector (eGFP-Conv. vector) as a control was prepared, which includes eGFP but includes neither IL-7 nor CCL19. The map of the eGFP-Conv. vector is shown in Fig. 1A. In SEQ ID NO: 10, bases 1 to 462 correspond to IL -7 (bases 1 to 75 correspond to a signal sequence of IL -7), bases 463 to 537 correspond to F2A, bases 538 to 861 correspond to CCL 19 (bases 538 to 612 correspond to a signal sequence of CCL 19), bases 868 to 942 correspond to F2A, bases 946 to 1662 correspond to an eGFP-encoding nucleic acid, and bases 1663 to 1665 correspond to a stop codon. The amino acid sequence corresponding to the base sequence of SEQ ID NO: 10 is shown as SEQ ID NO: 11. In order to adopt to use of restriction enzyme NcoI, the fourth base in SEQ ID NO: 10 was changed from thymine (t) to guanine (g) (resulting in a change of the second amino acid in SEQ ID NO: 11 from phenylalanine (F) to valine (V)).

A retrovirus was prepared for the transduction of mouse T cells. Using LIPOFECTAMINE 3000 (manufactured by Thermo Fisher Scientific K.K.), GP2-293 packaging cells (manufactured by Takara Bio Inc.) were transfected with the 7×19 expression vector or eGFP-Conv. vector and a pCL-Eco plasmid (manufactured by Imgenex), to prepare a retrovirus to which the 7×19 expression vector or eGFP-Conv. vector had been introduced.

The culture liquid used for the GP2-293 cells was DMEM supplemented with 10% FCS, 100 U/ml penicillin, and 100 μg/ml streptomycin. The culture liquid used for T cells in the later-described examples was RPMI-1640 supplemented with 10% FCS, 100 U/ml penicillin, 100 μg/ml streptomycin, and 50 mM 2-mercaptoethanol.

[Preparation of T cells Expressing IL-7, CCL19, eGFP, and TCR That Specifically Recognizes P815 Tumor Antigen P1A]

Male and female DBA/2 mice (at an age of 6 to 8 weeks) were purchased from Japan SLC, Inc. (Shizuoka, Japan) and used for experiments. Transgenic mice expressing a TCR that specifically recognizes a H-2$L^d$-restricted P815 tumor antigen P1A (Sarma, S., Y. Guo, Y. Guilloux, C. Lee, X.-F. Bai, Y. Liu. 1999. J. Exp. Med. 189: 811-820) were backcrossed with DBA/2 mice for at least 10 generations. All mice were maintained in pathogen-free conditions.

Splenocytes were collected from a transgenic mouse after backcrossing that expressed a TCR capable of specifically recognizing the P1A, and mouse T cells expressing the P815 tumor antigen P1A-specific TCR and obtained from the splenocytes (P1A-specific TCR-T cells; hereinafter also referred to as "vector unintroduced P1A-TCRT cells") were obtained. Mouse T cells expressing P1A-specific TCR will be collectively referred to as "P1A-TCRT cells", regardless of whether a vector has been introduced or not. In order to perform transduction, vector unintroduced P1A-TCRT cells were activated by being incubated, for 48 hours, in the presence of P1A peptide (LPYLGWLVF; SEQ ID NO: 12) and IL-2 in amounts suitable for cell activation. After 48 hours of incubation, the cultured cells were collected, and vector unintroduced P1A-TCRT cells were enriched by performing negative magnetic sorting using a mouse Pan T cell Isolation Kit (manufactured by Miltenyi Biotec, Bergisch Gladbach, Germany). Isolated vector unintroduced P1A-TCRT cells were transferred to plates that were coated with 25 μg/ml of RETRONECTIN (registered trademark, manufactured by Takara Bio Inc.). The supernatant prepared above, which contained a retrovirus transduced with the 7×19 expression vector or eGFP-Conv. vector, was mixed with the vector unintroduced P1A-TCRT cells (1×$10^6$ cells/ml), which had been activated on the plate, and the cells were centrifuged at 1500 rpm for 2 hours, and then cultured for 6 hours. In order to remove the retrovirus from the culture liquid, the T cells were collected, washed, transferred to a fresh growth culture liquid medium (RPMI-1640) containing IL-2, and cultured for another 2 days to prepare a population of P1A-TCRT cells that included P1A-TCRT cells transduced with the 7×19 expression vector (hereinafter also referred to as "eGFP-expressing P1A-7×19 TCRT cells"), or a population of P1A-TCRT cells that included P1A-TCRT cells transduced with the eGFP-Conv. vector (hereinafter also referred to as "eGFP-expressing P1A-TCRT cells"). The transduction with the respective expression vectors was confirmed by flow cytometry analysis, which detected eGFP as a surrogate marker. As described above, eGFP-expressing P1A-7×19 TCRT cells and eGFP-expressing P1A-TCRT cells accounted for only a part of their respective P1A-TCRT cell populations. Nevertheless, for the sake of simplicity of description, treatment on the corresponding cell population is described as treatment on the eGFP-expressing P1A-7×19 TCRT cells or on the eGFP-expressing P1A-TCRT cells in the present specification, unless otherwise specified.

[Confirmation of Transduction]

(Flow Cytometry Analysis)

The expression levels of eGFP and CD8 were checked by dual-color flow cytometry analysis. Vector unintroduced P1A-TCRT cells, which did not undergo transduction with a retrovirus, eGFP-expressing P1A-7×19 TCRT cells, and eGFP-expressing P1A-TCRT cells were cultured in the presence of allophycocyanin (APC)-conjugated anti-CD8 monoclonal antibody (53-6.7, manufactured by Affymetrix). Flow cytometry was performed using a EC800 (manufactured by Sony Corporation) or a BD LSRForetessa X-20 (manufactured by BD Biosciences), and data analysis was performed using FlowJo software (manufactured by Tree Star).

The results are shown as a scatter plot in Fig. 2. In Figs. 2, 3A and 3B, "Transduction (-)" represents P1A-TCRT cells that did not undergo transduction (vector unintroduced P1A-TCRT cells), "Conv. P1A-T cells" represents eGFP-expressing P1A-TCRT cells, and "7×19 P1A-T cells " represents eGFP-expressing P1A-7×19 TCRT cells. The percentage values in Fig. 2 each indicate a proportion of the number of cells present in each region.

As shown in Fig. 2, in the cases of the eGFP-expressing P1A-7×19 TCRT cells and the eGFP-expressing P1A-TCRT cells, it was observed that about 70% to 80% of T cells expressed eGFP, demonstrating that the 7×19 expression vector or the eGFP-Conv. vector was successfully introduced.

(Analysis by ELISA)

The conditioned medium obtained after the eGFP-expressing P1A-7×19 TCRT cells or eGFP-expressing P1A-TCRT cells were cultured for 2 days was collected, and the concentrations of IL-7 and CCL19 in the conditioned medium were measured using a commercially available ELISA kit (manufactured by R&D systems). The results are shown in Figs. 3A and 3B. Together with the average value over the triplicate wells, the standard deviation is also shown in the graph. The "N.D." in the graph indicates that the presence was not detected, and "***" indicates $P < 0.001$. As shown in Figs. 3A and 3B, the expression of IL-7 and the expression of CCL19 were confirmed in the case of the eGFP-expressing P1A-7×19 TCRT cells.

(Analysis of Anticancer Effect Produced by Administration to Tumor-Bearing Mice)

On day 0, $5\times10^5$ P815 mastocytoma cells that were suspended in 0.1 ml of HBSS were subcutaneously inoculated into the flanks of male and female DBA/2 mice at an age of 6 to 8 weeks. The P815 mastocytoma cells were syngeneic to DBA/2 mice, and hereinafter also simply referred to as "P815 cells" or "P815 tumor cells". On day 6, the mice were subjected to sublethal irradiation (3 to 5 Gy) for preconditioning. On day 7, the mice were divided into six groups. Group 1 and Group 2 received intravenous administration of $1\times10^6$ eGFP-expressing P1A-7×19 TCRT cells, Group 3 and Group 4 received intravenous administration of $1\times10^6$ eGFP-expressing P1A-TCRT cells, and Group 5 and Group 6 did not receive administration of T cells (as described above, the number of cells indicated is the total number of T cells in the P1A-TCRT cell population, which included the eGFP-expressing P1A-7×19 TCRT cells or the eGFP-expressing P1A-TCRT cells). The mice in Group 2, Group 4, and Group 6 were further injected intraperitoneally with an anti-PD-1 monoclonal antibody (manufactured by Merck, clone G4; the same applies to the anti-PD-1 monoclonal antibodies described below) at a dose of 100 μg/individual and a frequency of once a week for a total of six times, starting on day 10. The survival rates of the respective mice were analyzed, and the tumor volume of each mouse were measured twice a week. The tumor volume was measured with a digital calliper and obtained according to the following expression:

$$\text{Tumor volume} = 1/2 \times (\text{length of long axis of tumor}) \times (\text{length of short axis of tumor})^2$$

The results of the analysis of the survival rates of the respective mice are shown in Fig. 4, and the results of the measurement of the tumor volume are shown in Figs. 5A to 5F. The data indicated was obtained by pooling the results of five independent experiments.

In Figs. 4 and 5A to 5F, □ indicates mice that did not receive administration (sample size = 15 mice; the foregoing Group 5), ■ indicates mice that received administration of the anti-PD-1 monoclonal antibody alone (sample size = 11 mice; the foregoing Group 6), Δ indicates mice that received administration of the eGFP-expressing P1A-TCRT cells (sample size = 17 mice; the foregoing Group 3), ▲ indicates mice that received administration of the eGFP-expressing P1A-TCRT cells and the anti-PD-1 monoclonal antibody (sample size = 14 mice; the foregoing Group 4), ◦ indicates mice that received administration of the eGFP-expressing P1A-7×19 TCRT cells (sample size = 24 mice; the foregoing Group 1), and ● indicates mice that received administration of the eGFP-expressing P1A-7×19 TCRT cells and the anti-PD-1 monoclonal antibody (sample size = 19 mice; the foregoing Group 2). In Figs. 4 and 5A to 5F, the horizontal axis represents the number of days that elapsed since the subcutaneous inoculation with P815 mastocytoma cells. The number of tumor-rejecting mice (numerator) relative to the total number of mice in each group (denominator) is also shown in Figs. 5A to 5F. The P values in the log-rank test were P = 0.0005 between Δ and ◦ groups, P = 0.0002 between ▲ and ● groups, P = 0.7284 between Δ and ▲ groups, and P = 0.0253 between ◦ and ● groups.

The results shown in Figs. 4 and 5A to 5F demonstrate that the mice that received administration of the eGFP-expressing P1A-7×19 TCRT cells and the anti-PD-1 monoclonal antibody (●) achieved an increase in the survival rate and suppression of increase in the tumor volume of the solid tumor, at a level that could not be achieved in the mice (◦) that received administration of the eGFP-expressing P1A-7×19 TCRT cells or the mice (A) that received administration of the eGFP-expressing P1A-TCRT cells and the anti-PD-1 monoclonal antibody. In contrast, the mice that received administration of the eGFP-expressing P1A-TCRT cells and the anti-PD-1 monoclonal antibody (A) did not exhibit a high synergistic effect.

(Preparation of PD-1-disrupted or ROSA26-disrupted P1A-TCRT)

For gene editing, similarly to the above, vector unintroduced P1A-TCRT cells were activated by being incubated for 48 hours in the presence of P1A peptide (LPYLGWLVF; SEQ ID NO: 12) and IL-2 in amounts appropriate for cell activation. After 48 hours of incubation, the cultured cells were collected, and the vector unintroduced P1A-TCRT cells were enriched by negative magnetic sorting using a mouse Pan T cell Isolation Kit (Miltenyi Biotec, Bergisch Gladbach, Germany). The T cells obtained were washed with PBS, resuspended in Buffer R (manufactured by Thermo Fisher Scientific K.K.), and mixed with a Cas9-RNP complex, which included a TRUECUT (registered trademark) Cas9 protein v2 (manufactured by Thermo Fisher Scientific K.K.) and a gene-specific guide RNA. The Cas9-RNP complex was introduced into the T cells by electroporation, using a Neon Transfection System (manufactured by Invitrogen). This resulted in the disruption of the target gene. Furthermore, immediately after electroporation, the T cells were resuspended in IL-2-containing cRPMI, and the 7×19 expression vector was packaged into a retrovirus and introduced into the T cells in the same manner as that described above. The guide RNA targeted to mouse PD-1 was designed with reference to Okada M, et al. Blockage of Core Fucosylation Reduces Cell-Surface Expression of PD-1 and Promotes Anti-tumor Immune Responses of T Cells. Cell Rep. 2017; 20(5): 1017-1028, and the sequence of the guide RNA portion was 5'-UCUGGGCAUGUGGGUCCGGC-3' (SEQ ID NO: 13). The synthesis of the guide RNA was outsourced to Thermo Fisher Scientific K.K. As a control, the guide RNA was replaced by 5'-CUCCAGUCUUUCUAGAAGAU-3' (SEQ ID NO: 14) targeted to ROSA26, to disrupt mouse ROSA26. The guide RNA targeted to ROSA26 was purchased from Thermo Fisher Scientific K.K.

(Administration Experiment of PD-1-disrupted or ROSA26-disrupted P1A-TCRT)

On day 0, $5\times10^5$ P815 mastocytoma cells suspended in 0.1 ml of HBSS were subcutaneously inoculated into the flanks of male and female DBA/2 mice at an age of 6 to 8 weeks. On day 6, the mice were subjected to sublethal irradiation (3 to 5 Gy) for preconditioning. The mice were divided into four groups, which respectively received the following treatments on day 7 and subsequent days.

Group 1: Neither T cells nor an antibody was administered.

Group 2: On day 7, $1\times10^6$ eGFP-expressing P1A-7×19 TCRT cells of which ROSA 26 had been knocked down were administered intravenously.

Group 3: On day 7, $1\times10^6$ cells of a cell population including eGFP-expressing P1A-7×19 TCRT cells of which PD-1 had been knocked down were administered intravenously.

Group 4: On day 7, $1\times10^6$ cells of a cell population including eGFP-expressing P1A-7×19 TCRT cells of which PD-1 had been knocked down were administered intravenously, and the anti-PD-1 monoclonal antibody was injected intraperitoneally at a dose of 100 μg/individual and a frequency of once a week for a total of six times starting on day 10.

As described above, the number of cells indicated is the total number of T cells in the P1A-TCRT cell population, which included the eGFP-expressing P1A-7×19 TCRT cells.

Thereafter, the survival rates of the respective mice were analyzed, and the tumor volume of each mouse was measured twice a week according to the foregoing technique using a digital calliper. The results of the analysis of the survival rates of the respective mice are shown in Fig. 6A, and the results of the measurement of the tumor volume are shown in Figs. 6B to 6E. The sample size was N = 6 for all groups.

In Figs. 6A to 6E, × indicates mice that did not receive administration (the foregoing Group 1), □ indicates mice that received administration of the eGFP-expressing P1A-7×19 TCRT cells of which the ROSA26 region had been knocked down (the foregoing Group 2), ◊ indicates mice that received administration of the eGFP-expressing P1A-7×19 TCRT cells of which PD-1 had been knocked down (the foregoing Group 3), ♦ indicates mice that received administration of the anti-PD-1 monoclonal antibody in addition to the eGFP-expressing P1A-7×19 TCRT cells of which PD-1 had been knocked down (the foregoing Group 4).

In Figs. 6A to 6E, the horizontal axis represents the number of days that elapsed since the subcutaneous inoculation with P815 mastocytoma cells. The number of tumor-rejecting mice (numerator) relative to the total number of mice in each group (denominator) is also shown in Figs. 6B to 6E. The P-values in the log-rank test were P = 0.0454 between × and ◦ groups, P = 0.0431 between □ and ◊ groups, and P = 0.0402 between ◊ and ♦ groups.

The results shown in Figs. 6A to 6E demonstrate that the therapeutic effect against cancer that can be obtained in the mice that received administration of the eGFP-expressing P1A-7×19 TCRT cells and the anti-PD-1 monoclonal antibody is much higher than that obtained in the case of simply knocking down PD-1 in the eGFP-expressing P1A-7×19 TCRT cells. This demonstrates that the administration of the anti-PD-1 monoclonal antibody not only promotes the immune response of the eGFP-expressing P1A-7×19 TCRT cells, but also promotes the function of endogenous immune cells that were attracted to the vicinity of cancer cells by the eGFP-expressing P1A-7×19 TCRT cells.

(Retention of P1A-TCRT Cells in Mouse Spleen)

On day 0, $5\times10^5$ P815 mastocytoma cells suspended in 0.1 ml of HBSS were subcutaneously inoculated into the flanks of male and female DBA/2 mice at an age of 6 to 8 weeks. On day 6, the mice were subjected to sublethal irradiation (3 to 5 Gy) for preconditioning. On day 7, the mice were divided into two groups, of which the first group received intravenous administration of $1\times10^6$ eGFP-expressing P1A-7×19 TCRT cells, and the second group received intravenous administration of $1\times10^6$ eGFP-expressing P1A-TCRT cells. Mice individuals that exhibited complete tumor regression were euthanized on day 119, and their splenocytes were collected, counted, and analyzed by flow cytometry to assess the retention of the P1A-TCRT cells in the spleen. In addition, T cells were isolated from the splenocytes using a magnetic sorting method, and $2\times10^6$ T cells were incubated with $1\times10^6$ P815 cells that had been treated with mitomycin C. After 3 days and after 5 days, the conditioned medium was collected and the concentration of IFN-γ was measured by ELISA. The number of P1A-TCRT cells (per well) during the period of culturing with P815 cells was also measured by flow cytometry. Based on these tests, the function of the P1A-TCRT cells in the spleen were evaluated.

The sample size was five mice with respect to the group that received administration of the eGFP-expressing P1A-7×19 TCRT cells, and was two mice with respect to the group that received administration of the eGFP-expressing P1A-TCRT cells.

The collected splenocytes were measured with respect to the expression of CD8 and eGFP, based on flow cytometry similar to the that in the experiment shown in Fig. 2 (provided that the gate was changed from quarter gates to a custom gate including a $CD8^-eGFP^+$ gate and a $CD8^+eGFP^+$ gate), and the results are shown in Fig. 7. The average proportion of cells that expressed eGFP but did not express CD8 ($CD8^-eGFP^+$ cells) in splenocytes is indicated by open bars in Fig. 8A, and the average number of cells is indicated by open bars in Fig. 8B. The average proportion of cells that expressed both of CD8 and eGFP ($CD8^+eGFP^+$ cells) in splenocytes is indicated by black solid bars in Fig. 8A, and the average number of cells is indicated by black solid bars in Fig. 8B. In Figs. 8A and 8B, the open bars are located to the left of the black solid bars, although the data for "Conv. P1A-T cells" is barely visible due to their small values. In Figs. 7, 8A, and 8B, "Conv. P1A-T cells" represents the group that received administration of the eGFP-expressing P1A-TCRT cells, and "7×19 P1A-T cells" represents the group that received administration of the eGFP-expressing P1A-7×19 TCRT cells. The results shown in Figs. 7, 8A, and 8B demonstrate that, in the group of mice that received administration of the eGFP-expressing P1A-7×19 TCRT cells, the mice retained the P1A-TCRT cells holding the introduced genes, even on day 119. The percentages noted in Fig. 7 represent the proportion of cells that expressed eGFP but did not express CD8 ($CD8^-eGFP^+$ cells) (0.055% in the group that received administration of the eGFP-expressing P1A-TCRT cells, and 0.97% in the group that received administration of the eGFP-expressing P1A-7×19 TCRT cells), and the proportion of cells that expressed both of CD8 and eGFP ($CD8^+eGFP^+$ cells) (0.0068% in the group that received administration of the eGFP-expressing P1A-TCRT cells and 0.47% in the group that received administration of the eGFP-expressing P1A-7×19 TCRT cells).

Furthermore, the number of P1A-TCRT cells (per well) that expressed both of CD8 and eGFP during the period culturing with P815 cells was measured by flow cytometry, and the results are shown in Fig. 8C. The concentration of IFN-γ obtained by ELISA measurement of the conditioned medium is shown in Fig. 8D. In Figs. 8C and 8D, the results obtained in the group that received administration of the eGFP-expressing P1A-TCRT cells are indicated by the left bars (open bars) at respective time points, and the results obtained in the group that received administration of the eGFP-expressing P1A-7×19 TCRT cells are indicated by the right bars (black solid bars) at the respective time points. Here, in Fig. 8C, the left-hand bars indicating the results obtained in the group that received administration of the eGFP-expressing P1A-TCRT cells overlaps the horizontal axis (i.e., the number of P1A-TCRT cells that expressed both of CD8 and eGFP is almost zero), as a result of which the left-hand bars are actually not observable. In Figs. 8C and 8D, the results are shown in terms of the average value and the standard deviation, where "*" indicates that the P value satisfies $P < 0.05$, and "**" indicates that the P value satisfies $P < 0.01$.

The results shown in Figs. 8C and 8D demonstrate that the function of the P1A-TCRT cells holding the introduced genes was retained in the mice even on day 119.

(Rechallenge Experiment with Cancer Cells)

On day 0, $5\times10^5$ P815 cells suspended in 0.1 ml of HBSS were subcutaneously inoculated into the flanks of male and female DBA/2 mice at an age of 6 to 8 weeks. On day 6, the mice were subjected to sublethal irradiation (3 to 5 Gy) for preconditioning. On day 7, $1\times10^6$ eGFP-expressing P1A-7×19 TCRT cells were administered intravenously (as described above, the number of cells indicated is the total number of T cells in the P1A-TCRT cell populations, which included the eGFP-expressing P1A-7×19 TCRT cells). In addition, the anti-PD-1 monoclonal antibody was injected intraperitoneally at a dose of 100 μg/individual and a frequency of once a week for a total of six times, starting on day 10. On day 117, $5\times10^5$ P815 mastocytoma cells suspended in 0.1 ml of HBSS were subcutaneously inoculated again into the flanks of four mice that exhibited complete tumor regression. In addition, $5\times10^5$ P815 mastocytoma cells suspended in 0.1 ml of HBSS were subcutaneously inoculated into the flanks of six naive DBA/2 mice, as a control. The results of measurement of the tumor volume of the mice are shown in Fig. 9 together with the number of elapsed days up to day 20, assuming that the day of the reinoculation of P815 cells into the mice that exhibited complete tumor regression or the day of the inoculation of P815 cells into the naive DBA/2 mice is day 0. The tumor volumes of the mice were measured with a digital calliper, as described regarding Figs. 5A to 5F, but the tumor volume is indicated in terms of the average value and the standard deviation in this experiment.

In Fig. 9, × indicates the result of the inoculation of P815 cells into the naive DBA/2 mice, and ● indicates the result of the reinoculation of P815 cells into the mice that exhibited complete tumor regression.

The results shown in Fig. 9 demonstrate that the mice that had been cured by the administration of the eGFP-expressing P1A-7×19 TCRT cells and the anti-PD-1 monoclonal antibody retained their resistance to cancer cells even on day 117.

[Preparation of CAR-T Cells Expressing IL-7 and CCL19]

A control anti-hCD20 CAR vector and an IL-7/CCL19-expressing-anti-hCD20 CAR vector were prepared according to the method described in paragraphs [0061] to [0066] of WO 2016/56228, and introduced into $3\times10^6$ purified mouse T cells obtained from the spleen and lymph nodes of DBA/2 mice, to prepare anti-hCD20 CAR-IL-7/CCL19-expressing T cells or anti-hCD20-CAR-expressing T cells. The control anti-hCD20 CAR vector is a vector containing a nucleic acid encoding an anti-hCD20 CAR, and the IL-7/CCL19 expression-anti-hCD20 CAR vector is a vector containing a nucleic acid encoding anti-hCD20 CAR-F2A-IL-7-F2A-CCL19 in this order.

(Analysis of Anticancer Effect Produced by Administration to Tumor-bearing Mice)

On day 0, $5\times10^5$ P815-hCD20 tumor cells suspended in 0.1 ml of HBSS were subcutaneously inoculated into the flanks of male DBA/2 mice at an age of 6 to 12 weeks (see Nat Biotechnol. 2018; 36(4): 346-351). On day 11, cyclophosphamide (CPA, 100 mg/kg), which is an anticancer agent, was administered intraperitoneally to the mice. The mice were divided into five groups, and subsequent treatment was performed as follows.

Group 1 received neither administration of CAR-expressing T cells nor administration of an antibody.

Group 2 received intraperitoneal administration of the anti-PD-1 monoclonal antibody every 4 or 5 days for a total of five times, starting on day 17.

Group 3 received intravenous administration of $0.25 \times 10^6$ anti-hCD20-CAR-expressing T cells on day 14, and received intraperitoneal administration of the anti-PD-1 monoclonal antibody at a dose of 100 μg/individual every 4 to 5 days for a total of five times, starting on day 17.

Group 4 received intravenous administration of $0.25 \times 10^6$ anti-hCD20 CAR-IL-7/CCL19-expressing T cells on day 14, and received intraperitoneal administration of a hamster control IgG antibody that does not recognize PD-1 at a dose of 100 μg/individual every 4 to 5 days for a total of 5 times, starting on day 17.

Group 5 received intravenous administration of $0.25 \times 10^6$ anti-hCD20 CAR-IL-7/CCL19-expressing T cells on day 14, and received intraperitoneal administration of the anti-PD-1 monoclonal antibody at a dose of 100 μg/individual every 4 to 5 days for a total of five times, starting on day 17.

The survival rates of the respective mice were analyzed, and the tumor volume of each mouse was measured twice a week according to the foregoing technique using a digital calliper. The results of the analysis of the survival rates of the respective mice are shown in Fig. 10A, and the results of the measurement of the tumor volume up to day 70 are shown in Figs. 10B to 10F. The data indicated was obtained by pooling the results of two independent experiments.

In Figs. 10A to 10F, × indicates mice that did not receive administration (sample size = 10 mice; the foregoing Group 1), □ indicates mice that received administration of the anti-PD-1 monoclonal antibody alone (sample size = 10 mice; the foregoing Group 2), ♦ indicates mice that received administration of the anti-hCD20-CAR-expressing T cells and the anti-PD-1 monoclonal antibody (sample size = 5 mice; the foregoing Group 3), ◦ indicates mice that received administration of the anti-hCD20 CAR-IL-7/CCL19-expressing T cells and the hamster control IgG antibody that does not recognize PD-1 (sample size = 10 mice; the foregoing Group 4), and ● indicates mice that received administration of the anti-hCD20-CAR-IL-7/CCL19-expressing T cells and the anti-PD-1 monoclonal antibody (sample size = 10 mice; the foregoing Group 5). In Figs. 10B to 10F, N = 5 for all groups. In Figs. 10A to 10F, the horizontal axis represents the number of days that elapsed since the subcutaneous inoculation with P815-hCD20 tumor cells. The number of tumor-rejecting mice (numerator) relative to the total number of mice in each group (denominator) is also shown in Figs. 10B to 10F. The P values in the log-rank test were $P = 0.7293$ between □ and ♦ groups, $P < 0.0001$ between □ and ● groups, $P < 0.0001$ between ♦ and ● groups, and $P < 0.0001$ between ◦ and ● groups.

The results shown in Figs. 10A to 10F demonstrate that the mice that received administration of the anti-hCD20 CAR-IL-7/CCL19-expressing T cells and the anti-PD-1 monoclonal antibody (●) achieved a significantly high survival rate and suppression of increase in the tumor volume of the solid tumor, even when the anti-hCD20 CAR-IL-7/CCL19-expressing T cells and the anti-PD-1 monoclonal antibody were administered in their respective doses, each of which would not produce a sufficient anticancer effect in a solo administration of the anti-hCD20 CAR-IL-7/CCL19-expressing T cells or the anti-PD-1 monoclonal antibody. In contrast, this effect was not obtained in the mice (A) that received administration of the anti-hCD20-CAR-expressing T cells and the anti-PD-1 monoclonal antibody or the mice (◦) that received administration of the anti-hCD20 CAR-IL-7/CCL19-expressing T cells and the control IgG antibody.

Further, the dose of the anti-hCD20-CAR-expressing T cells was as low as $0.25 \times 10^6$ cells as described above, and it was found that high efficacy was achieved even with a small number of cells in the case of co-administration with the anti-PD-1 monoclonal antibody.

(Analysis of Lymphocyte Infiltration into Tumor Tissue)

On day 0, P815 tumor cells were inoculated into DBA/2 mice, and the eGFP-expressing P1A-TCRT cells or the eGFP-expressing P1A-7×19 TCRT cells were intravenously injected thereto on day 7. Before injection, the purity of these cells was raised such that the proportion of eGFP-positive cells became more than 95%. Tumor cells were collected and processed into a single cell suspension, and on day 12, the cell number was counted, and the expression of c-kit, eGFP, CD11c, CD3, CD4, and CD8 was examined by flow cytometry analysis. Fig. 11A shows a representative dot plot. This figure shows the proportion of c-kit-negative cells, which were identified as non-tumor cells (upper left panel), the proportion of CD-3-negative/CD11c-positive dendritic cells in the c-kit-negative cell population (upper right panel), and the proportion of endogenous T cells, which were CD-3-positive/eGFP-negative, and the proportion of injected P1A-TCRT cells, which were CD3-positive/eGFP-positive (middle panel). In Fig. 11A, "Conv. P1A-T cells" represents the group that received administration of the eGFP-expressing P1A-TCRT cells, and "7×19 P1AT cells" represents the group that received administration of the eGFP-expressing P1A-7×19 TCRT cells. The proportion of CD4-positive cells or CD8-positive cells in the endogenous T cell population (lower left panel) and the proportion of the injected P1A-TCRT cells (lower right panel) are shown. Fig. 11B indicates the number of cells of various tumor infiltrating lymphocytes per $1 \times 10^5$ P815 tumor cells, in terms of the average value and the standard deviation (SD) (n = 5). "DC" represents dendritic cells, "Endogenous T" represents endogenous T cells, and "P1A-T" represents P1A-TCRT cells.

Open bars indicate tumor-infiltrating lymphocyte subsets in the mice that were treated with the eGFP-expressing P1A-TCRT cells, and black solid bars indicate tumor-infiltrating lymphocyte subsets in the mice that were treated with the eGFP-expressing P1A-7×19 TCRT cells. Further, * represents P < 0.05, ** represents P < 0.01, and *** represents P < 0.001.

In order to separate tumor infiltrating lymphocytes from P815 tumor cells, c-kit staining was used. It is known that P815 mastocytoma is highly positive to c-kit staining and that dendritic cells and T cells are less positive to c-kit staining. The results in Figs. 11A and 11B first demonstrate that the proportion of c-kit-negative tumor-infiltrating lymphocytes in the mice injected with the eGFP-expressing P1A-7×19 TCRT cells was higher than the proportion of c-kit-negative tumor-infiltrating lymphocytes in the mice injected with the eGFP-expressing P1A-TCRT cells. The proportion of CD3 negative/CD11c positive dendritic cells in the c-kit negative tumor infiltrating lymphocyte subset is also shown in Fig. 11A. Fig. 11A also shows the proportion of CD4 and CD8 expression in the endogenous T cell subset, which is CD3-positive/eGFP-negative, and in the injected P1A-TCRT cell or P1A-7×19 TCRT cell subset, which is CD3-positive/eGFP-positive. Further analysis of CD4/CD8 staining revealed that the number of tumor-infiltrating dendritic cells, the number of CD4-positive and CD8-positive endogenous T cells, and the number of CD8-positive P1AT cells in the mice treated with the eGFP-expressing P1A-7×19 TCRT cells were markedly higher than those in the mice treated with the eGFP-expressing P1A-TCRT cells. This is demonstrated in Fig. 11B. In Fig. 11B, black solid bars (the tumor-infiltrating lymphocyte subset in the mice treated with the eGFP-expressing P1A-7×19 TCRT cells) exhibit larger cell numbers than open bars (the tumor-infiltrating lymphocyte subset in the mice treated with the eGFP-expressing P1A-TCRT cells) with respect to each of "DC" (dendritic cells), "Engogenous T" (endogenous T cells), and the injected P1A-TCRT cells or P1A-7×19 TCRT cells. These results support the importance of the role of endogenous T cells in the treatment with the eGFP-expressing P1A-7×19 TCRT cells. This is consistent with the fact that the synergistic effect was obtained from the combination of the anti-PD-1 monoclonal antibody and the eGFP-expressing P1A-7×19 TCRT cells, but not from the combination of the anti-PD-1 monoclonal antibody and the eGFP-expressing P1A-TCRT cells.

(Preparation of Anti-hCD20-CAR-anti-PD-1-antibody Expression Vector)

A construct having the nucleic acid sequence of SEQ ID NO: 15 was introduced into a pMSGV retrovirus expression vector (Tamada k et al., Clin Cancer Res 18: 6436-6445(2002)) by making use of NcoI and SalI sites, to prepare a pMSGV vector containing anti-hCD20scFv CAR and anti-mPD-1 scFv (hereinafter also referred to as "anti-hCD20-CAR-anti-PD-1-antibody expression vector). The arrangement of the vector obtained is shown in Fig. 12 as a conventional CAR-PD-1 scFv. In the nucleic acid sequence of SEQ ID NO: 15, counting from the 5'-terminus, nucleotides at positions 1 to 57 correspond to a nucleic acid sequence encoding a leader sequence, nucleotides at positions 58 to 375 correspond to a nucleic acid sequence encoding an anti-hCD 20 scFv light chain, nucleotides at positions 376 to 420 correspond to a nucleic acid sequence encoding a linker, nucleotides at positions 421 to 783 correspond to a nucleic acid sequence encoding an anti-hCD 20 scFv heavy chain, nucleotides at positions 793 to 1635 correspond to a nucleic acid sequence encoding a transmembrane domain and a cytoplasmic domain, nucleotides at positions 1642 to 1716 correspond to a nucleic acid sequence encoding a 2A peptide, nucleotides at positions 1717 to 1773 correspond to a nucleic acid sequence encoding a leader sequence, nucleotides at positions 1774 to 2106 correspond to a nucleic acid sequence encoding an anti-mPD-1 scFv light chain, nucleotides at positions 2107 to 2151 correspond to a nucleic acid sequence encoding a linker sequence, nucleotides at positions 2152 to 2505 correspond to a nucleic acid sequence encoding an anti-mPD-1 scFv heavy chain, nucleotides at positions 2506 to 2529 correspond to a nucleic acid sequence encoding a FLAG tag, and nucleotides at positions 2539 to 2556 correspond to a nucleic acid sequence encoding the His tag. In the anti-hCD20-CAR-anti-PD-1-antibody expression vector and the anti-hCD20-CAR-IL-7/CCL19-anti-PD-1-antibody expression vector described below, the 2A peptide used was the 2A peptide of foot-and-mouth disease virus (also referred to as "F2A peptide"). The 2A peptide inserted between the genes enables plural polypeptides to be expressed simultaneously. In addition, as described above, the anti-hCD20-CAR-anti-PD-1-antibody expression vector and the anti-hCD20-CAR-IL-7/CCL19-anti-PD-1-antibody expression vector described below contain the FLAG tag and His tag (His × 6) sequences.

The amino acid sequence encoded by the nucleic acid sequence of SEQ ID NO: 15 is shown as SEQ ID NO: 16. In SEQ ID NO: 16, counting from the N-terminus, the amino acid sequence stretching from position 1 to position 545 is the amino acid sequence of the anti-hCD20 CAR, the amino acid sequence stretching from position 548 to position 572 is the amino acid sequence of the 2A peptide, the amino acid sequence stretching from position 592 to position 702 is the amino acid sequence of the anti-mPD-1 VL, the amino acid sequence stretching from position 703 to position 717 is the amino acid sequence of the linker, the amino acid sequence stretching from position 718 to position 835 is the amino acid sequence of the anti-mPD-1 VH, the amino acid sequence stretching from position 836 to position 843 is the amino acid sequence of the FLAG tag, and the amino acid sequence stretching from position 847 to position 852 is the amino acid sequence of the His tag.

(Preparation of Anti-hCD20-CAR-IL-7/CCL19-anti-PD-1-antibody expression vector)

A construct with the nucleic acid sequence of SEQ ID NO: 17 was introduced into a pMSGV retrovirus expression vector (Tamada k et al., Clin Cancer Res 18: 6436-6445(2002)) by making use of NcoI and SalI sites, to prepare a pMSGV vector containing anti-hCD20scFv CAR, mIL-7, mCCL19, and anti-mPD-1 scFv (hereinafter also referred to as "anti-hCD20-CAR-IL-7/CCL19-anti-PD-1-antibody expression vector"). The arrangement of the vector obtained is shown in Fig. 12 as 7×19 CAR-PD-1 scFv. In the nucleic acid sequence of SEQ ID NO: 17, counting from the 5'-terminus, nucleotides at positions 1 to 57 correspond to a nucleic acid sequence encoding a leader sequence, nucleotides at positions 58 to 375 correspond to a nucleic acid sequence encoding an anti-hCD 20 scFv light chain, nucleotides at positions 376 to 420 correspond to a nucleic acid sequence encoding a linker, nucleotides at positions 421 to 783 correspond to a nucleic acid sequence encoding an anti-hCD 20 scFv heavy chain, nucleotides at positions 792 to 1038 correspond to a nucleic acid sequence encoding mouse CD8, nucleotides at positions 1039 to 1161 correspond to a nucleic acid sequence encoding mouse CD 28, nucleotides at positions 1162 to 1296 correspond to a nucleic acid sequence encoding mouse 4-1BB, nucleotides at positions 1297 to 1635 correspond to a nucleic acid sequence encoding mouse CD3ζ, nucleotides at positions 1642 to 1716 correspond to a nucleic acid sequence encoding a 2A peptide, nucleotides at positions 1720 to 1794 correspond to a nucleic acid sequence encoding a leader sequence of mIL-7, nucleotides at positions 1720 to 2181 correspond to a nucleic acid sequence encoding mIL-7, nucleotides at positions 2182 to 2256 correspond to a nucleic acid sequence encoding a 2A peptide, amino acid sequences 2257 to 2331 correspond to an amino acid sequence encoding a leader sequence of mCCL19, nucleotides at positions 2257 to 2580 correspond to a nucleic acid sequence encoding mCCL19, nucleotides at positions 2584 to 2658 correspond to a nucleic acid sequence encoding a 2A peptide, nucleotides at positions 2659 to 2715 correspond to a nucleic acid sequence encoding a leader sequence, nucleotides at positions 2716 to 3048 correspond to a nucleic acid sequence encoding an anti-mPD-1 scFv light chain, nucleotides at positions 3049 to 3093 correspond to a nucleic acid sequence encoding a linker sequence, nucleotides at positions 3094 to 3447 correspond to a nucleic acid sequence encoding an anti-mPD-1scFv heavy chain, nucleotides at positions 3448 to 3471 correspond to a nucleic acid sequence encoding a FLAG tag, and nucleotides at positions 3480 to 3497 correspond to a nucleic acid sequence encoding a His tag.

The amino acid sequence encoded by the nucleic acid sequence of SEQ ID NO: 17 is shown as SEQ ID NO: 18. In SEQ ID NO: 18, counting from the N-terminus, the amino acid sequence stretching from position 1 to position 261 is an amino acid sequence of anti-hCD20scFv, the sequence stretching from position 265 to position 346 is an amino acid sequence of mCD8, the amino acid sequence stretching from position 347 to position 387 is an amino acid sequence of mCD28, the amino acid sequence stretching from position 388 to position 432 is an amino acid sequence of m4-1BB, the amino acid sequence stretching from position 433 to position 545 is an amino acid sequence of mCD3, the amino acid sequence stretching from position 548 to position 572 is an amino acid sequence of the 2A peptide, the amino acid sequence stretching from position 574 to position 727 is an amino acid sequence of mIL-7, the amino acid sequence stretching from position 728 to position 752 is an amino acid sequence of the 2A peptide, the amino acid sequence stretching from position 753 to position 860 is an amino acid sequence of mCCL19, the amino acid sequence stretching from position 862 to position 886 is an amino acid sequence of the 2A peptide, the amino acid sequence stretching from position 906 to position 1016 is an amino acid sequence of anti-mPD-1 VL, the amino acid sequence stretching from position 1017 to position 1031 is an amino acid sequence of the linker, the amino acid sequence stretching from position 1032 to position 1149 is the amino acid sequence of anti-mPD-1 VH, the amino acid sequence stretching from position 1150 to position 1157 is the amino acid sequence of the FLAG tag, and the amino acid sequence stretching from position 1161 to position 1166 is the amino acid sequence of the His tag.

(Preparation of Retrovirus to which Anti-hCD20-CAR-anti-PD-1-antibody Expression Vector or Anti-hCD20-CAR-IL-7/CCL19-anti-PD-1-antibody Expression Vector Has Been Introduced)

A retroviral vectors was prepared for the transduction of mouse T cells. Using LIPOFECTAMINE (registered trademark) 2000 or 3000 (manufactured by Life Technologies Corporation), GP2-293 packaging cells (manufactured by Takara Bio Inc.) were transfected with the anti-hCD20-CAR-anti-PD-1-antibody expression vector or anti-hCD20-CAR-IL-7/CCL19-anti-PD-1-antibody expression vector described above and pCL-Eco retroviral packaging plasmid (manufactured by Imgenex), to prepare a retrovirus to which the anti-hCD20-CAR-anti-PD-1-antibody expression vector or anti-hCD20-CAR-IL-7/CCL19-anti-PD-1-antibody expression vector had been introduced. The supernatant containing the retrovirus was collected 48 hours after transfection.

The culture liquid used for the GP2-293 cells was DMEM supplemented with 10% FCS, 100 U/ml penicillin, and 100 mg/ml streptomycin. The culture liquid used for T cells in the later-described examples was RPMI-1640 supplemented with 10% FCS, 100 U/ml penicillin, 100 mg/ml streptomycin, 50 mM 2-mercaptoethanol, and 2 mM L-glutamine.

In the same manner, an expression vector that differs from the anti-hCD20-CAR-anti-PD-1-antibody expression vector only in that the expression vector does not include the nucleic acid sequence encoding the anti-PD-1 scFv (hereinafter also referred to as anti-hCD20 CAR expression vector), and an expression vector that differs from the anti-hCD20-CAR-IL-7/CCL19-anti-PD-1-antibody expression vector only in that the expression vector does not include the nucleic acid sequence encoding the anti-PD-1 scFv (hereinafter also referred to as anti-hCD20 CAR-IL-7/CCL19 expression vector) were prepared, and retroviral vectors were prepared in the same manner, using these expression vectors.

(Transduction of Mouse T Cells)

In order to perform transduction into mouse T cells, purified mouse T cells obtained from spleen and lymph nodes were activated with immobilized an anti-CD3 monoclonal antibody (3 μg/ml), an anti-CD28 monoclonal antibody (1 μg/ml), and IL-2 (100 IU/ml) for 48 hours. Next, the supernatant containing the retrovirus to which the anti-hCD20-CAR-anti-PD-1-antibody expression vector, the anti-hCD20-CAR-IL-7/CCL19-anti-PD-1-antibody expression vector, the anti-hCD20 CAR expression vector, or the anti-hCD20 CAR-IL-7/CCL19 expression vector prepared above had been introduced was mixed with the above-described mouse T cells ($1\times10^6$ cells/ml) that had been activated on a plate coated with 25 μg/ml of RETRONECTIN (registered trademark, manufactured by Takara Bio Inc.), centrifuged at 1500 rpm for 2 hours, and then cultured in the presence of IL-2 (100 IU/ml) for 6 hours. In order to remove the retrovirus from the culture liquid, the mouse T cells were collected, transferred to a new growth medium (RPMI) containing IL-2 (100 IU/ml), and cultured for another 42 hours, to prepare mouse T cells transduced with the anti-hCD20-CAR-anti-PD-1-antibody expression vector (hereinafter also referred to as "anti-hCD20-CAR-anti-PD-1-antibody-expressing T cells"), mouse T cells transduced with the anti-hCD20-CAR-IL-7/CCL19-anti-PD-1-antibody expression vector (hereinafter also referred to as "anti-hCD20-CAR-IL-7/CCL19-anti-PD-1-antibody-expressing T cells"), mouse T cells transduced with the anti-hCD20 CAR expression vector (hereinafter also referred to as "anti-hCD20-CAR-expressing T cells"), or mouse T cells transduced with the anti-hCD20 CAR-IL-7/CCL19 expression vector (hereinafter also referred to as "anti-hCD20 CAR-IL-7/CCL19-expressing T cells").

In order to detect CAR expression, untranduced T cells (denoted as "uninf." in Fig. 13) or T cells transduced with various retroviral vectors encoding the foregoing CAR were stained with biotinylated recombinant protein L (also denoted as "proteinL-bio") and then stained with streptavidin conjugated with allophycocyanin (also denoted as "sav-apc"). The expression level of the CAR was analyzed by flow cytometry. The results are shown in Fig. 13.

In Fig. 13 and subsequent figures, "uninf." represents the untranduced T cells, "conv." represents the anti-hCD20-CAR-expressing T cells, "conv./PD-1" represents the anti-hCD20-CAR-anti-PD-1-antibody-expressing T cells, "7×19" represents the anti-hCD20 CAR-IL-7/CCL19-expressing T cells, and "7×19/PD-1" represents the anti-hCD20-CAR-IL-7/CCL19-anti-PD-1-antibody-expressing T cells. In the data for each T cell type in Fig. 13, the left graph is an FSC-SSC plot, and the right graph is a graph showing the positivity of allophycocyanin staining (horizontal axis) and the cell count (vertical axis). The histogram shown in the right graph represents the proportion (%) of cells that were positive for staining. As shown in Fig. 13, other than the untransduced T cells, T cells transduced with a retroviral vector encoding the CAR expressed the CAR, in accordance with expectation.

Prior to using the various CAR-T cells in experiments, the concentration of IL-7 and the concentration of CCL19 were measured using an ELISA kit. Specifically, the untransduced T cells, the anti-hCD20-CAR-expressing T cells, the anti-hCD20 CAR-IL-7/CCL19-expressing T cells, the anti-hCD20-CAR-anti-PD-1-antibody-expressing T cells, or the anti-hCD20-CAR-IL-7/CCL19-anti-PD-1-antibody-expressing T cells were cultured for 2 days, the conditioned medium thereof was collected, and the concentrations of IL-7 and CCL19 in the conditioned medium was measured using a commercially available ELISA kit (R&D systems). The results are shown in Fig. 14. In Fig. 14, the average value over the triplicate wells, as well as the standard deviation thereof, are indicated in the graph. In each graph, data are arranged, in the order from the left to the right: the untransduced T cells (uninf.), the anti-hCD20-CAR-expressing T cells (conv.), the anti-hCD20 CAR-IL-7/CCL19-expressing T cells (7×19), the anti-hCD20-CAR-anti-PD-1-antibody-expressing T cells (conv./PD-1), the anti-hCD20-CAR-IL-7/CCL19-anti-PD-1-antibody-expressing T cells (7×19/PD-1), and a culture medium only (data obtained by performing the same treatment on the culture liquid to which T cells were not added). As shown in Fig. 14, the expression of IL-7 and CCL19 was observed in the case of the anti-hCD20 CAR-IL-7/CCL19-expressing T cells (7×19) and in the case of the anti-hCD20-CAR-IL-7/CCL19-anti-PD-1-antibody-expressing T cells (7×19/PD-1).

Prior to using various CAR-T cells in experiments, the concentration of anti-mPD-1 scFv (also referred to PD-1scFv) was measured using two kinds of ELISA kit. Specifically, a conditioned medium of the untransduced T cells, the anti-hCD20-CAR-expressing T cells, the anti-hCD20 CAR-IL-7/CCL19-expressing T cells, the anti-hCD20-CAR-anti-PD-1-antibody-expressing T cells, or the anti-hCD20-CAR-IL-7/CCL19-anti-PD-1-antibody-expressing T cells was collected after 2 days of cultivation, and the concentrations of IL-7 and CCL19 in the conditioned medium were measured using ELISA. In the ELISA, a recombinant fusion protein including mouse PD-1 and immunoglobulin Fc portion was immobilized in the wells, PD-1 scFv was captured thereto, and an anti-FLAG tag or anti-6×His tag was used for detection. The results are shown in Fig. 15. In Fig. 15, the average value over the triplicate wells as well as the standard deviation thereof are indicated in the graph. In each graph, data are arranged from the left to the right: the untransduced T cells (uninf.), the anti-hCD20-CAR-expressing T cells (conv.), the anti-hCD20 CAR-IL-7/CCL19-expressing T cells (7×19), the anti-hCD20-CAR-anti-PD-1-antibody-expressing T cells (conv./PD-1), the anti-hCD20-CAR-IL-7/CCL19-anti-PD-1-antibody-expressing T cells (7×19/PD-1), and a culture medium only (data obtained by performing the same treatment on the culture liquid to which T cells were not added). As shown in Fig. 15, the expression of anti-PD-1 scFv was observed in the case of the anti-hCD20-CAR-anti-PD-1-antibody-expressing T cells (conv./PD-1) and in the case of the anti-hCD20-CAR-IL-7/CCL19-anti-PD-1-antibody-expressing T cells (7×19/PD-1).

(Analysis of Anticancer Effect Produced by Administration to Tumor-bearing Mice)

On day 0, $5\times10^5$ P815-hCD20 tumor cells suspended in 0.1 ml of HBSS (see Nat Biotechnol. 2018; 36(4): 346-351) were subcutaneously inoculated into the flanks of male DBA/2 mice at an age of 6 to 12 weeks. On day 11, cyclophosphamide (CPA, 100 mg/kg), which is an anticancer agent, was administered intraperitoneally to the mice. The mice were divided into five groups, and subsequent treatment was performed as follows.

Group 1 did not received administration of any CAR-expressing T cells.

Group 2 received intravenous administration of $0.25\times10^6$ anti-hCD20-CAR-expressing T cells on day 14.

Group 3 received intravenous administration of $0.25\times10^6$ anti-hCD20 CAR-IL-7/CCL19-expressing T cells on day 14.

Group 4 received intravenous administration of $0.25\times10^6$ anti-hCD20-CAR-anti-PD-1-antibody-expressing T cells on day 14.

Group 5 received intravenous administration of $0.25\times10^6$ anti-hCD20-CAR-IL-7/CCL19-anti-PD-1-antibody-expressing T cells on day 14.

The survival rates of the respective mice were analyzed, and the tumor volume of each mouse was measured twice a week according to the foregoing technique using a digital calliper. The results of the analysis of the survival rates of the respective mice are shown in Fig. 16, and the results of the measurement of the tumor volume up to day 70 are shown in Fig. 17. In Fig. 17, the tumor volume during the first 14 days is shown in a magnified view at the right side of each graph.

In Fig. 17, "cpa only" represents Group 1 (a group that received administration of cyclophosphamide but did not receive administration of CAR-expressing T cells). In Fig. 17, N = 10 for all groups. In Fig. 17, the horizontal axis represents the number of days from the subcutaneous inoculation of P815-hCD20 tumor cells. The results of the log-rank test with respect to the survival time of the mice between the respective groups are indicated in terms of P-values in the table at the bottom of Fig. 16.

The results shown in Fig. 16 demonstrate that the survival time of mice bearing P815-hCD20 was remarkably increased by using the anti-hCD20-CAR-IL-7/CCL19-anti-PD-1-antibody-expressing T cells, which correspond to the immunoresponsive cells C according to the present disclosure. In particular, it was a noteworthy result that 50% of mice were still alive after 98 days in a case in which the anti-hCD20-CAR-IL-7/CCL19-anti-PD-1-antibody-expressing T cells were used, even though most of mice died during the test period in a case in which the anti-hCD20-CAR-anti-PD-1-antibody-expressing T cells were used or in a case in which the anti-hCD20 CAR-IL-7/CCL19-expressing T cells were used. These results demonstrate that even in the case of treatment of a cancer that is difficult to treat with an immunoresponsive cell expressing interleukin-7, CCL19, and a cell surface molecule that specifically recognizes a cancer antigen, but not expressing an immunosuppression inhibiting polypeptide, or with an immunoresponsive cell expressing an immunosuppression inhibiting polypeptide and a cell surface molecule that specifically recognizes a cancer antigen, but not expressing interleukin 7 or CCL19, such a cancer can be treated with the immunoresponsive cell C according to the present disclosure. Further, the P-values demonstrate that the therapeutic effect exerted by the anti-hCD20-CAR-IL-7/CCL19-anti-PD-1-antibody-expressing T cells is a high therapeutic effect, which is statistically clearly different from the results obtained in the other groups.

As can be seen from the comparison between the amount of the anti-PD-1 scFv expressed by the anti-hCD20-CAR-anti-PD-1-antibody-expressing T cells and the amount of the anti-PD-1 scFv expressed by the anti-hCD20-CAR-IL-7/CCL19-anti-PD-1-antibody-expressing T cells in Fig. 15, the anti-hCD20-CAR-IL-7/CCL19-anti-PD-1-antibody-expressing T cells are disadvantaged in terms of introduction and expression, due to the higher number of the introduced genes, which leads to a longer nucleotide length. In consideration of this point, it will be understood that the therapeutic effect produced by the anti-hCD20-CAR-IL-7/CCL19-anti-PD-1-antibody-expressing T cells discussed above iss surprising.

From the results shown in Fig. 17, it can also be understood that an increase in tumor volume itself was suppressed in mice that received administration of the anti-hCD20-CAR-IL-7/CCL19-anti-PD-1-antibody-expressing T cells. The suppression of increase in tumor volume was not only conspicuous as compared to the case of no administration of CAR-expressing T cells (cpa only) and the case of administration of the anti-hCD20-CAR-expressing T cells (conv.), but also conspicuous as compared to the case of administration of the anti-hCD20 CAR-IL-7/CCL19-expressing T cells and the case of administration of the anti-hCD20-CAR-anti-PD-1-antibody-expressing T cells.

The dose of the anti-hCD20-CAR-expressing T cells was as low as $0.25\times10^6$ cells as described above, and it was demonstrated that high efficacy was achieved even with a small cell number.

As described above, it is demonstrated, in Examples, that a high therapeutic effect against cancer cells can be obtained based on the combination drugs and the immunoresponsive cells according to various aspects of the present disclosure.

Exemplary embodiments of the present disclosure include the following embodiments.

<1> A combination drug for use in treatment of a cancer in a subject, including:

(a) an immunoresponsive cell expressing interleukine-7, CCL19, and a cell surface molecule that specifically recognizes a cancer antigen; and
(b) an immunosuppression inhibitor.

<2> The combination drug according to <1>, wherein the immunoresponsive cell and the immunosuppression inhibitor are separately administered at different times.
<3> The combination drug according to <1> or <2>, wherein a nucleic acid encoding interleukin-7 and a nucleic acid encoding CCL19 are integrated into the genome of the immunoresponsive cell, or the nucleic acid encoding interleukin-7 and the nucleic acid encoding CCL19 are integrated together or separately in one or more vectors present in the immunoresponsive cell.
<4> The combination drug according to any one of <1> to <3>, wherein the immunoresponsive cell is derived from the subject itself.
<5> The combination drug according to any one of <1> to <4>, wherein the immunoresponsive cell is selected from the group consisting of lymphocytic cells such as T cells, natural killer cells (NK cells), and B cells, antigen-presenting cells such as monocytes, macrophages, and dendritic cells, and neutrophils, eosinophils, basophils, and mast cells.
<6> A combination drug for use in treatment of a cancer in a subject, including:

(a) one or more kinds of cells, one or more kinds of nucleic acid delivery vehicles, or a combination thereof, which cooperatively include a nucleic acid encoding interleukine-7 and a nucleic acid encoding CCL19; and
(b) an immunosuppression inhibitor.

<7> The combination drug according to <6>, wherein the one or more kinds of cells, one or more kinds of nucleic acid delivery vehicles, or combination thereof include at least one selected from the group consisting of an immunoresponsive cell, a virus, an anaerobic microorganism, a liposome, a mesenchymal stem cell (MSC), and a nanoparticle.
<8> The combination drug according to <6> or <7>, wherein the one or more kinds of cells, one or more kinds of nucleic acid delivery vehicles, or combination thereof have, on a surface thereof, a molecule that specifically recognizes a cancer antigen.
<9> The combination drug according to <6> or <7>, wherein the one or more kinds of cells, one or more kinds of nucleic acid delivery vehicles, or combination thereof further include a nucleic acid encoding a cell surface molecule that specifically recognizes a cancer antigen, and the cell surface molecule that specifically recognizes a cancer antigen is a chimeric antigen receptor (CAR) or a T-cell receptor (TCR).
<10> The combination drug according to any one of <6> to <9>, wherein the immunosuppression inhibitor is a polypeptide, and the cells, nucleic acid delivery vehicles, or combination thereof, cooperatively further include a nucleic acid encoding an immunosuppression inhibiting polypeptide.
<11> The combination drug according to any one of <6> to <9>, wherein the cells, nucleic acid delivery vehicles, or combination thereof, and the immunosuppression inhibitor are separately administered at different times.
<12> The combination drug according to any one of <1> to <5>, wherein the cell surface molecule that specifically recognizes a cancer antigen is a chimeric antigen receptor (CAR) or a T-cell receptor (TCR).
<13> The combination drug according to any one of <1> to <12>, wherein the immunosuppression inhibitor includes at least one selected from the group consisting of a PD-1 inhibitor, a PD-L1 inhibitor, a PD-L2 inhibitor, a CTLA-4 inhibitor, a BTLA (B- and T-lymphocyte attenuator) inhibitor, a TIM-3 (T-cell immunoglobulin and mucin domain 3) inhibitor, a TIGIT (T-cell immunoreceptor with Ig and ITIM domains) inhibitor, a LAG-3 (Lymphocyte Activation Gene-3) inhibitor, and a Siglec-15 inhibitor.
<14> The combination drug according to any one of <1> to <13>, wherein the immunosuppression inhibitor is an antibody.
<15> The combination drug according to <14>, wherein the antibody is an IgG monoclonal antibody or an antibody fragment.
<16> The combination drug according to any one of <1> to <15>, wherein the cancer is a solid cancer.

<17> A drug for combined use with an immunosuppression inhibitor in treatment of a cancer in a subject, the drug including (i) an immunoresponsive cell expressing interleukin-7, CCL19, and a cell surface molecule that specifically recognizes a cancer antigen, or (ii) one or more kinds of cells, one or more kinds of nucleic acid delivery vehicles, or a combination thereof, which cooperatively include a nucleic acid encoding interleukine-7 and a nucleic acid encoding CCL19.
<18> A drug for combined use with (i) an immunoresponsive cell expressing interleukin-7, CCL19, and a cell surface molecule that specifically recognizes a cancer antigen, or (ii) one or more kinds of cells, one or more kinds of nucleic acid delivery vehicles, or a combination thereof, which cooperatively include a nucleic acid encoding interleukine-7 and a nucleic acid encoding CCL19, in treatment of a cancer in a subject, the drug including an immunosuppression inhibitor.
<19> The drug according to <17> or <18> for use in a mode in which the immunosuppression inhibitor, and the immunoresponsive cell or the one or more kinds of cells, one or more kinds of nucleic acid delivery vehicles, or combination thereof, are administered separately at different times.
<20> A drug including (i) an immunoresponsive cell expressing interleukin-7, CCL19, and a cell surface molecule that specifically recognizes a cancer antigen, or (ii) one or more kinds of cells, one or more kinds of nucleic acid delivery vehicles, or a combination thereof, which cooperatively include a nucleic acid encoding interleukine-7 and a nucleic acid encoding CCL19, the drug being contained in a container carrying an indication of instruction for combined use with an immunosuppression inhibitor.
<21> A product including:

a label describing an instruction for combined use with an immunosuppression inhibitor, and
a container containing a drug including (i) an immunoresponsive cell expressing interleukin-7, CCL19, and a cell surface molecule that specifically recognizes a cancer antigen or (ii) one or more kinds of cells, one or more kinds of nucleic acid delivery vehicles, or a combination thereof, which cooperatively include a nucleic acid encoding interleukine-7 and a nucleic acid encoding CCL19.

<22> A pharmaceutical composition for use in treatment of a cancer in a subject, the pharmaceutical composition including:

(a) (i) an immunoresponsive cell expressing interleukine-7, CCL19, and a cell surface molecule that specifically recognizes a cancer antigen, or (ii) one or more kinds of cells, one or more kinds of nucleic acid delivery vehicles, or a combination thereof, which cooperatively include a nucleic acid encoding interleukine-7 and a nucleic acid encoding CCL19; and
(b) an immunosuppression inhibitor.

<23> The pharmaceutical composition according to <22>, wherein the cell surface molecule that specifically recognizes a cancer antigen is a chimeric antigen receptor (CAR) or a T-cell receptor (TCR).
<24> An immunoresponsive cell, expressing interleukin-7, CCL19, an immunosuppression inhibiting polypeptide, and a cell surface molecule that specifically recognizes a cancer antigen.
<25> The immunoresponsive cell according to <24> wherein a nucleic acid encoding interleukin-7 and a nucleic acid encoding CCL19 are integrated into the genome of the immunoresponsive cell, or the nucleic acid encoding interleukin-7 and the nucleic acid encoding CCL19 are integrated together or separately in one or more vectors present in the immunoresponsive cell.
<26> The immunoresponsive cell according to <25>, wherein a nucleic acid encoding an immunosuppression inhibiting polypeptide is integrated into the genome of the immunoresponsive cell, or integrated into a vector that is the same as one of the one or more vectors that are present in the immunoresponsive cell, or different from any of the one or more vectors that are present in the immunoresponsive cell.
<27> The immunoresponsive cell according to any one of <24> to <26>, wherein the cell surface molecule that specifically recognizes a cancer antigen is a chimeric antigen receptor (CAR) or a T-cell receptor (TCR).
<28> The immunoresponsive cell according to any one of <24> to <27>, wherein the immunosuppression inhibiting polypeptide includes at least one selected from the group consisting of a PD-1 inhibiting polypeptide, a PD-L1 inhibiting polypeptide, a PD-L2 inhibiting polypeptide, a CTLA-4 inhibiting polypeptide, a BTLA (B- and T-lymphocyte attenuator) inhibiting polypeptide, a TIM-3 (T-cell immunoglobulin and mucin domain 3) inhibiting polypeptide, a TIGIT (T-cell immunoreceptor with Ig and ITIM domains) inhibiting polypeptide, a LAG-3 (Lymphocyte Activation Gene-3) inhibiting polypeptide, and a Siglec-15 inhibiting polypeptide.
<29> The immunoresponsive cell according to any one of <24> to <28>, wherein the immunosuppression inhibiting polypeptide is an antibody.
<30> The immunoresponsive cell according to <29>, wherein the antibody is an IgG monoclonal antibody or an antibody fragment.

<31> The immunoresponsive cell according to any one of <24> to <30> wherein the immunoresponsive cell is selected from the group consisting of lymphocytic cells such as T cells, natural killer cells (NK cells), and B cells, antigen-presenting cells such as monocytes, macrophages, and dendritic cells, and neutrophils, eosinophils, basophils, and mast cells.
<32> A drug including the immunoresponsive cell according to any one of <24> to <31>.
<33> The drug according to <32>, for use in treatment of a cancer in a subject.
<34> The drug according to <33>, wherein the cancer is a solid cancer.
<35> The drug according to <33> or <34>, wherein the immunoresponsive cell is derived from the subject itself.
<36> One or more kinds of nucleic acid delivery vehicles that cooperatively include a nucleic acid encoding interleukine-7, a nucleic acid encoding CCL19, and a nucleic acid encoding an immunosuppression inhibiting polypeptide.
<37> The nucleic acid delivery vehicles according to <36>, further including a nucleic acid encoding a cell surface molecule that specifically recognizes a cancer antigen.

This application claims priority from Japanese Patent Application No. 2019-195407, filed October 28, 2019. The disclosure of Japanese Patent Application No. 2019-195407 is incorporated by reference herein in its entirety.
All publications, patent applications, and technical standards mentioned in this specification are herein incorporated by reference to the same extent as if each individual publication, patent application, or technical standard was specifically and individually indicated to be incorporated by reference.

## Claims

1. A combination drug for use in treatment of a cancer in a subject, comprising:

   (a) an immunoresponsive cell expressing interleukine-7, CCL19, and a cell surface molecule that specifically recognizes a cancer antigen; and
   (b) an immunosuppression inhibitor.

2. The combination drug according to claim 1, wherein the immunoresponsive cell and the immunosuppression inhibitor are separately administered at different times.

3. The combination drug according to claim 1 or claim 2, wherein a nucleic acid encoding interleukin-7 and a nucleic acid encoding CCL19 are integrated into a genome of the immunoresponsive cell, or the nucleic acid encoding interleukin-7 and the nucleic acid encoding CCL19 are integrated together or separately in one or more vectors present in the immunoresponsive cell.

4. The combination drug according to any one of claims 1 to 3, wherein the immunoresponsive cell is derived from the subject itself.

5. The combination drug according to any one of claims 1 to 4, wherein the immunoresponsive cell is selected from the group consisting of lymphocytic cells such as T cells, natural killer cells (NK cells), and B cells, antigen-presenting cells such as monocytes, macrophages, and dendritic cells, and neutrophils, eosinophils, basophils, and mast cells.

6. A combination drug for use in treatment of a cancer in a subject, comprising:

   (a) one or more kinds of cells, one or more kinds of nucleic acid delivery vehicles, or a combination thereof, which cooperatively include a nucleic acid encoding interleukine-7 and a nucleic acid encoding CCL19; and
   (b) an immunosuppression inhibitor.

7. The combination drug according to claim 6, wherein the one or more kinds of cells, one or more kinds of nucleic acid delivery vehicles, or combination thereof comprise at least one selected from the group consisting of an immunoresponsive cell, a virus, an anaerobic microorganism, a liposome, a mesenchymal stem cell (MSC), and a nanoparticle.

8. The combination drug according to claim 6 or claim 7, wherein the one or more kinds of cells, one or more kinds of nucleic acid delivery vehicles, or combination thereof have, on a surface thereof, a molecule that specifically recognizes a cancer antigen.

9. The combination drug according to claim 6 or claim 7, wherein the one or more kinds of cells, one or more kinds of nucleic acid delivery vehicles, or combination thereof further comprise a nucleic acid encoding a cell surface molecule that specifically recognizes a cancer antigen, and the cell surface molecule that specifically recognizes a cancer antigen is a chimeric antigen receptor (CAR) or a T-cell receptor (TCR).

10. The combination drug according to any one of claims 6 to 9, wherein the immunosuppression inhibitor is a polypeptide, and the cells, nucleic acid delivery vehicles, or combination thereof, cooperatively further comprise a nucleic acid encoding an immunosuppression inhibiting polypeptide.

11. The combination drug according to any one of claims 6 to 9, wherein the cells, nucleic acid delivery vehicles, or combination thereof, and the immunosuppression inhibitor are separately administered at different times.

12. The combination drug according to any one of claims 1 to 5, wherein the cell surface molecule that specifically recognizes a cancer antigen is a chimeric antigen receptor (CAR) or a T-cell receptor (TCR).

13. The combination drug according to any one of claims 1 to 12, wherein the immunosuppression inhibitor comprises at least one selected from the group consisting of a PD-1 inhibitor, a PD-L1 inhibitor, a PD-L2 inhibitor, a CTLA-4 inhibitor, a BTLA (B- and T-lymphocyte attenuator) inhibitor, a TIM-3 (T-cell immunoglobulin and mucin domain 3) inhibitor, a TIGIT (T-cell immunoreceptor with Ig and ITIM domains) inhibitor, a LAG-3 (Lymphocyte Activation Gene-3) inhibitor, and a Siglec-15 inhibitor.

14. The combination drug according to any one of claims 1 to 13, wherein the immunosuppression inhibitor is an antibody.

15. The combination drug according to claim 14, wherein the antibody is an IgG monoclonal antibody or an antibody fragment.

16. The combination drug according to any one of claims 1 to 15, wherein the cancer is a solid cancer.

17. A drug for combined use with an immunosuppression inhibitor in treatment of a cancer in a subject, the drug comprising (i) an immunoresponsive cell expressing interleukin-7, CCL19, and a cell surface molecule that specifically recognizes a cancer antigen, or (ii) one or more kinds of cells, one or more kinds of nucleic acid delivery vehicles, or a combination thereof, which cooperatively include a nucleic acid encoding interleukine-7 and a nucleic acid encoding CCL19.

18. A drug for combined use with (i) an immunoresponsive cell expressing interleukin-7, CCL19, and a cell surface molecule that specifically recognizes a cancer antigen, or (ii) one or more kinds of cells, one or more kinds of nucleic acid delivery vehicles, or a combination thereof, which cooperatively include a nucleic acid encoding interleukine-7 and a nucleic acid encoding CCL19, in treatment of a cancer in a subject, the drug comprising an immunosuppression inhibitor.

19. The drug according to claim 17 or claim 18 for use in a mode in which the immunosuppression inhibitor, and the immunoresponsive cell or the one or more kinds of cells, one or more kinds of nucleic acid delivery vehicles, or combination thereof, are administered separately at different times.

20. A drug comprising (i) an immunoresponsive cell expressing interleukin-7, CCL19, and a cell surface molecule that specifically recognizes a cancer antigen, or (ii) one or more kinds of cells, one or more kinds of nucleic acid delivery vehicles, or a combination thereof, which cooperatively include a nucleic acid encoding interleukine-7 and a nucleic acid encoding CCL19, the drug being contained in a container carrying an indication of instruction for combined use with an immunosuppression inhibitor.

21. A product comprising:

a label describing an instruction for combined use with an immunosuppression inhibitor, and
a container containing a drug including (i) an immunoresponsive cell expressing interleukin-7, CCL19, and a cell surface molecule that specifically recognizes a cancer antigen or (ii) one or more kinds of cells, one or more kinds of nucleic acid delivery vehicles, or a combination thereof, which cooperatively include a nucleic acid encoding interleukine-7 and a nucleic acid encoding CCL19.

22. A pharmaceutical composition for use in treatment of a cancer in a subject, the pharmaceutical composition comprising:

(a) (i) an immunoresponsive cell expressing interleukine-7, CCL19, and a cell surface molecule that specifically recognizes a cancer antigen, or (ii) one or more kinds of cells, one or more kinds of nucleic acid delivery vehicles, or a combination thereof, which cooperatively include a nucleic acid encoding interleukine-7 and a nucleic acid encoding CCL19; and
(b) an immunosuppression inhibitor.

23. The pharmaceutical composition according to claim 22, wherein the cell surface molecule that specifically recognizes a cancer antigen is a chimeric antigen receptor (CAR) or a T-cell receptor (TCR).

24. An immunoresponsive cell, expressing interleukin-7, CCL19, an immunosuppression inhibiting polypeptide, and a cell surface molecule that specifically recognizes a cancer antigen.

25. The immunoresponsive cell according to claim 24, wherein a nucleic acid encoding interleukin-7 and a nucleic acid encoding CCL19 are integrated into a genome of the immunoresponsive cell, or the nucleic acid encoding interleukin-7 and the nucleic acid encoding CCL19 are integrated together or separately in one or more vectors present in the immunoresponsive cell.

26. The immunoresponsive cell according to claim 25, wherein a nucleic acid encoding an immunosuppression inhibiting polypeptide is integrated into a genome of the immunoresponsive cell, or integrated into a vector that is the same as one of the one or more vectors that are present in the immunoresponsive cell or different from any of the one or more vectors that are present in the immunoresponsive cell.

**27.** The immunoresponsive cell according to any one of claims 24 to 26, wherein the cell surface molecule that specifically recognizes a cancer antigen is a chimeric antigen receptor (CAR) or a T-cell receptor (TCR).

**28.** The immunoresponsive cell according to any one of claims 24 to 27, wherein the immunosuppression inhibiting polypeptide comprises at least one selected from the group consisting of a PD-1 inhibiting polypeptide, a PD-L1 inhibiting polypeptide, a PD-L2 inhibiting polypeptide, a CTLA-4 inhibiting polypeptide, a BTLA (B- and T-lymphocyte attenuator) inhibiting polypeptide, a TIM-3 (T-cell immunoglobulin and mucin domain 3) inhibiting polypeptide, a TIGIT (T-cell immunoreceptor with Ig and ITIM domains) inhibiting polypeptide, a LAG-3 (Lymphocyte Activation Gene-3) inhibiting polypeptide, and a Siglec-15 inhibiting polypeptide.

**29.** The immunoresponsive cell according to any one of claims 24 to 28, wherein the immunosuppression inhibiting polypeptide is an antibody.

**30.** The immunoresponsive cell according to claim 29, wherein the antibody is an IgG monoclonal antibody or an antibody fragment.

**31.** The immunoresponsive cell according to any one of claims 24 to 30, wherein the immunoresponsive cell is selected from the group consisting of lymphocytic cells such as T cells, natural killer cells (NK cells), and B cells, antigen-presenting cells such as monocytes, macrophages, and dendritic cells, and neutrophils, eosinophils, basophils, and mast cells.

**32.** A drug comprising the immunoresponsive cell according to any one of claims 24 to 31.

**33.** The drug according to claim 32, for use in treatment of a cancer in a subject.

**34.** The drug according to claim 33, wherein the cancer is a solid cancer.

**35.** The drug according to claim 33 or claim 34, wherein the immunoresponsive cell is derived from the subject itself.

**36.** One or more kinds of nucleic acid delivery vehicles that cooperatively comprise a nucleic acid encoding interleukine-7, a nucleic acid encoding CCL19, and a nucleic acid encoding an immunosuppression inhibiting polypeptide.

**37.** The nucleic acid delivery vehicles according to claim 36, further comprising a nucleic acid encoding a cell surface molecule that specifically recognizes a cancer antigen.

FIG.1A

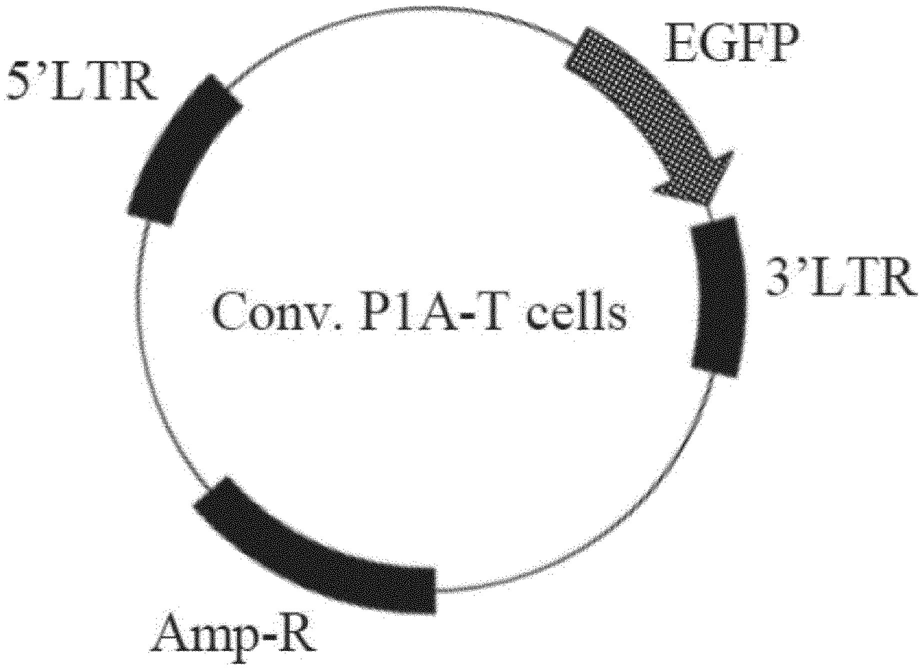
5'LTR
EGFP
3'LTR
Conv. P1A-T cells
Amp-R

FIG.1B

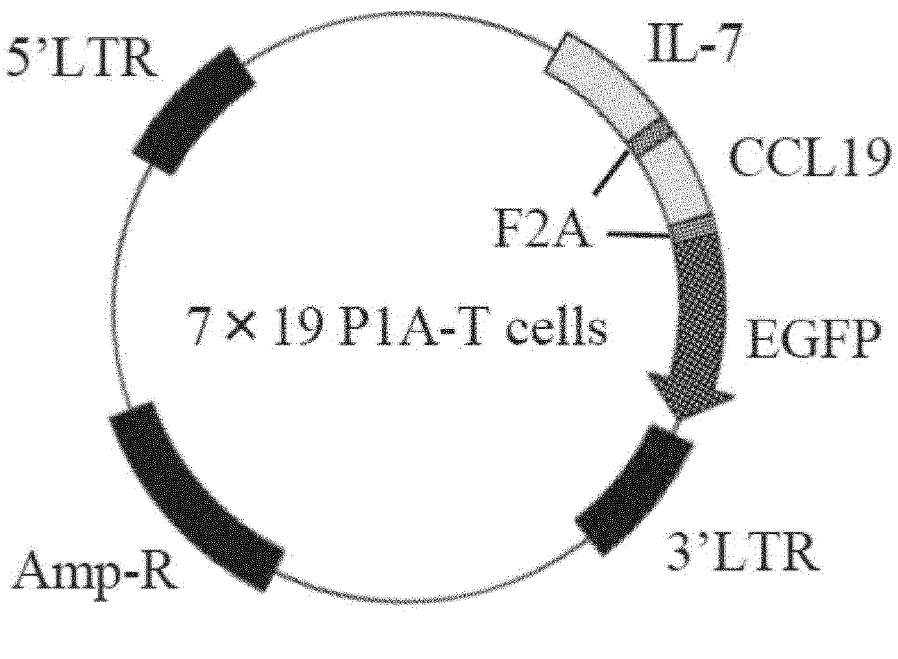
5'LTR
IL-7
CCL19
F2A
7×19 P1A-T cells
EGFP
Amp-R
3'LTR

FIG.2

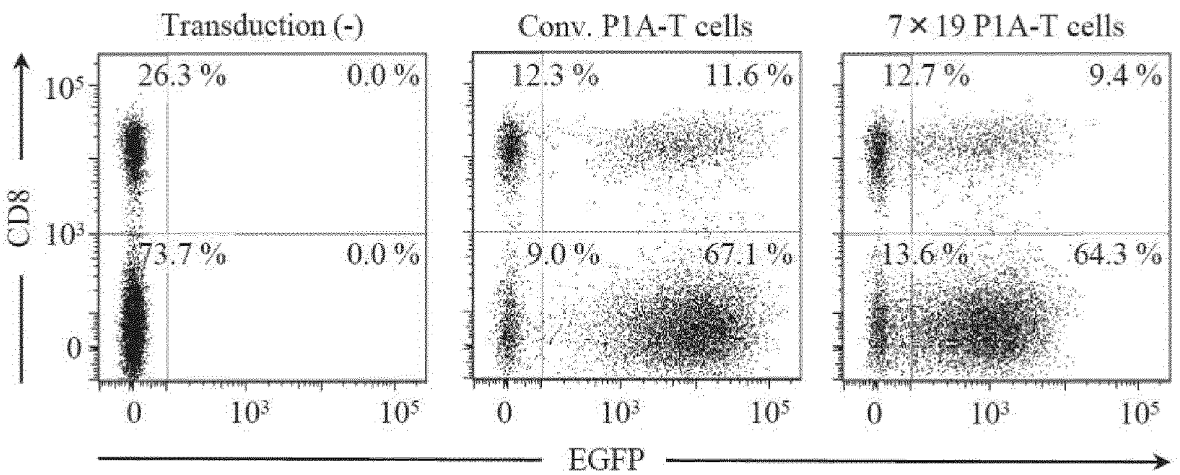
Transduction (-)
Conv. P1A-T cells
7×19 P1A-T cells
26.3 %
0.0 %
73.7 %
0.0 %
12.3 %
11.6 %
9.0 %
67.1 %
12.7 %
9.4 %
13.6 %
64.3 %
CD8
EGFP
0
$10^3$
$10^5$

FIG.3A

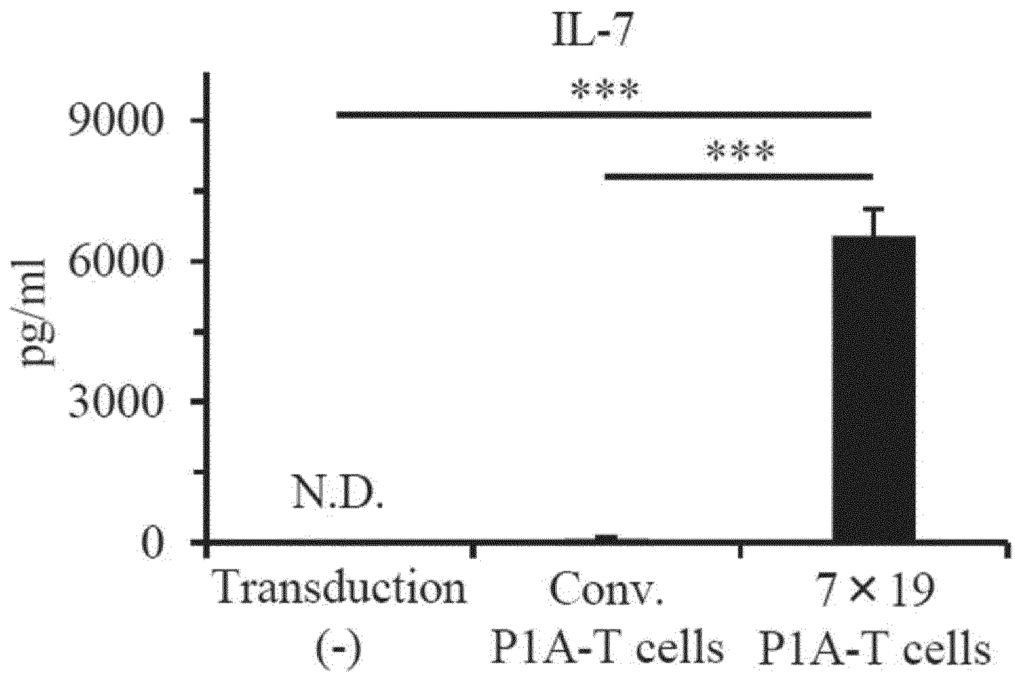
IL-7
pg/ml
9000
6000
3000
0
***
***
N.D.
Transduction (-)
Conv. P1A-T cells
7×19 P1A-T cells

FIG.3B

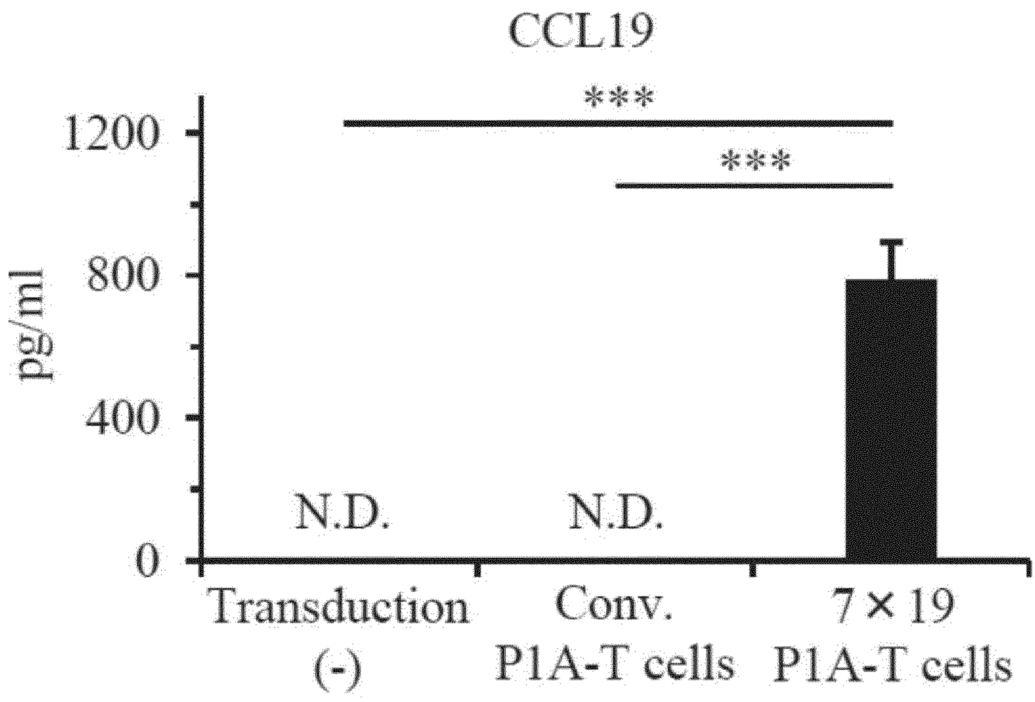
CCL19
pg/ml
1200
800
400
0
***
***
N.D.
N.D.
Transduction (-)
Conv. P1A-T cells
7×19 P1A-T cells

FIG.4

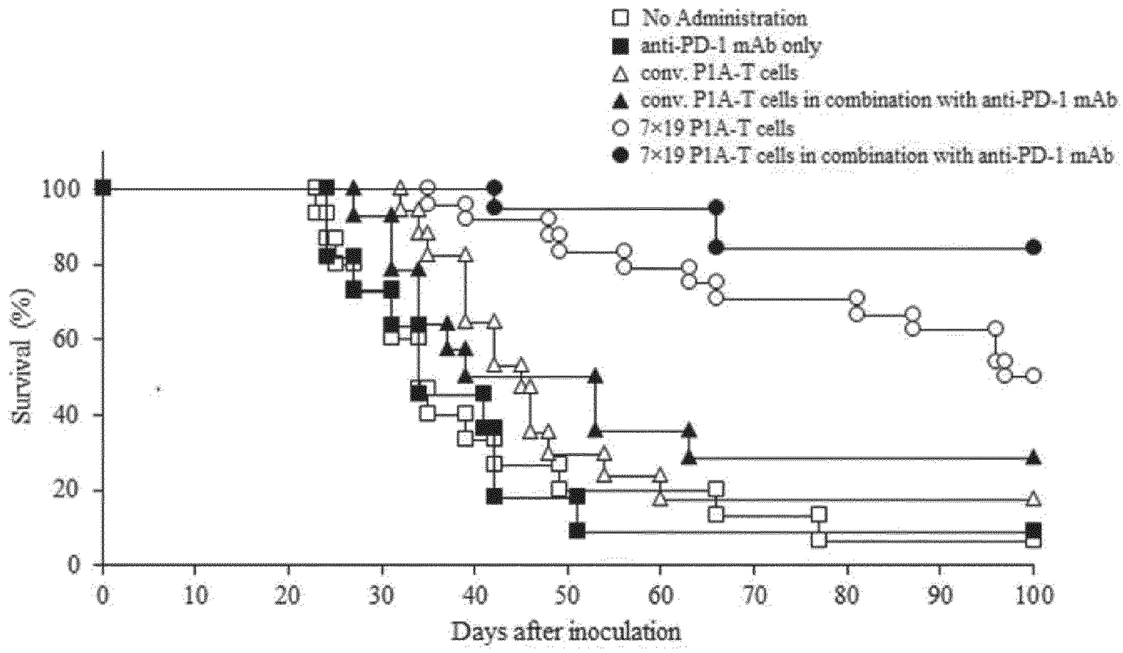
No Administration
anti-PD-1 mAb only
conv. P1A-T cells
conv. P1A-T cells in combination with anti-PD-1 mAb
7×19 P1A-T cells
7×19 P1A-T cells in combination with anti-PD-1 mAb
Survival (%)
100
80
60
40
20
0
0
10
20
30
40
50
60
70
80
90
100
Days after inoculation

FIG.5A

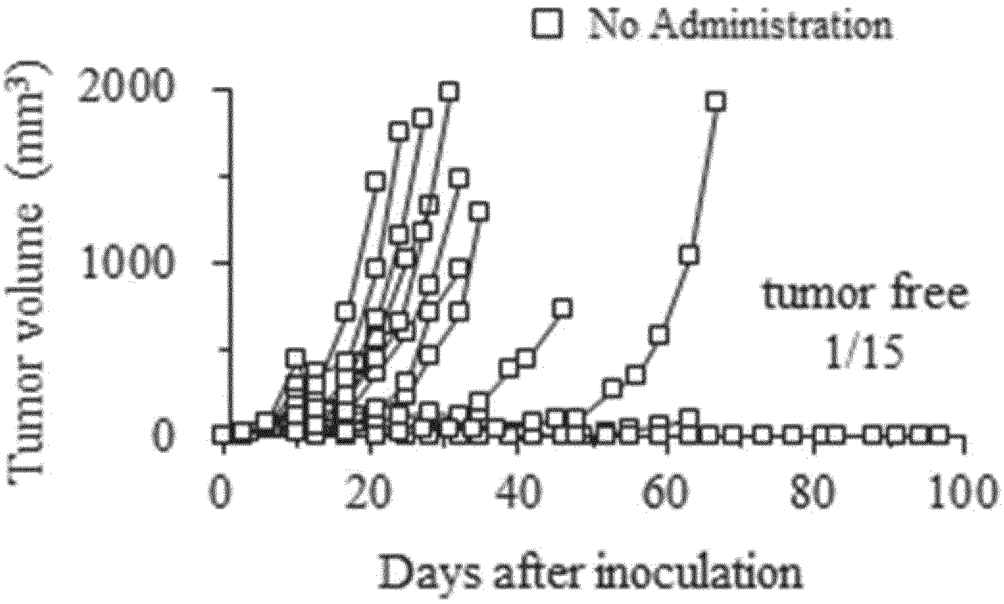
No Administration
Tumor volume (mm³)
2000
1000
0
tumor free
1/15
0
20
40
60
80
100
Days after inoculation

FIG.5B

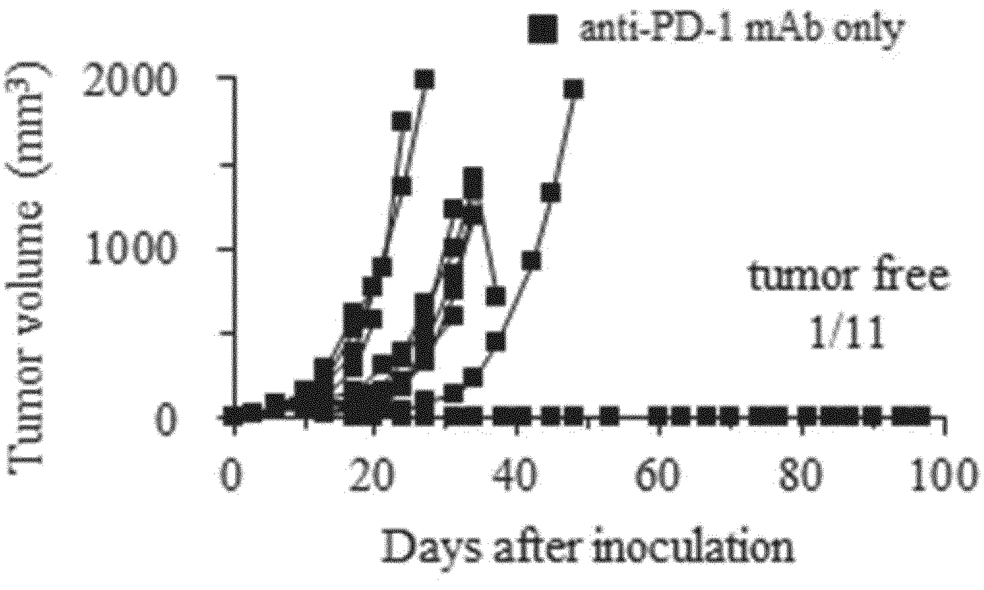
anti-PD-1 mAb only
Tumor volume (mm³)
2000
1000
0
tumor free
1/11
0
20
40
60
80
100
Days after inoculation

FIG.5C

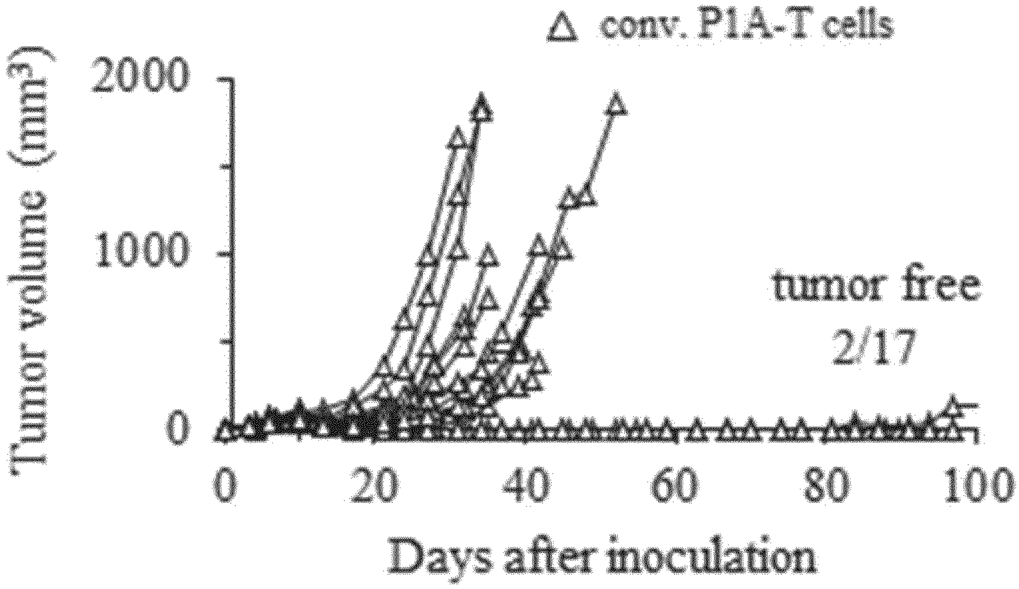
△ conv. P1A-T cells
Tumor volume (mm³)
2000
1000
0
tumor free
2/17
0
20
40
60
80
100
Days after inoculation

FIG.5D

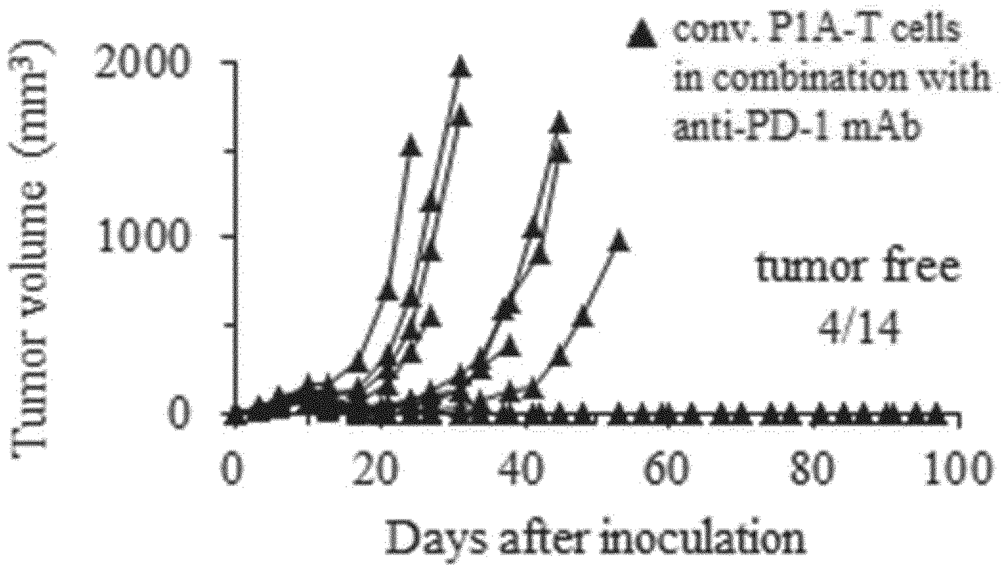
▲ conv. P1A-T cells
in combination with
anti-PD-1 mAb
Tumor volume (mm³)
2000
1000
0
tumor free
4/14
0
20
40
60
80
100
Days after inoculation

FIG.5E

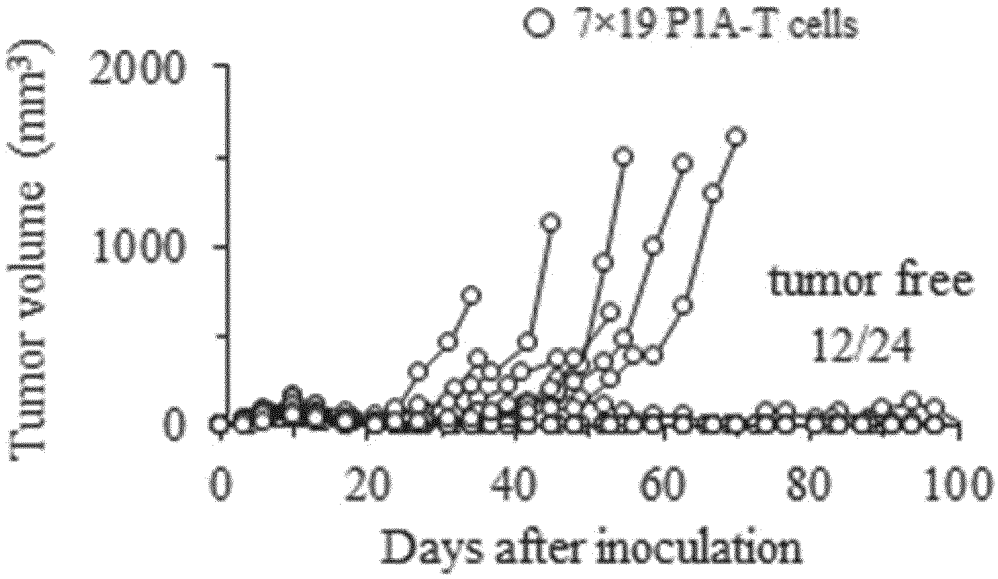
○ 7×19 P1A-T cells
2000
1000
0
Tumor volume (mm$^3$)
tumor free
12/24
0
20
40
60
80
100
Days after inoculation

FIG.5F

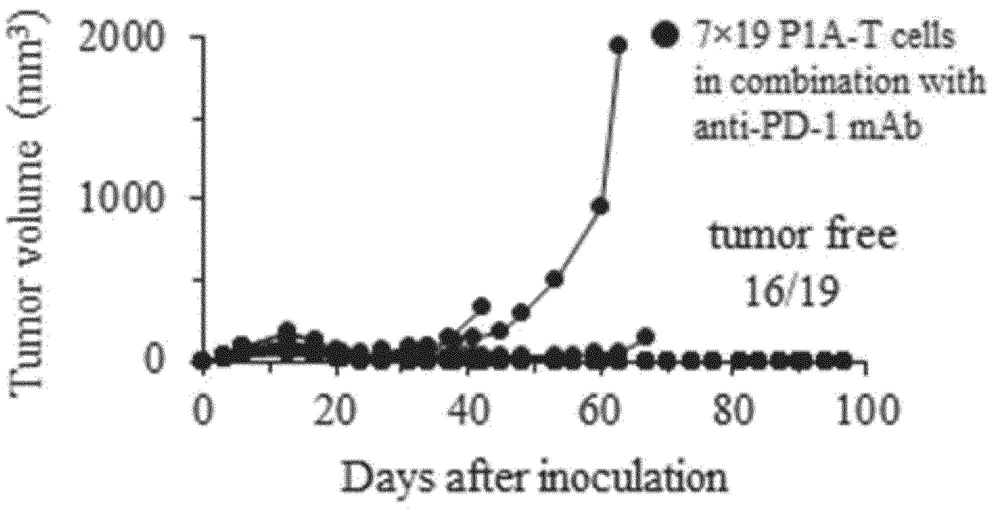
● 7×19 P1A-T cells
in combination with
anti-PD-1 mAb
2000
1000
0
Tumor volume (mm$^3$)
tumor free
16/19
0
20
40
60
80
100
Days after inoculation

FIG.6A

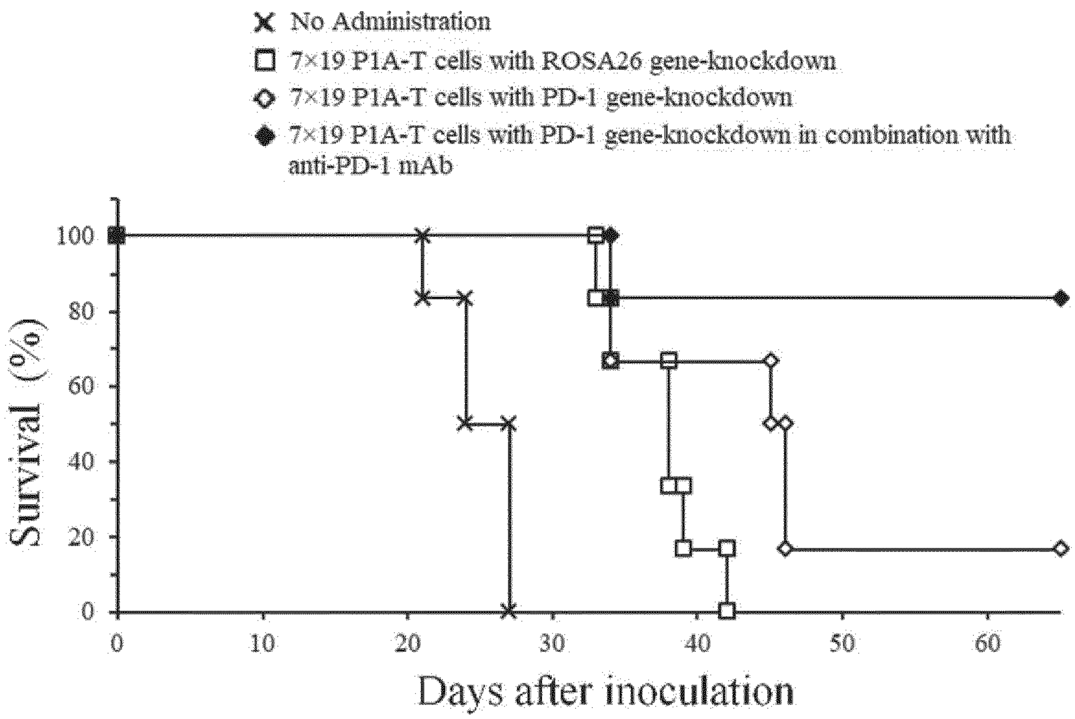
No Administration
7×19 P1A-T cells with ROSA26 gene-knockdown
7×19 P1A-T cells with PD-1 gene-knockdown
7×19 P1A-T cells with PD-1 gene-knockdown in combination with anti-PD-1 mAb
Survival (%)
Days after inoculation

FIG.6B

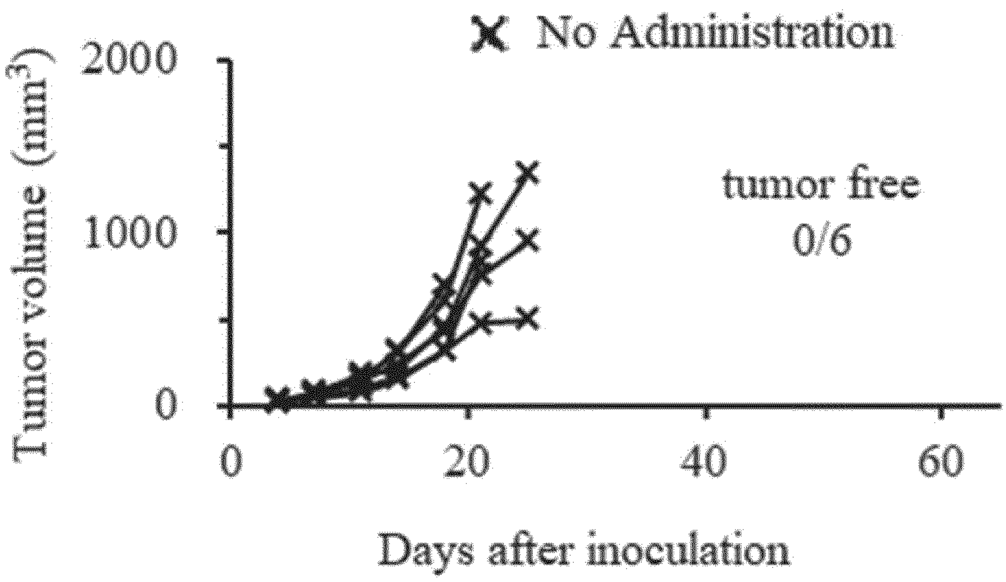
No Administration
tumor free
0/6
Tumor volume (mm3)
Days after inoculation

FIG.6C

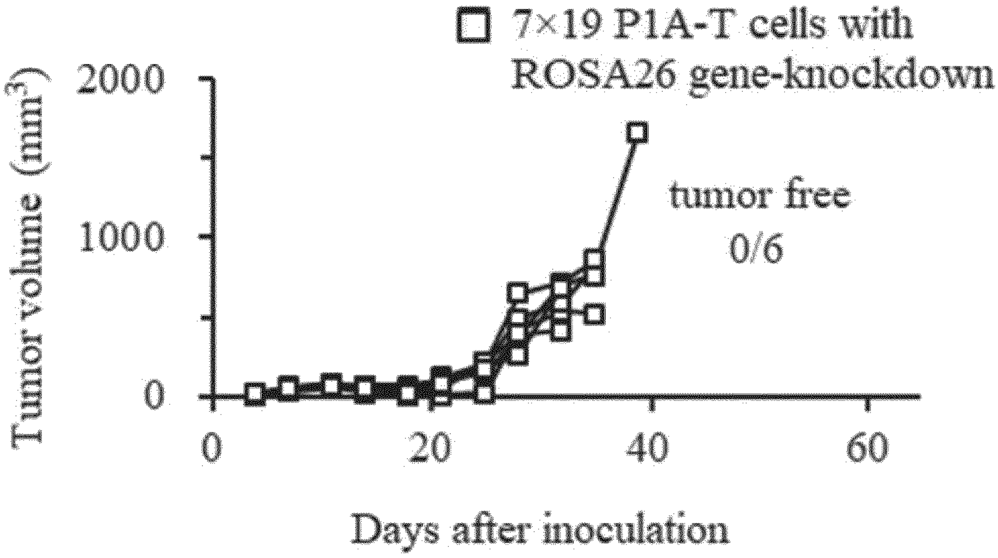
7×19 P1A-T cells with ROSA26 gene-knockdown
tumor free
0/6
Tumor volume (mm$^3$)
2000
1000
0
0
20
40
60
Days after inoculation

FIG.6D

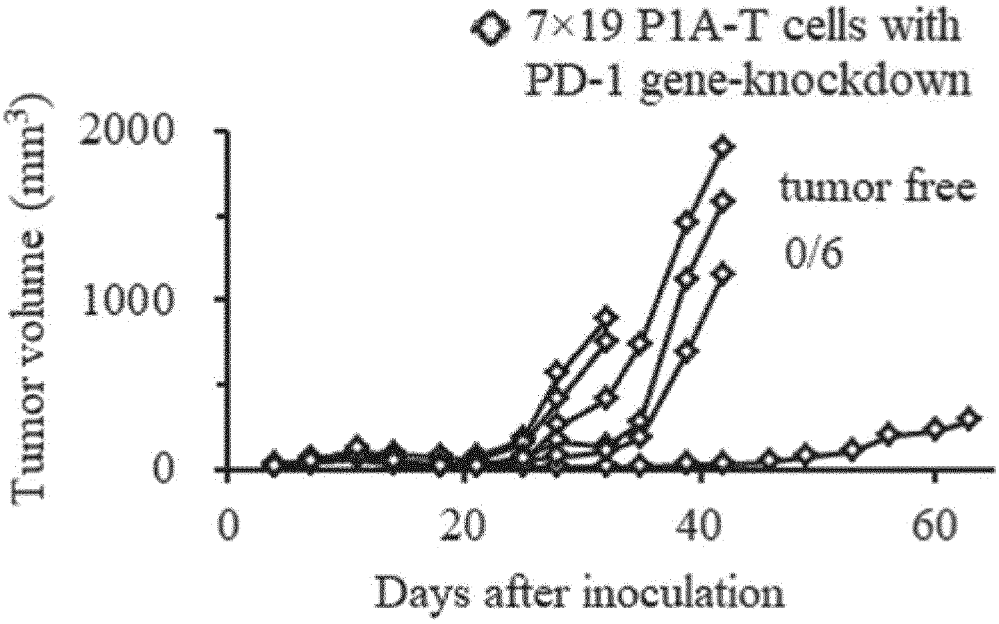
7×19 P1A-T cells with PD-1 gene-knockdown
tumor free
0/6
Tumor volume (mm$^3$)
2000
1000
0
0
20
40
60
Days after inoculation

FIG.6E

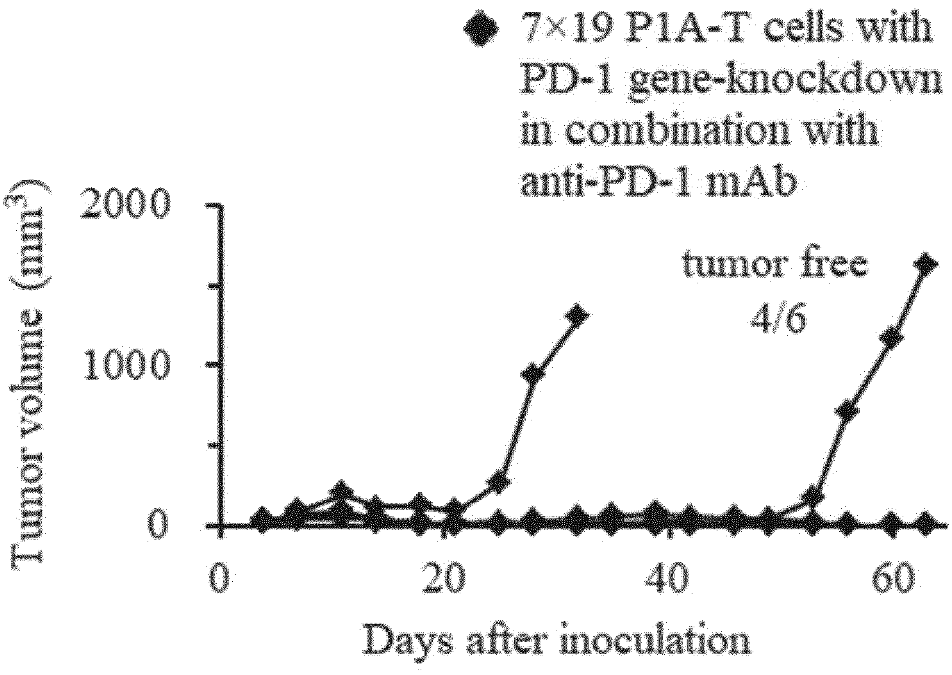
7×19 P1A-T cells with PD-1 gene-knockdown in combination with anti-PD-1 mAb
tumor free 4/6
Tumor volume (mm³)
2000
1000
0
0
20
40
60
Days after inoculation

FIG.7

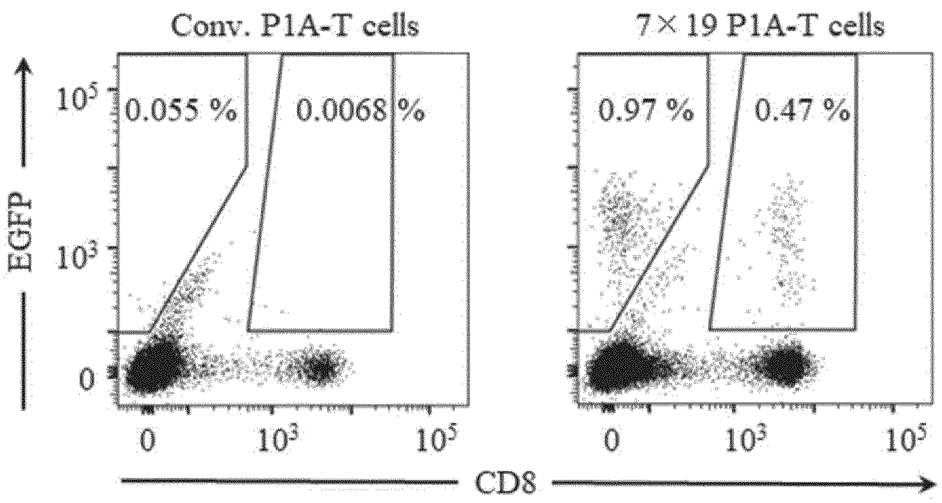
Conv. P1A-T cells
7×19 P1A-T cells
0.055 %
0.0068 %
0.97 %
0.47 %
EGFP
$10^5$
$10^3$
0
$10^3$
$10^5$
CD8

FIG.8A

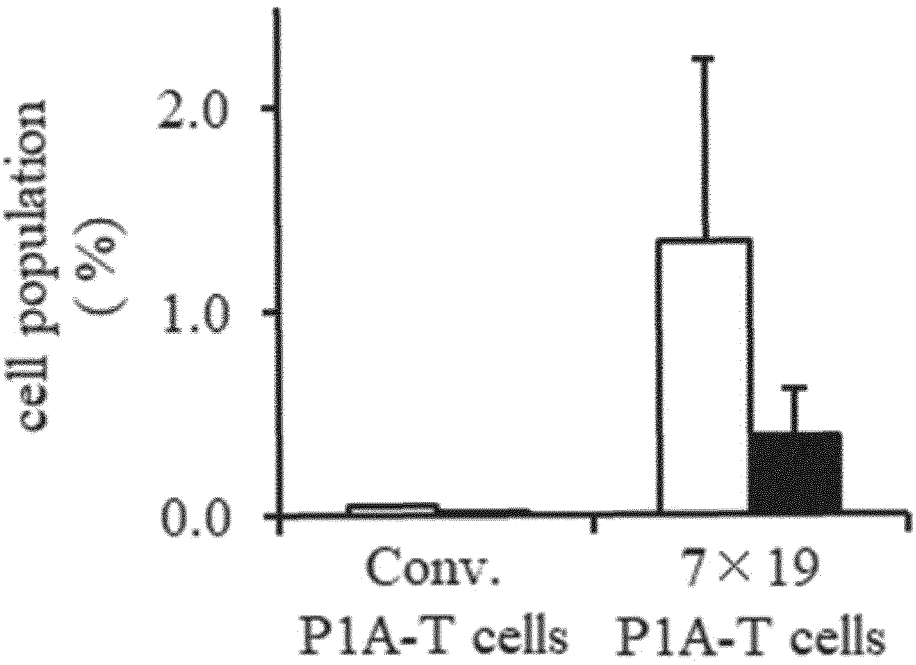
cell population
( % )
2.0
1.0
0.0
Conv.
P1A-T cells
7×19
P1A-T cells

FIG.8B

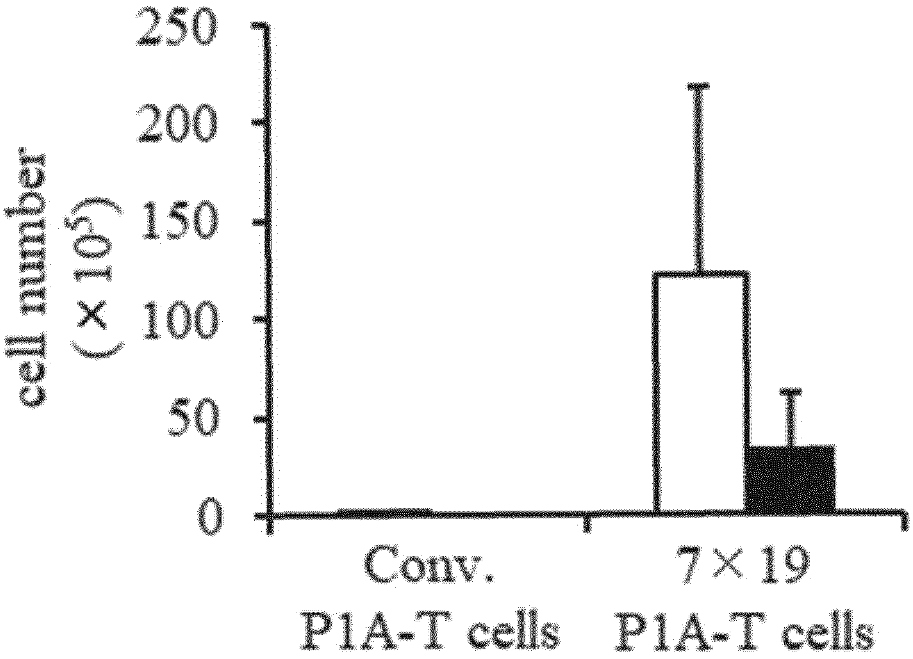
cell number
( ×10$^5$)
250
200
150
100
50
0
Conv.
P1A-T cells
7×19
P1A-T cells

FIG.8C

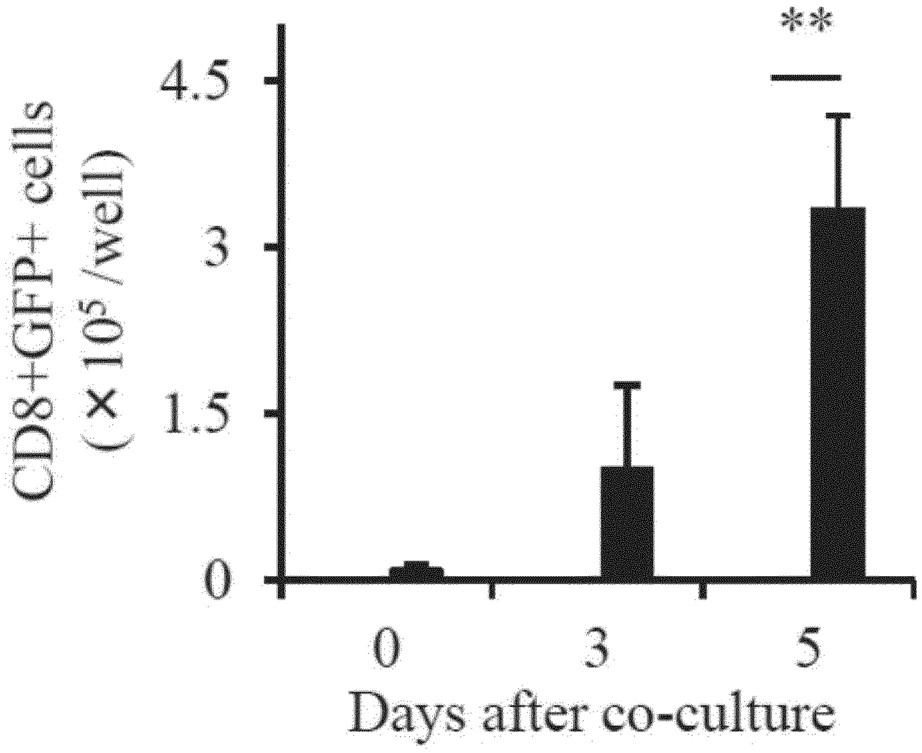
**
4.5
3
1.5
0
CD8+GFP+ cells
( ×10$^5$ /well)
0
3
5
Days after co-culture

FIG.8D

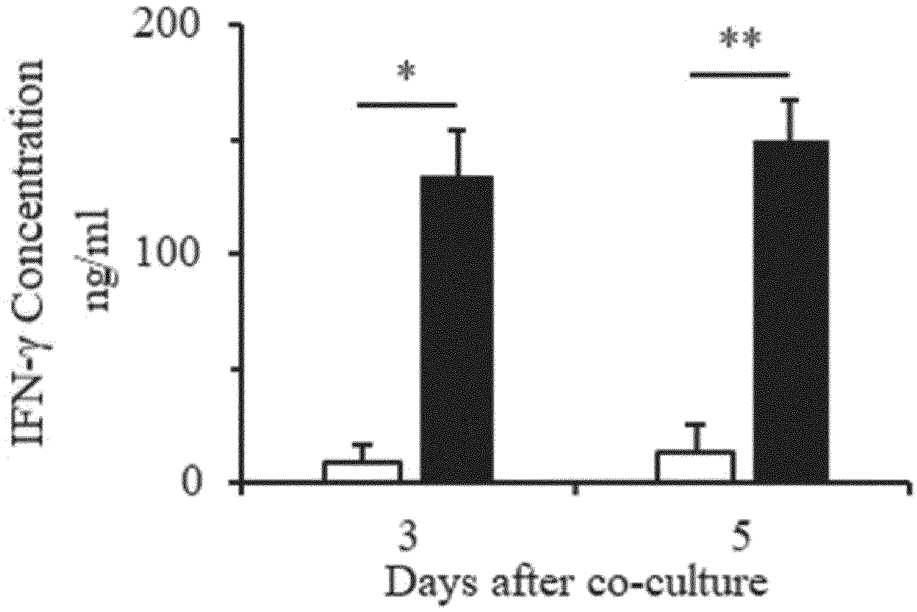
200
100
0
*
**
IFN-γ Concentration
ng/ml
3
5
Days after co-culture

FIG.9

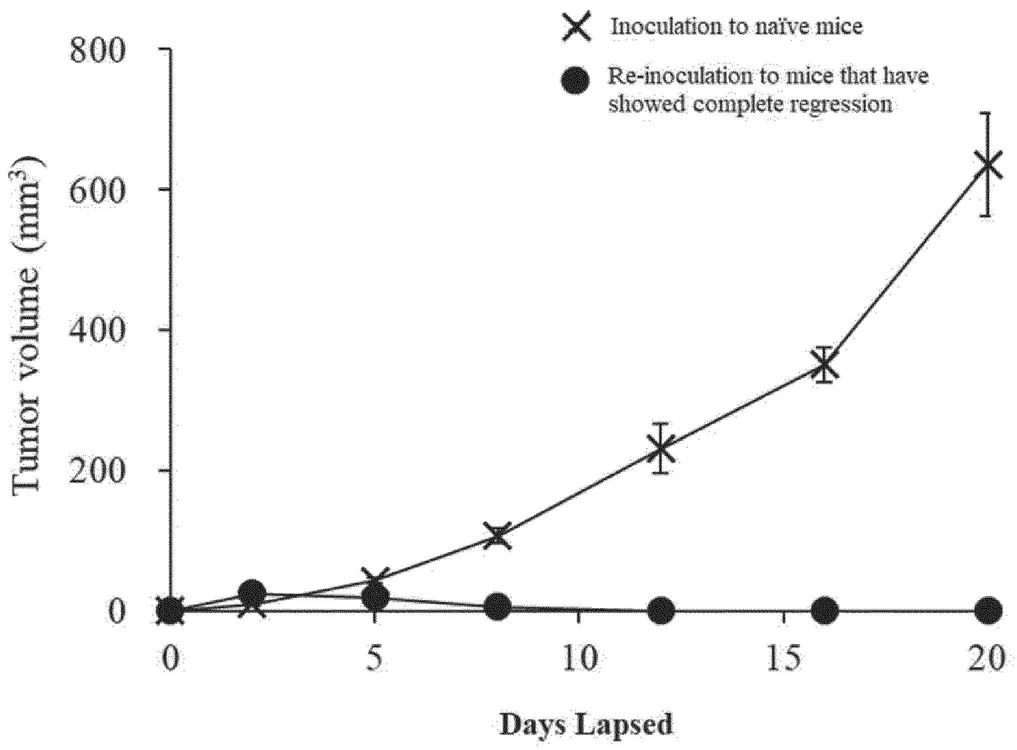
Inoculation to naïve mice
Re-inoculation to mice that have showed complete regression
800
600
400
200
0
Tumor volume (mm3)
0
5
10
15
20
Days Lapsed

FIG.10A

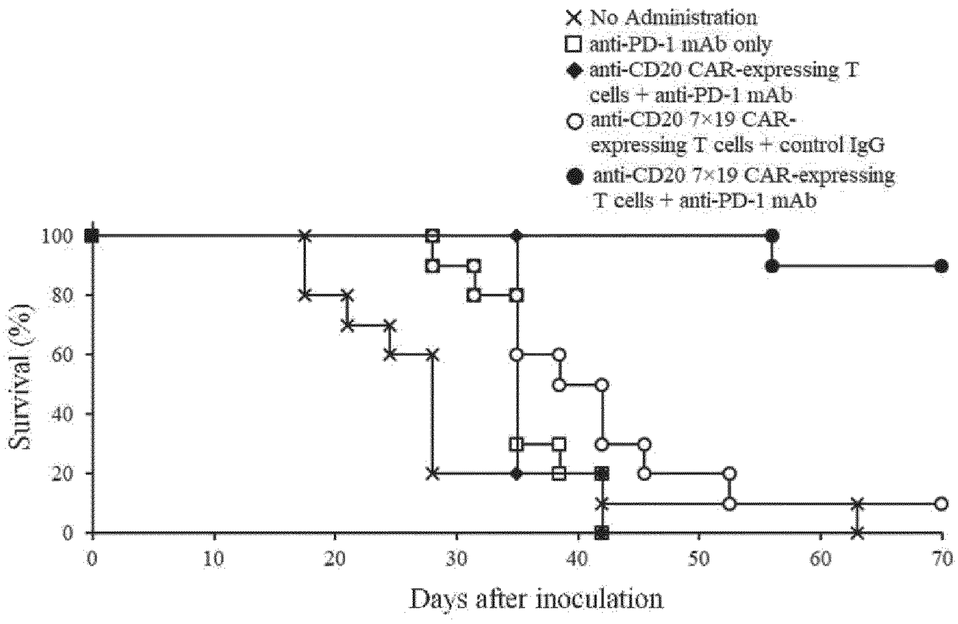


FIG.10B

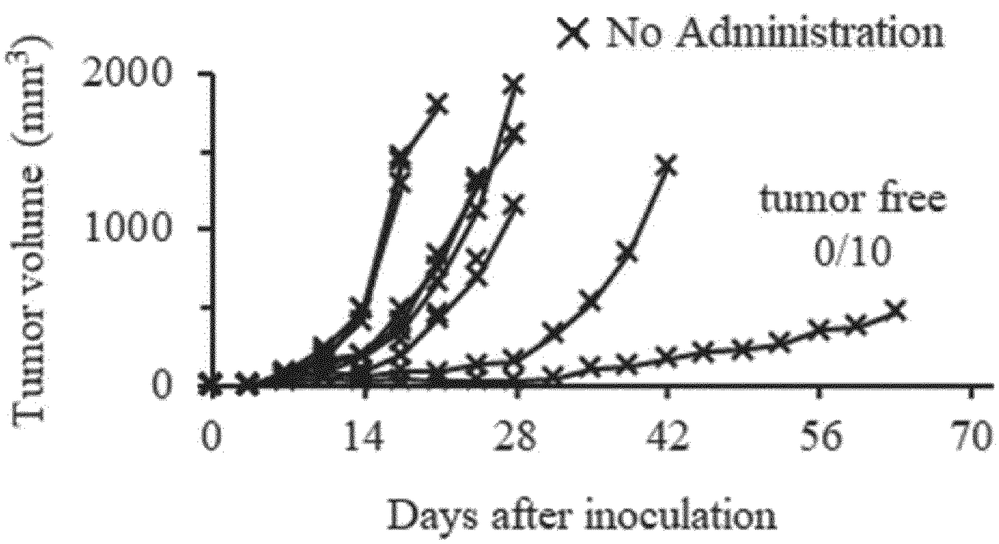

FIG.10C

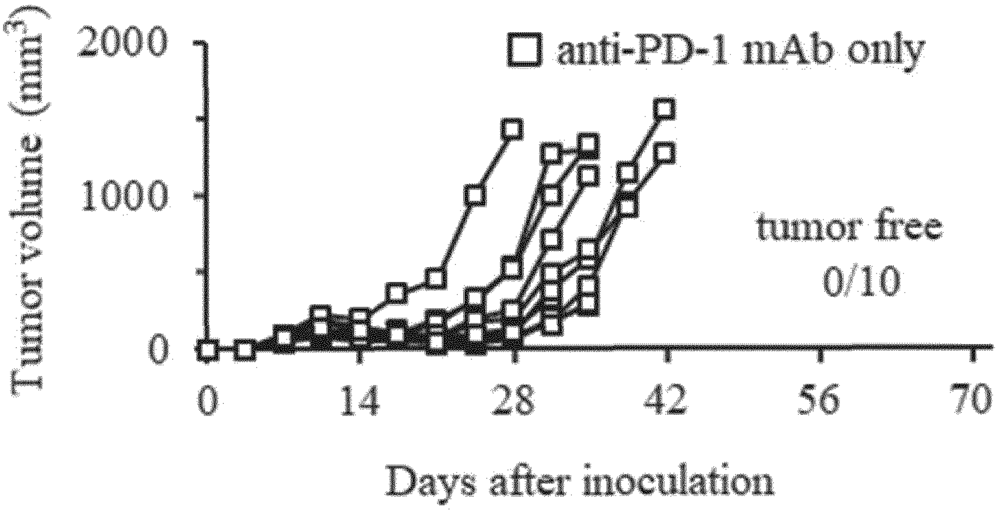


FIG.10D

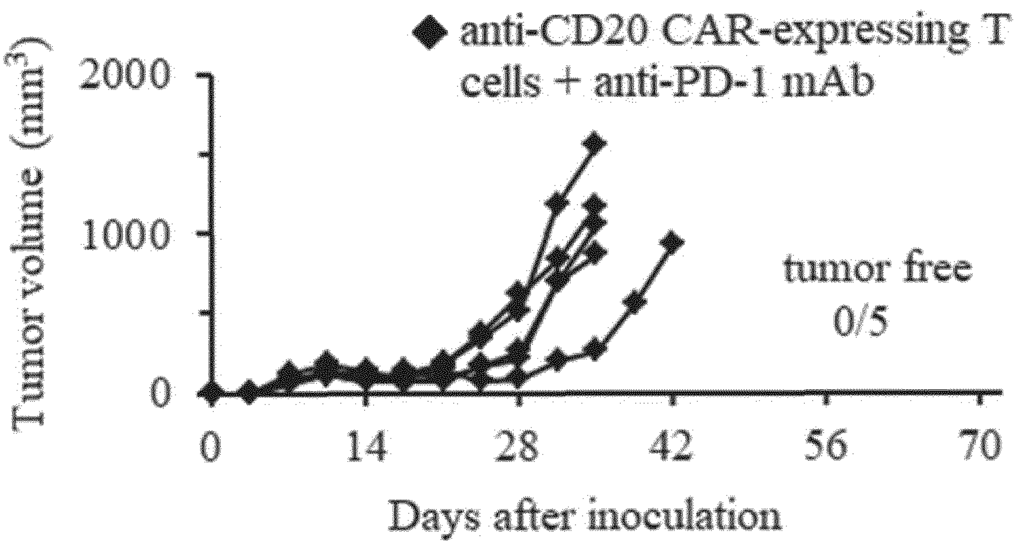

FIG.10E

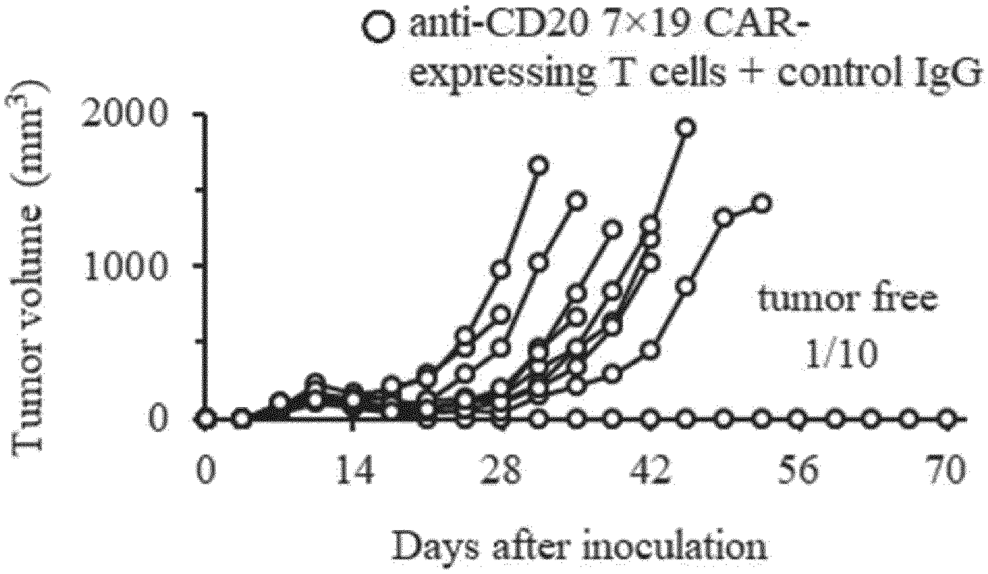


FIG.10F

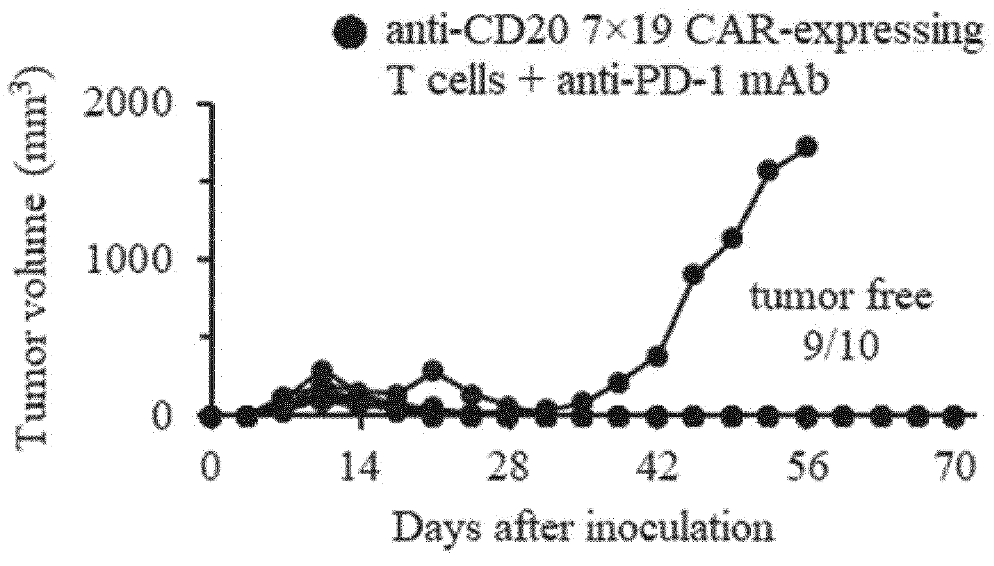

FIG.11A

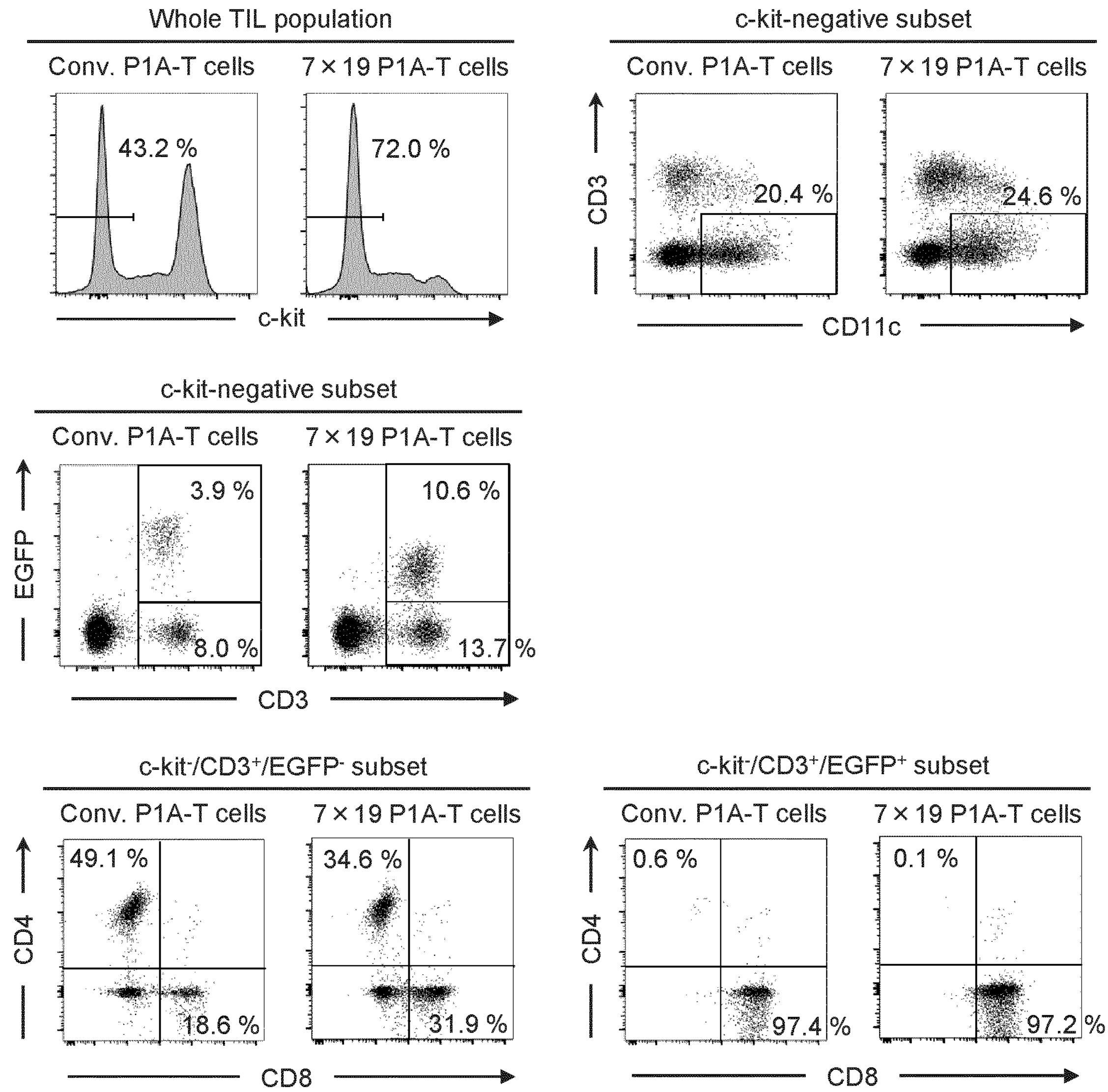


FIG.11B

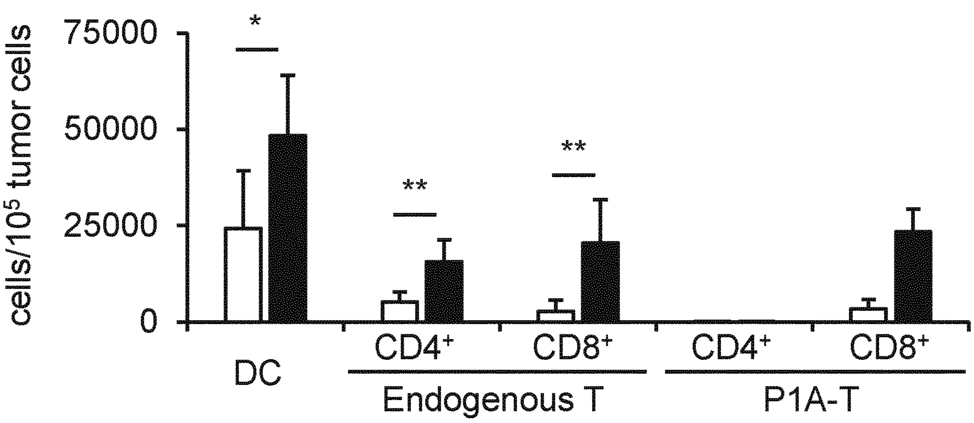

FIG.12

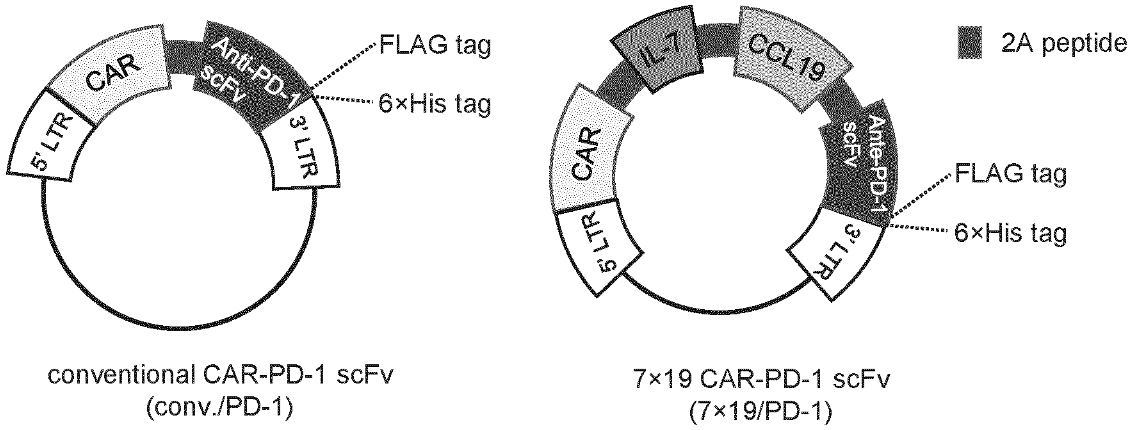
CAR
Anti-PD-1 scFv
5' LTR
3' LTR
FLAG tag
6×His tag
IL-7
CCL19
CAR
Ante-PD-1 scFv
5' LTR
3' LTR
FLAG tag
6×His tag
2A peptide
conventional CAR-PD-1 scFv
(conv./PD-1)
7×19 CAR-PD-1 scFv
(7×19/PD-1)

FIG.13

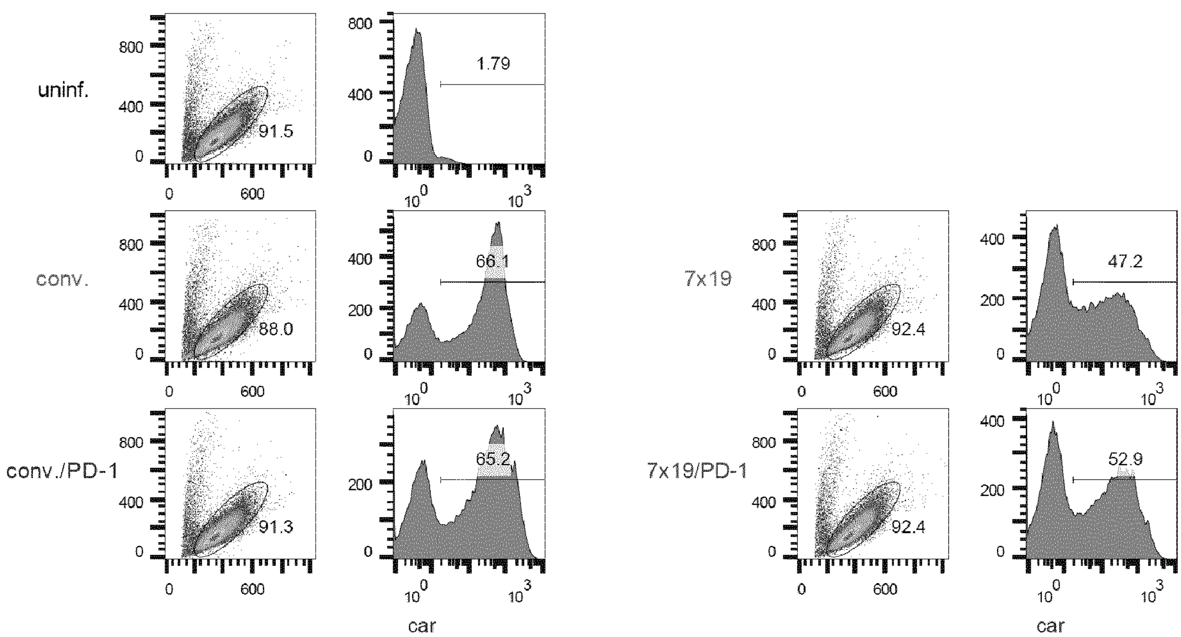
uninf.
91.5
1.79
conv.
88.0
66.1
conv./PD-1
91.3
65.2
7x19
92.4
47.2
7x19/PD-1
92.4
52.9
car
car

# FIG.14

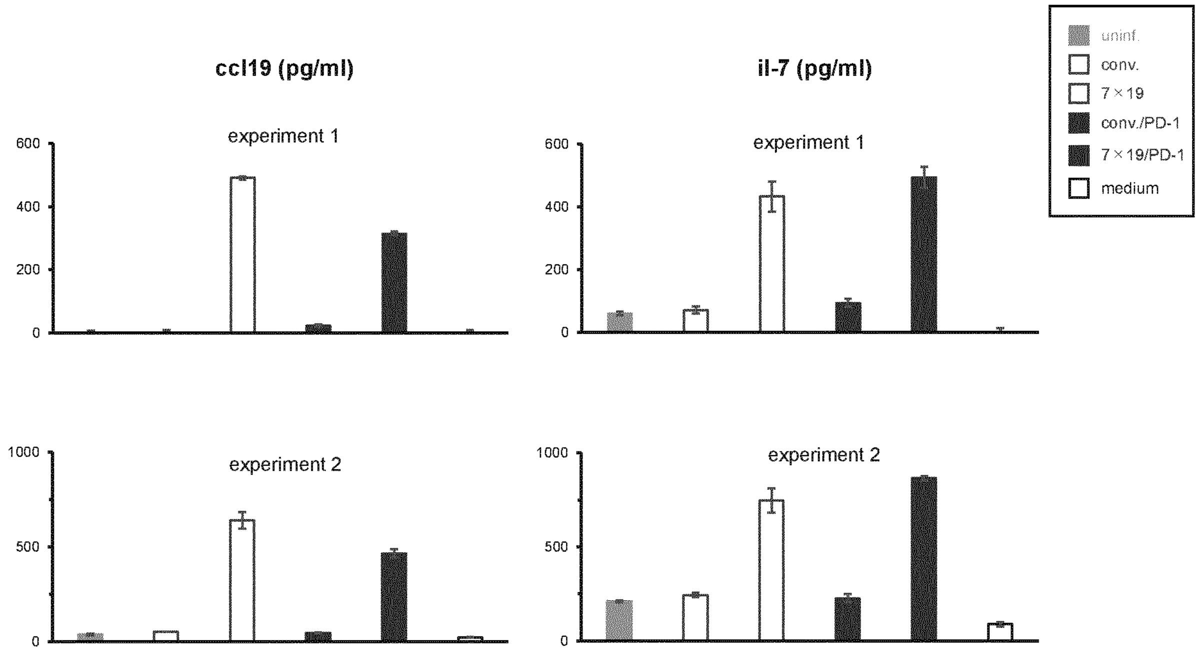

ccl19 (pg/ml)
il-7 (pg/ml)
experiment 1
600
400
200
0
experiment 2
1000
500
0
uninf.
conv.
7×19
conv./PD-1
7×19/PD-1
medium


# FIG.15

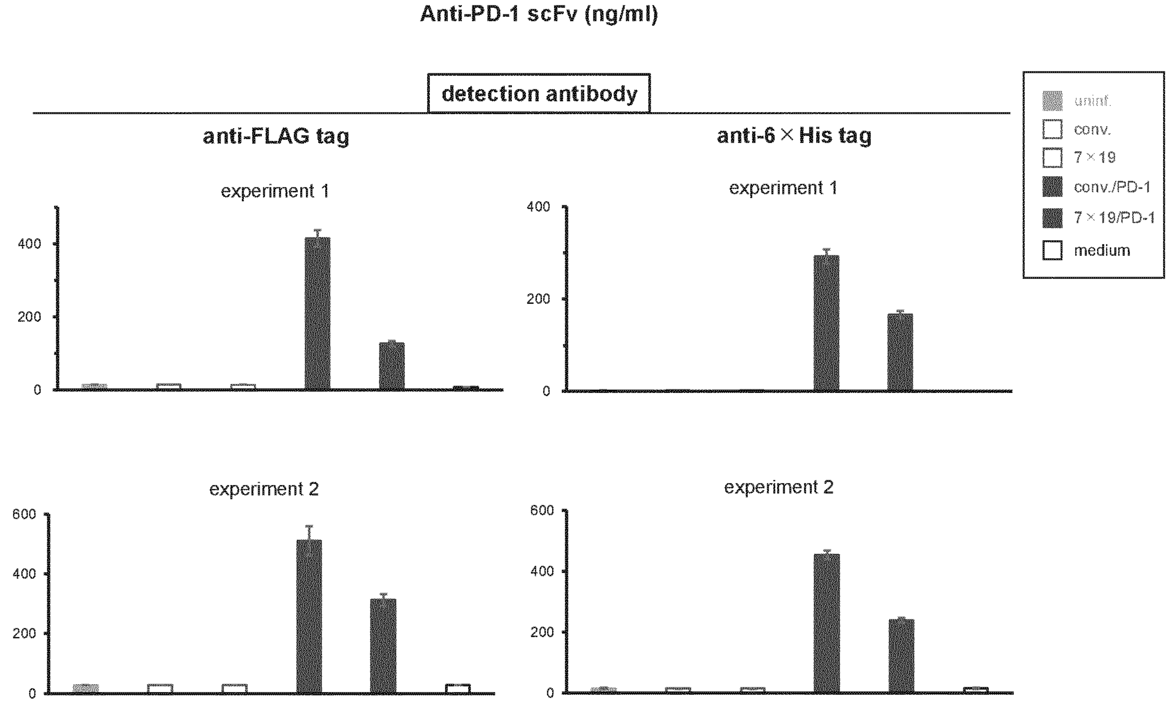

Anti-PD-1 scFv (ng/ml)
detection antibody
anti-FLAG tag
anti-6×His tag
experiment 1
400
200
0
experiment 2
600
400
200
0
uninf.
conv.
7×19
conv./PD-1
7×19/PD-1
medium

FIG.16

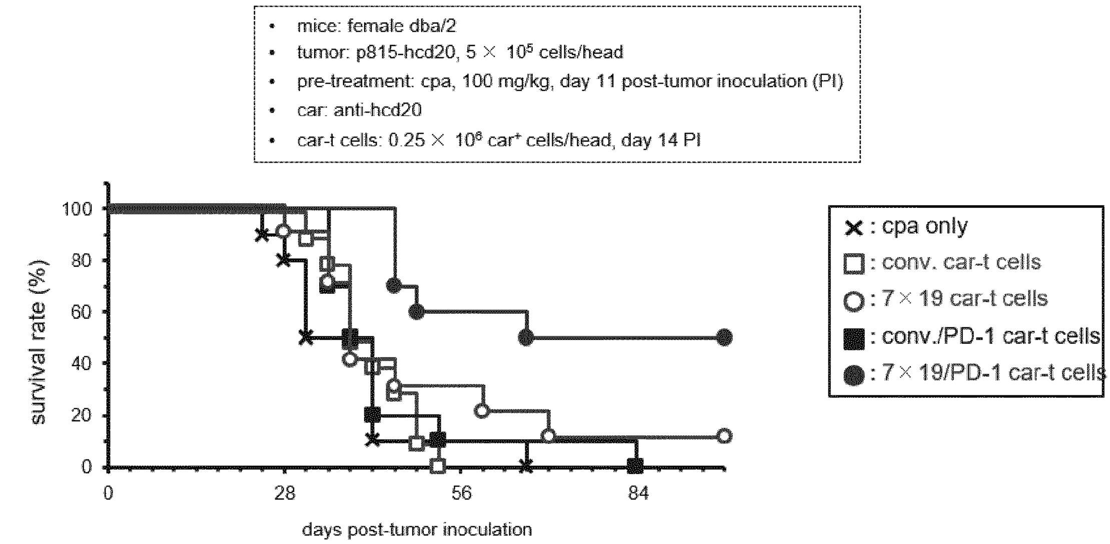


| log rank tests | no treat | conv. | 7×19 | PD-1 scfv-producing conv. | PD-1 scfv-producing 7×19 |
|---|---|---|---|---|---|
| no treat | | 0.579 | 0.251 | 0.395 | 0.000222 |
| conv. | | | 0.425 | 0.827 | 0.00138 |
| 7×19 | | | | 0.648 | 0.0168 |
| PD-1 scfv-producing conv. | | | | | 0.000939 |
| PD-1 scfv-producing 7×19 | | | | | |

FIG.17

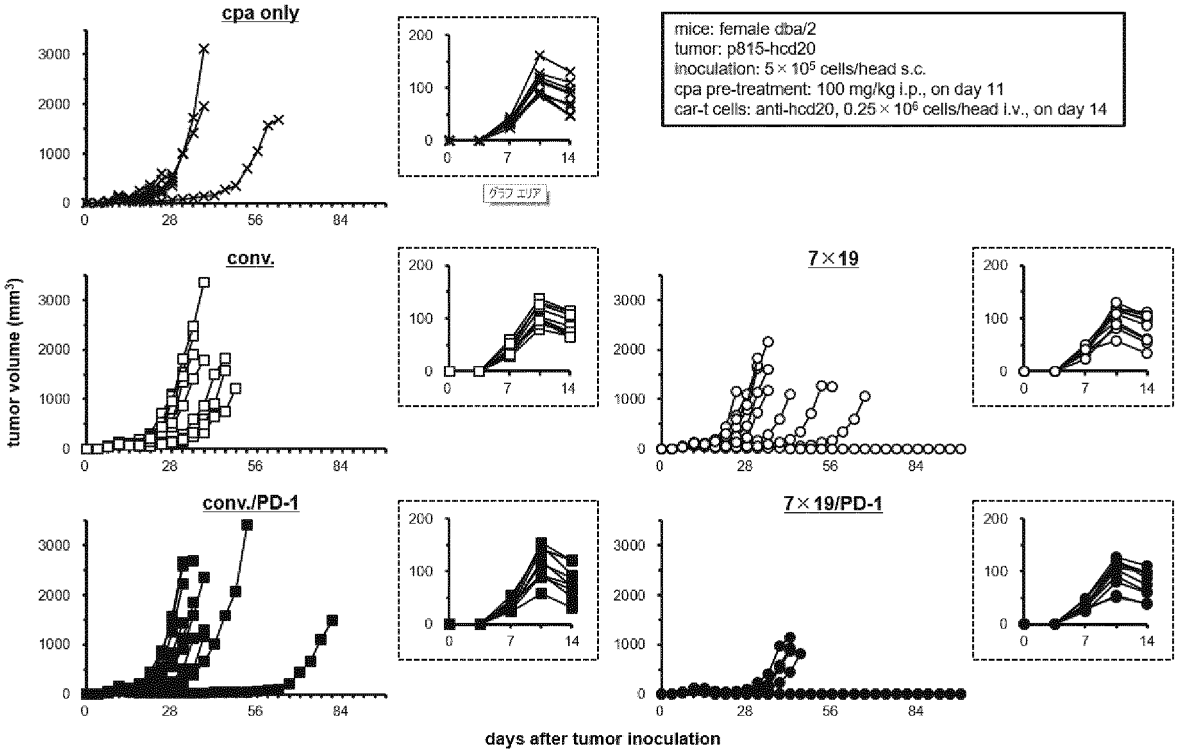
cpa only
conv.
7×19
conv./PD-1
7×19/PD-1
tumor volume (mm$^3$)
days after tumor inoculation
mice: female dba/2
tumor: p815-hcd20
inoculation: 5 × 10$^5$ cells/head s.c.
cpa pre-treatment: 100 mg/kg i.p., on day 11
car-t cells: anti-hcd20, 0.25 × 10$^6$ cells/head i.v., on day 14
グラフ エリア

**INTERNATIONAL SEARCH REPORT**

International application No. PCT/JP2020/040503

A. CLASSIFICATION OF SUBJECT MATTER

A61K 35/15(2015.01)i; A61K 35/17(2015.01)i; A61K 38/16(2006.01)i; A61K 38/19(2006.01)i; A61K 38/20(2006.01)i; A61K 39/395(2006.01)i; A61K 45/00(2006.01)i; A61P 35/00(2006.01)i; C07K 14/47(2006.01)i; C07K 14/54(2006.01)i; C07K 14/725(2006.01)i; C07K 16/28(2006.01)i; C07K 19/00(2006.01)i; C12N5/10(2006.01)i; C12N15/12(2006.01)i; C12N 15/13(2006.01)i; C12N 15/24(2006.01)i; C12N 15/62(2006.01)i; C12N 15/867(2006.01)i

FI: A61K35/15 Z; A61K35/17 Z; A61P35/00; A61K45/00; A61K38/20; A61K39/395 N; A61K38/19; A61K38/16; C12N15/13; C12N15/12; C12N15/24; C07K16/28; C07K14/725; C07K14/54; C07K19/00; C07K14/47; C12N15/62 Z; C12N15/867 Z; C12N5/10 ZNA

According to International Patent Classification (IPC) or to both national classification and IPC

B. FIELDS SEARCHED

Minimum documentation searched (classification system followed by classification symbols)

A61K35/15; A61K35/17; A61K38/16; A61K38/19; A61K38/20; A61K39/395; A61K45/00; A61P35/00; C07K14/47; C07K14/54; C07K14/725; C07K16/28; C07K19/00; C12N5/10; C12N15/12; C12N15/13; C12N15/24; C12N15/62; C12N15/867

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)

JSTPlus/JMEDPlus/JST7580 (JDreamIII); CAplus/MEDLINE/EMBASE/BIOSIS/WPIDS (STN)

C. DOCUMENTS CONSIDERED TO BE RELEVANT

| Category* | Citation of document, with indication, where appropriate, of the relevant passages | Relevant to claim No. |
|---|---|---|
| Y | WO 2017/159736 A1 (YAMAGUCHI UNIVERSITY) 21 September 2017 (2017-09-21) in particular, claims, examples | 1-37 |
| Y | WO 2019/073973 A1 (YAMAGUCHI UNIVERSITY) 18 April 2019 (2019-04-18) in particular, claims, examples | 1-37 |

☒ Further documents are listed in the continuation of Box C. ☒ See patent family annex.

* Special categories of cited documents:

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| 15 December 2020 (15.12.2020) | 28 December 2020 (28.12.2020) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2020/040503

C (Continuation). DOCUMENTS CONSIDERED TO BE RELEVANT

| Category* | Citation of document, with indication, where appropriate, of the relevant passages | Relevant to claim No. |
|---|---|---|
| Y | ADACHI, K. et al., "IL-7 and CCL19 expression in CAR-T cells improves immune cell infiltration and CAR-T cell survival in the tumor", Nature Biotechnology, April 2018, 36(4), pp. 346-351, abstract, fig. 2 | 1-37 |
| Y | RAFIQ, S. et al., "Targeted delivery of a PD-1-blocking scFv by CAR-T cells enhances anti-tumor efficacy in vivo", Nature Biotechnology, September 2018, 36(9), pp. 847-856, abstract | 1-37 |
| Y | CAO, Y. et al., "Anti-CD19 chimeric antigen receptor T cells in combination with nivolmab are safe and effective against relapsed/refractory B-cell non-hodgkin lymphoma", Frontiers in Oncology, August 2019, 9, article 767, pp. 1-11, abstract | 1-37 |
| Y | CHERKASSKY, L. et al., "Human CAR T cells with cell-intrinsic PD-1 checkpoint blockade resist tumor-mediated inhibition", J Clin Invest., 2016, 126(8), pp. 3130-3144, abstract, fig. 8 | 1-37 |
| Y | JP 2018-538339 A (NOVARTIS AG.) 27 December 2018 (2018-12-27) in particular, claims, examples, paragraphs [0565]-[0579] | 1-37 |

Form PCT/ISA/210 (continuation of second sheet) (January 2015)

**INTERNATIONAL SEARCH REPORT**
Information on patent family members

International application No.
PCT/JP2020/040503

| Patent Documents referred in the Report | Publication Date | Patent Family | Publication Date |
|---|---|---|---|
| WO 2017/159736 A1 | 21 Sep. 2017 | US 2019/0099446 A1<br>in particular, claims, examples<br>EP 3431597 A1<br>KR 10-2018-0127403 A<br>CN 109153989 A<br>JP 2019-154455 A | |
| WO 2019/073973 A1 | 18 Apr. 2019 | US 2020-0352997 A1<br>in particular, claims, examples<br>EP 3695846 A1<br>CN 111163758 A<br>KR 10-2020-0068661 A | |
| JP 2018-538339 A | 27 Dec. 2018 | US 2019/0000944 A1<br>in particular, claims, examples, paragraphs [0599]-[0613]<br>WO 2017/112741 A1<br>EP 3393504 A1<br>CN 109069597 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017159736 A
- WO 2019073973 A
- JP 2018538339 A
- WO 2007032255 A
- US 5091513 A
- US 5132405 A
- US 4956778 A
- US 20050196754
- WO 2012094386 A
- JP 3867968 B
- JP 5574284 B
- JP H2227075 A
- WO NO2017159736 A
- WO 201656228 A
- JP 2019195407 A


### Non-patent literature cited in the description


- *Cancer Res.,* 1994, vol. 54, 5265-5268
- *Anticancer Res.,* 2000, vol. 20, 1793-1799
- *J. Immunol.,* 2003, vol. 170, 2186-2194
- *J. Immunol.,* 2005, vol. 174, 4415-4423
- *Blood,* 2005, vol. 106, 470-476
- *Int. Immunol.,* 1996, vol. 8, 1463-1466
- **SARMA, S. ; Y. GUO ; Y. GUILLOUX ; C. LEE ; X.-F. BAI ; Y. LIU.** Cytotoxic T lymphocytes to an unmutated tumor antigen P1A: normal development but restrained effector function. *J. Exp. Med.,* 1999, vol. 189, 811
- **MORGAN et al.** *J. Immunol,* 2003, vol. 171, 3288
- **SZYMCZAK et al.** *Expert Opin. Biol. Ther.,* 2005, vol. 5 (5), 627-638
- **TAMADA K. et al.** *Clin. Cancer Res.,* 2002, vol. 18, 6436-6445
- **COOPER L. J.** *Cytotherapy,* 2006, vol. 8 (2), 105-17
- **JENSEN M. C.** *Biol BloodMarrow Transplant,* September 2010, vol. 16 (9), 1245-56
- **JONES B. S.** *Front Pharmacol.,* 27 November 2014, vol. 5, 254
- **MINAGAWA K.** *Pharmaceuticals (Basel),* 08 May 2015, vol. 8 (2), 230-49
- **BOLE-RICHARD E.** *Front Pharmacol.,* 25 August 2015, vol. 6, 174
- **HUSTON et al.** *Proc. Nat. Acad. Sci. USA,* 1988, vol. 85, 5879-5883
- REMINGTON'S PHARMACEUTICAL SCIENCE. 1985
- *Molecular Therapy,* February 2010, vol. 18 (2), 233-234
- **KIM MK et al.** *Science Translational Medicine,* 15 May 2013, vol. 5 (185), 185-63
- **HEO J et al.** *Nature Medicine,* 10 February 2013, vol. 3, 329-36
- **TEDCASTLE A et al.** *Mol. Ther.,* 2016, vol. 24, 796-804
- **MARINO N ; ILLINGWORTH S ; KODIALBAIL P ; PATEL A ; CALDERON H ; LEAR R ; FISHER KD ; CHAMPION BR ; BROWN ACN.** *PLoS One,* 2017, vol. 12 (5), e0177810
- **FREEDMAN JD et al.** *EMBO Mol. Med.,* 2017, vol. 9, 1067-1087
- **LANG FF et al.** *Journal of Clinical Oncology,* 2018
- **JAMES M. et al.** *The Journal of Oncology,* 2012, vol. 188 (6), 2391-7
- **MAZZACURATI et al.** *Mol. Ther.,* January 2015, vol. 23 (1), 99-107
- **HIROOKA Y et al.** *BMC Cancer,* 2018, vol. 18, 596
- **NAKATAKE R et al.** *Cancer Sci.,* March 2018, vol. 109 (3), 600-610
- **ANDTBACKA RHI et al.** *J. Clin. Oncol.,* 2015, vol. 33, 2780-2788
- **MAHALINGAM et al.** *Cancers,* 2018, vol. 10, 160
- *Journal of Virology,* June 2016, vol. 90 (11), 5343-5352
- **MUIK A. et al.** *Cancer Res,* vol. 74 (13), 3567-78
- *Cytotechnology,* 1990, vol. 3, 133
- *Proc. Natl. Acad. Sci. U.S.A.,* 1987, vol. 84, 7413
- **TAMADA K. et al.** *Clin Cancer Res,* 2002, vol. 18, 6436-6445
- **TAMADA K et al.** *Clin Cancer Res,* 2002, vol. 18, 6436-6445
- **SARMA, S. ; Y. GUO ; Y. GUILLOUX ; C. LEE ; X.-F. BAI ; Y. LIU.** *J. Exp. Med.,* 1999, vol. 189, 811-820

- **OKADA M et al.** Blockage of Core Fucosylation Reduces Cell-Surface Expression of PD-1 and Promotes Anti-tumor Immune Responses of T Cells. *Cell Rep.,* 2017, vol. 20 (5), 1017-1028
- *Nat Biotechnol.,* 2018, vol. 36 (4), 346-351